# EUROPEAN PATENT SPECIFICATION

(11) **EP 0 705 816 B1**
(45) Date of publication and mention of the grant of the patent: **20.09.2000**
(21) Application number: 94918559.9
(22) Date of filing: 23.06.1994
(51) Int. Cl.: C07C 233/78, C07C 235/50, C07C 261/04, C07C 311/18, C07C 311/29, C07C 327/48, C07D 233/64, C07D 249/08, C07D 271/06

(54) **BENZENE DERIVATIVE USEFUL FOR ISCHEMIC DISEASES**
BENZOL-DERIVATE, VERWENDBAR FÜR ISCHÄMISCHE ERKRANKUNGEN
DERIVE DU BENZENE UTILE CONTRE LES ISCHEMIES

(30) Priority: 23.06.1993 JP 15224893
(43) Date of publication of application: 10.04.1996
(73) Proprietor: Chugai Seiyaku Kabushiki Kaisha, Tokyo 115 (JP)
(72) Inventor: OHI, Nobuhiro Chugai Seiyaku Kabushiki Kaisha, Gotenba-shi Shizuoka-ken 412 (JP); KATO, Tatsuya Chugai Seiyaku Kabushiki Kaisha, Gotenba-shi Shizuoka-ken 412 (JP); OZAKI, Tomokazu Chugai Seiyaku Kabushiki Kaisha, Gotenba-shi Shizuoka-ken 412 (JP)
(74) Representative: VOSSIUS & PARTNER
(86) International application number: JP9401009
(87) International publication number: WO9500471

(56) References cited:
- EP-A- 0 515 684
- WO-A-90/02114
- JP-A- 57 181 053
- JP-A- 62 042 977
- JP-A- 63 313 757
- US-A- 2 520 179
- US-A- 2 623 048
- US-A- 3 309 377
- JOURNAL OF HETEROCYCLIC CHEMISTRY, vol. 29, 1992, PROVO US, pages 1081-1084, XP002028431 R. MILCENT ET AL.: "Cyclic transformations of 5-Aryl(or Benzyl)-3-(2-bromoethyl-1,3,4-oxadiazol-2( 3H)-ones into 1-Aminoimidazolidin-2-one and 5,6-Dihydro-4H-1,3,4-oxadiazine Derivatives"
- JOURNAL OF THE AMERICAN CHEMICAL SOCIETY, vol. 73, 1951, DC US, pages 1967-1968, XP002028432 R.W.BOST ET AL.: "N-(beta-Aminoethyl)morpholine as a reagent for the characterization of esters"
- HELVETICA CHIMICA ACTA, vol. 39, 1956, BASEL CH, pages 607-618, XP002028433 H.J. SCHMEID ET AL.: "Sterische und elektrostatische Effekte in der Schmidt-Reaktion"
- JOURNAL OF ORGANIC CHEMISTRY, vol. 26, 1961, EASTON US, pages 3414-3419, XP002028434 SHIN HAYAO ET AL.: "New sedative and hypertensive Phenylpiperazine Amides"
- JOURNAL OF THE AMERICAN CHEMICAL SOCIETY, vol. 71, 1949, DC US, pages 3105-3107, XP002028435 A.R. SURREY: "The preparation of 4-Thiazolidones. III. The reaction of Methyl Thioglycolate with benzylidene dialkylaminoalkylamines"
- CHEMISCHE BERICHTE, vol. 70, no. 6, 1937, WEINHEIM DE, pages 1230-1240, XP002028436 J. V. BRAUN ET AL. : "Synthese des Spermidins und analoger Traimine der Fettreihe"
- FARMACO, EDIZIONE SCIENTIFICA, vol. 35, no. 6, 1980, PAVIA IT, pages 527-534, XP002028437 G. MAZONNE ET AL.: "Derivati amminoetilici di alcuni 5-aril-1,3,4-ossadiazol-2-oni: sintesi e attivita farmacologica"

## Description

This invention relates to compounds by the general formula (I) and pharmaceutically acceptable salts or each possible stereoisomer and optical isomer thereof, which have an inhibitory action on calcium overload in addition to a vasorelaxing activity (calcium antagonism) and an inhibitory action on lipid peroxidation and are useful as a preventive or treating agent for ischemic diseases and hypertension.

### Background of the Invention

The process of cell injuries owing to ischemia is broadly divided into two courses: (1) damages caused by reduction in intracellular ATP level or increase in intracellular calcium concentration etc. under insufficient oxygen supply during ischemia and (2) damages caused by increasing influx of calcium or production of free radicals, etc. followed by reperfusion or restoration of blood vessels after ischemia (Yoshiwara, et al., Metabolism and disease, 29, 379 (1992)). As typical ischemic diseases, cardiovascular diseases such as variant angina, unstable angina and arrythmia brought by restoration of coronary vessels by PTCA/PTCR/CABG, etc. or cerebrovascular diseases such as transient cerebroischemic attack; traumatic injury to the head and sequels after brain surgery can be given. In the treatment for variant angina or unstable angina, nitro compounds exemplified by nitroglycerin and nicorandil and calcium antagonists exemplified by diltiazem, nifedipine and verapamil are used and for the amelioration of cardiac infarction or coronary reperfusion injury followed by PTCA/PTCR/CABG, etc., use of 5-lipoxygenase inhibitors or radical scavengers are under investigation. As preventive and treating agents for ischemic cerebrovascular diseases, glyceol (registered trade mark), ozagrel, nizofenone, ticlopidine, nicaraven, etc. have been investigated and used with the idea of reducing the occurrence of cerebral edema or cerebrovascular spasms in the acute stage of cerebrovascular accident. In the chronic stage, cerebral circulation enhancers such as calcium antagonists exemplified by nicardipine, cinnarizine and flunarizine, cerebral circulation enhancers with promotive action for metabolism such as vinpocetine, nicergoline, pentoxifylline, and ifenprodil, or cerebral metabolic activators such as idebenone, GABA, and calcium hopantenate have been used in order to increase blood flow or improve the metabolic state in the tissue which survived the ischemic injury.

Based on extensive investigation to seek for an excellent preventive and treating agents for ischemic diseases or hypertension which would be able to suppress the generation of active oxygen species and increase of intracellular calcium concentration that are considered as the main causes of ischemic diseases and hypertension, the present inventors have discovered compounds represented by formula (I) having vasorelaxing activity (calcium antagonism), inhibitory actions on lipid peroxidation and calcium overload and have resulted in the completion of the present invention.

On the other hand, compounds having a phenol moiety substituted by two t-butyl groups and 1,3-thiazolidine moiety, for example, 5-[[3,5-bis(t-butyl)-4-hydroxyphenyl]methylene]-3-methyl-4-thiazolidine, are known from EP-B-0 211 670 as anti-inflammatory drugs.

### Disclosure of the Invention

The present invention provides compounds represented by the general formula (I): 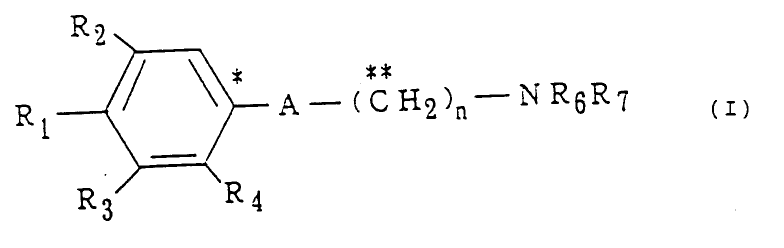 wherein R₁ represents a hydrogen atom, a hydroxyl group, an acyloxy group having 1 to 9 carbon atoms or a lower alkoxy group having 1 to 6 carbon atoms; R₂ and R₃, which may be the same or different, each represents, a hydroxyl group, an isopropyl group or a t-butyl group, R₄ represents a hydrogen atom, a lower alkyl group having 1 to 6 carbon atoms; A represents a fragment represented by formula (II): 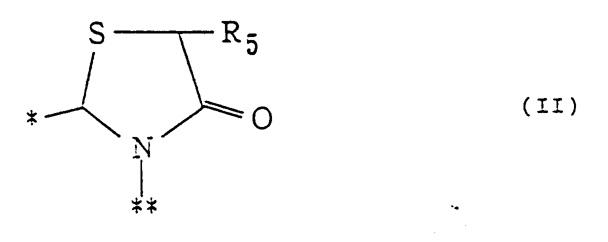 wherein R₅ represents a hydrogen atom, a substituted or unsubstituted lower alkyl group having 1 to 6 carbon atoms, a substituted or unsubstituted lower alkenyl group having 1 to 6 carbon atoms, a substituted or unsubstituted alkoxy group having 1 to 6 carbon atoms, a substituted or unsubstituted aryl group or a substituted or unsubstituted heterocyclic group, or R₅ forms a 5- or 6-membered ring containing two or more oxygen atoms or sulfur atoms, in which case the carbon atom to which it is bonded is a spiro atom; or a fragment represented by formula (III):

B (III)

wherein B represents a fragment selected from the group of following fragments represented by formulae (IV), (V), (VI), (VII), (VIII), (X), (XI), (XII), (XIII), (XIV), (XV), and (XVI): 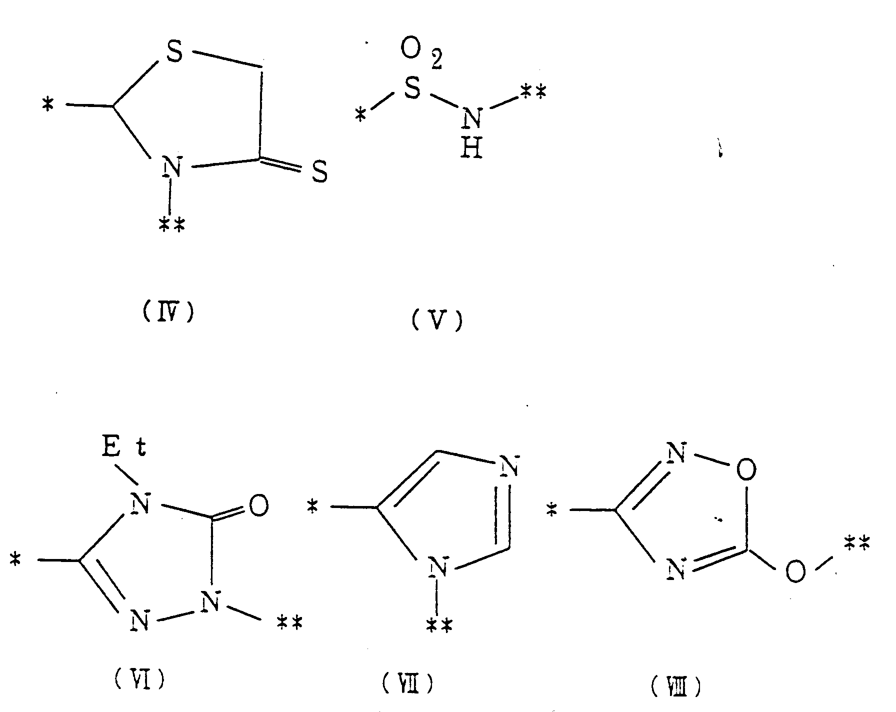 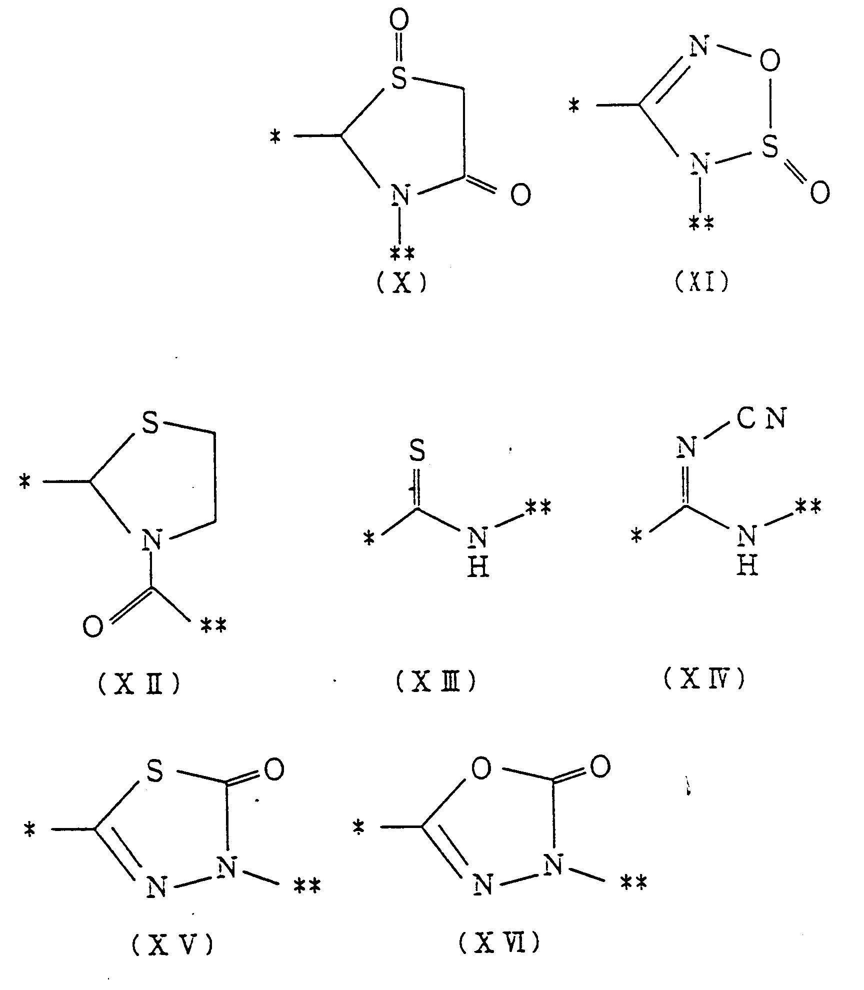 R₅ and R₇, which may be the same or different, each represents a hydrogen atom, a substituted or unsubstituted lower alkyl group having 1 to 6 carbon atoms, a substituted or unsubstituted lower alkenyl group having 1 to 6 carbon atoms, a substituted or unsubstituted aryl group or a substituted or unsubstituted heterocyclic group, provided that R₆ and R₇ do not represent a methyl group simultaneously, or R₆ and R₇ are taken together to form a substituted or unsubstituted ring which may be a condensed ring; and n represents an integer of 2, 3, 4, 5 or 6, and a pharmaceutically acceptable salt thereof or a possible stereoisomer or optical isomer thereof.

### Best Embodiment for Carrying out the Invention

The compound represented by formula (I) can be prepared by the following processes A to Q.

### Process A:

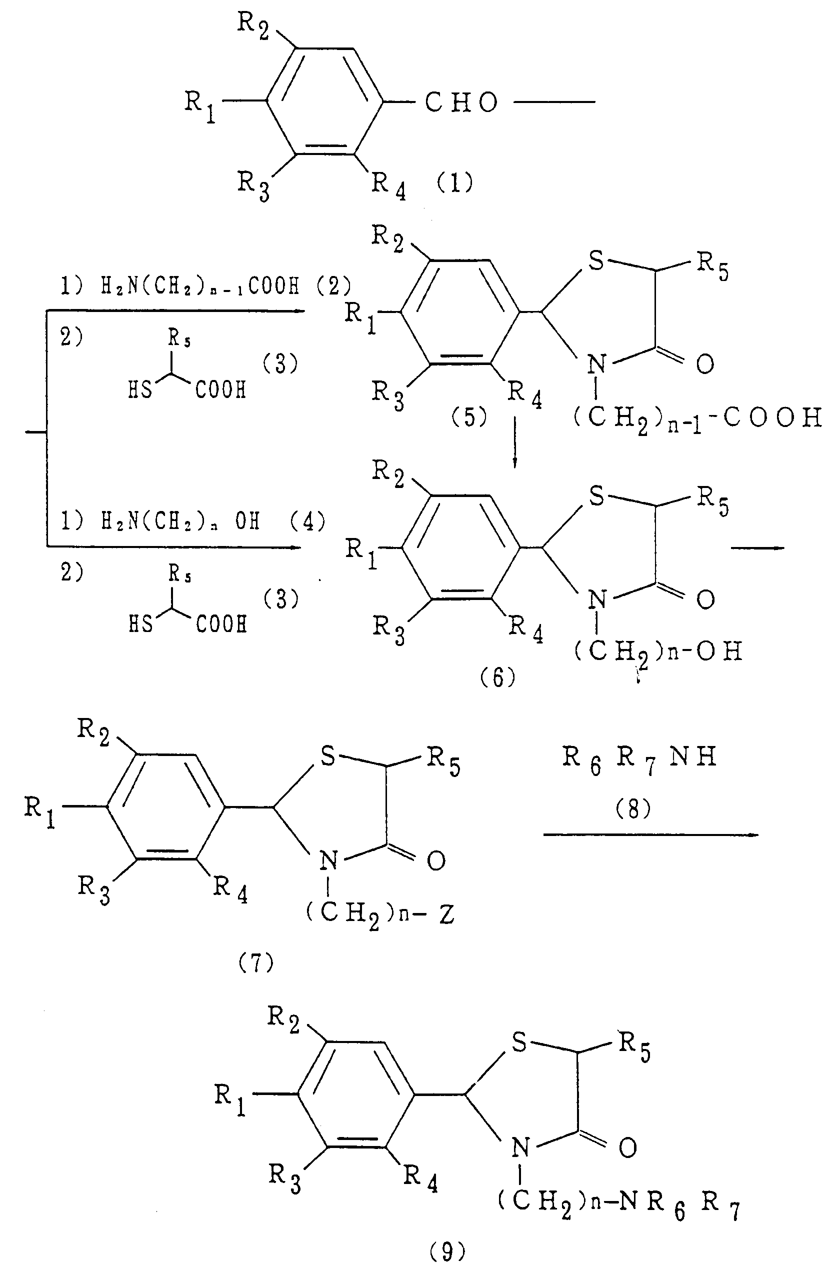

In the scheme of process A, R₁ represents a hydrogen atom, a hydroxyl group, an acyloxy group having 1 to 9 carbon atoms or a lower alkoxy group having 1 to 6 carbon atoms; R₂ and R₃, which may be the same or different, each represents a hydrogen atom, a hydroxyl group, a halogen atom, a lower alkyl group having 1 to 6 carbon atoms or a lower alkoxy group having 1 to 6 carbon atoms; R₄ represents a hydrogen atom, a lower alkyl group having 1 to 6 carbon atoms; R₅ represents a hydrogen atom, a substituted or unsubstituted lower alkyl group having 1 to 6 carbon atoms, a substituted or unsubstituted lower alkenyl group having 1 to 6 carbon atoms, a substituted or unsubstituted aryl group or a substituted or unsubstituted heterocyclic group; R₆ and R₇, which may be the same or different, each represent a hydrogen atom, a substituted or unsubstituted lower alkyl group having 1 to 6 carbon atoms, a substituted or unsubstituted lower alkenyl group having 1 to 6 carbon atoms, a substituted or unsubstituted aryl group or a substituted or unsubstituted heterocyclic group, provided that R₆ and R₇ do not represent a methyl group simultaneously, or R₆ and R₇ are taken together to form a substituted or unsubstituted ring which may be a condensed ring; n represents an integer of 2, 3, 4, 5 or 6; and Z represents a halogen atom.

### Process B:

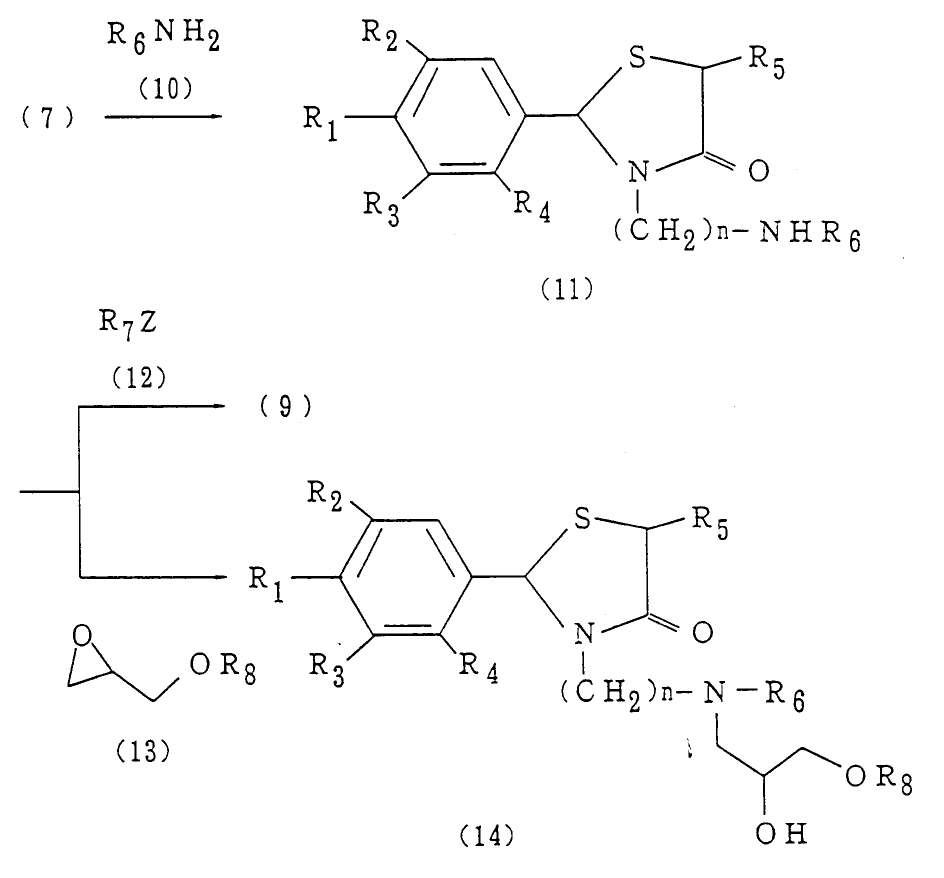

In the scheme of process B, R₁, R₂, R₃, R₄, R₅, R₆, R₇, and n are the same as defined previously; R₈ represents a substituted or unsubstituted aryl group or a substituted or unsubstituted heterocyclic group; and Z represents a chlorine atom or a bromine atom.

### Process C:

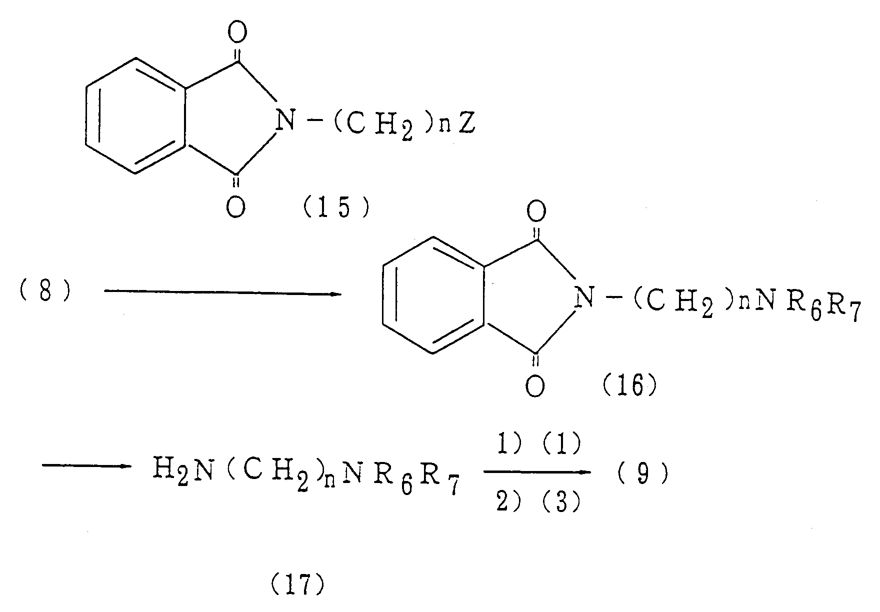

In the scheme of process C, R₁, R₂, R₃, R₄, R₅, R₆, R₇, R₈, n, and Z are the same as defined previously.

### Process D:

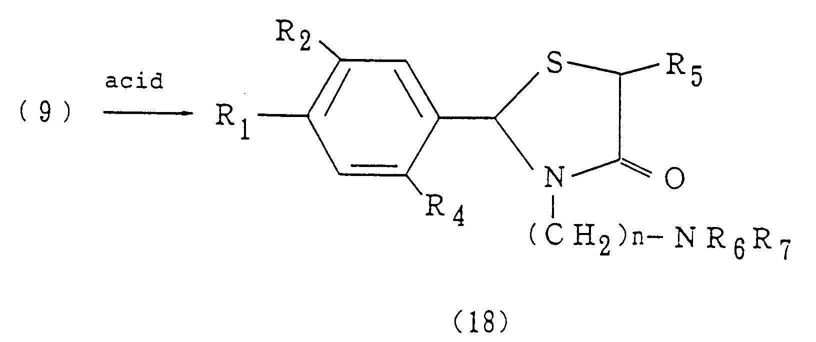

In the scheme of process D, R₁, R₂, R₃, R₄, R₅, R₆, R₇, and n are the same as defined previously.

### Process E:

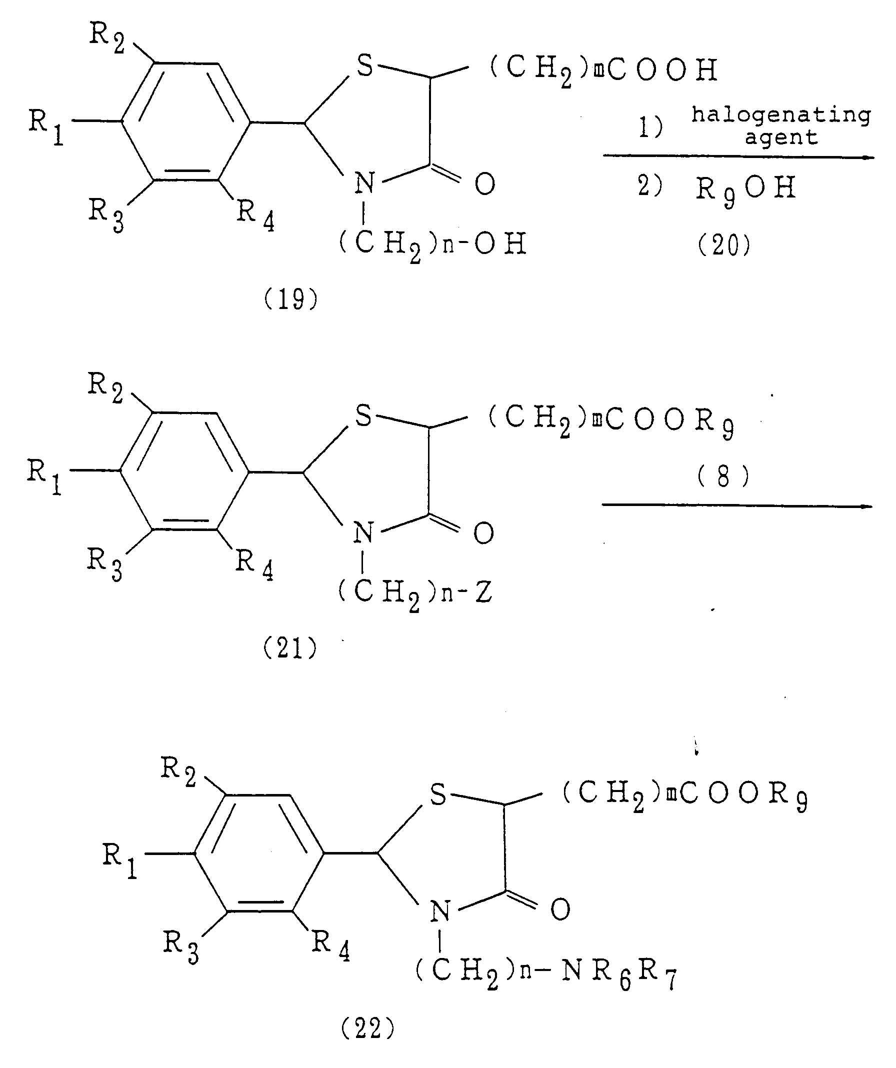

In the scheme of process E, R₁, R₂, R₃, R₄, R₅, R₆, R₇, n, and Z are the same as defined previously; R₉ represents an alkyl group having 1 to 6 carbon atoms; and m represents an integer of 1 to 7.

### Process F:

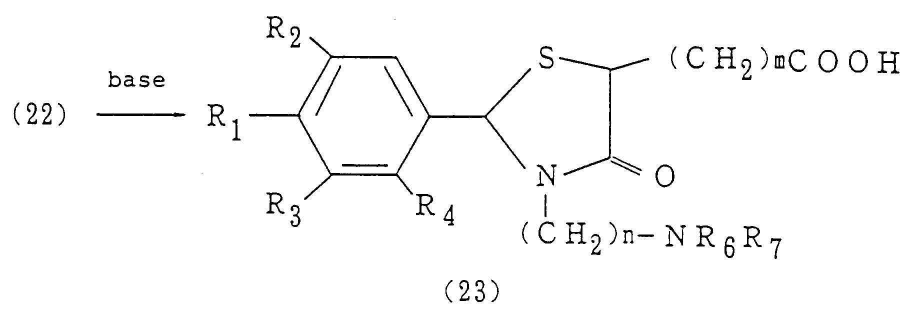

In the scheme of process F, R₁, R₂, R₃, R₄, R₅, R₆, R₇, R₈, R₉, n, and m are the same as defined previously.

### Process G:

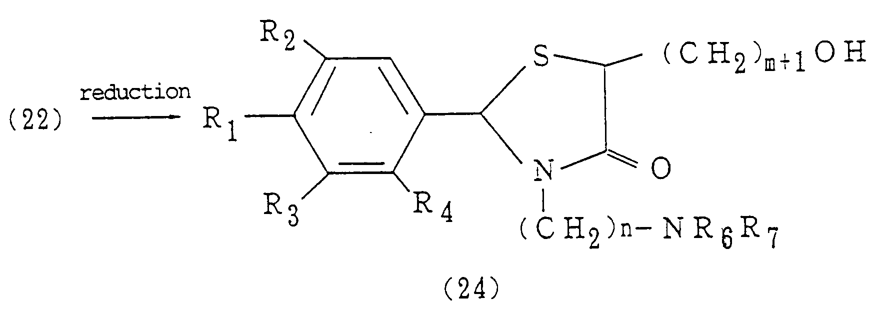

In the scheme of process G, R₂, R₃, R₄, R₅, R₆, R₇, R₈, R₉, n, and m are the same as defined previously.

### Process H:

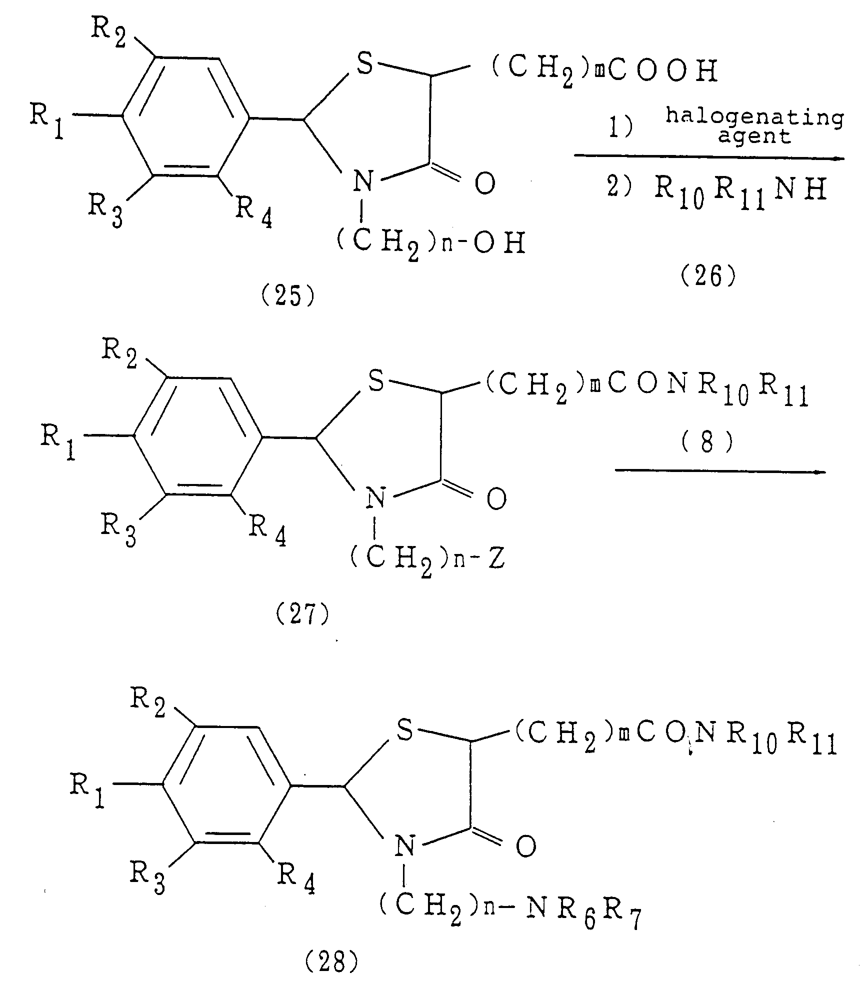

In the scheme of process H, R₁, R₂, R₃, R₄, R₅, R₅, R₆, Z, n, and m are the same as defined previously; and R₁₀ and R₁₁, which may be the same or different, each represents a hydrogen atom, a substituted or unsubstituted lower alkyl group having 1 to 6 carbon atoms, a substituted or unsubstituted lower alkenyl group having 1 to 6 carbon atoms, a substituted or unsubstituted aryl group or a substituted or unsubstituted heterocyclic group, or R₁₀ and R₁₁ are taken together to form a substituted or unsubstituted ring which may be a condensed ring.

### Process I:

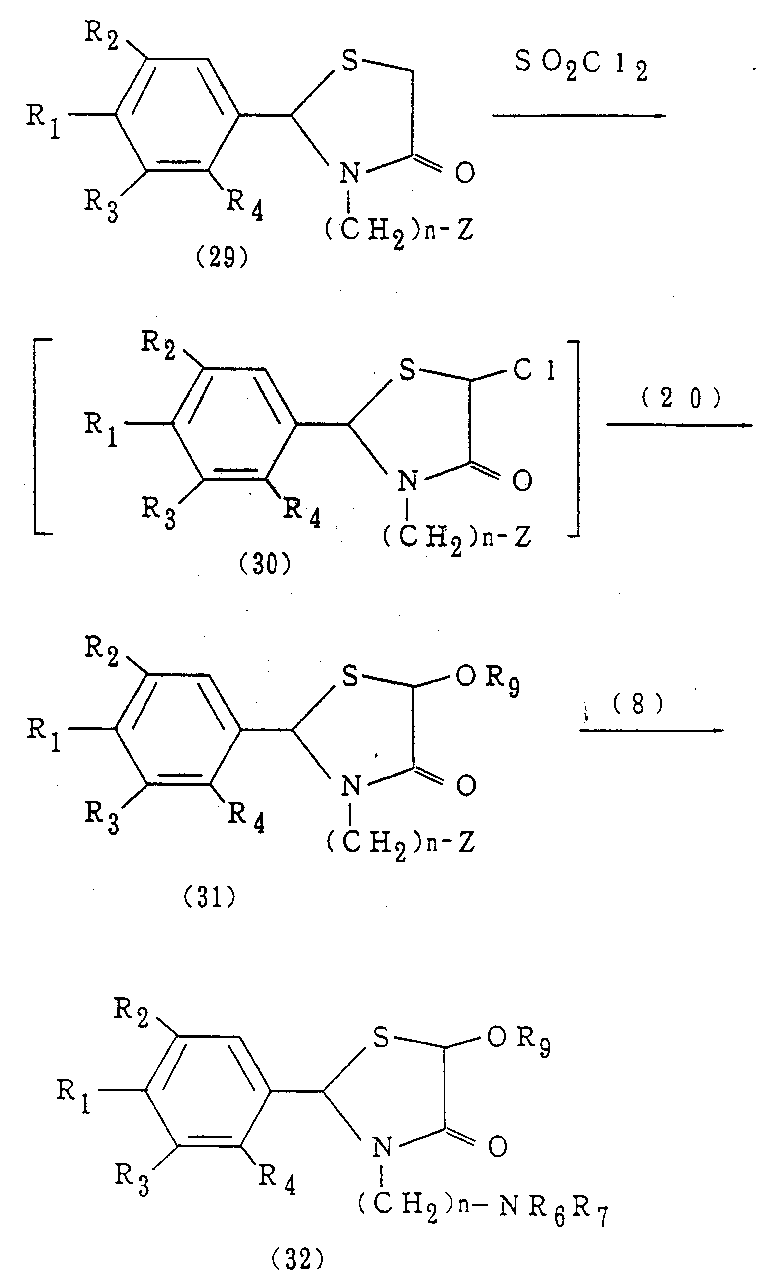

In the scheme of process I, R₁, R₂, R₃, R₄, R₅, R₆, R₇, Z, and n are the same as defined previously; and R₉ represents a substituted or unsubstituted lower alkyl group having 1 to 6 carbon atoms.

### Process J:

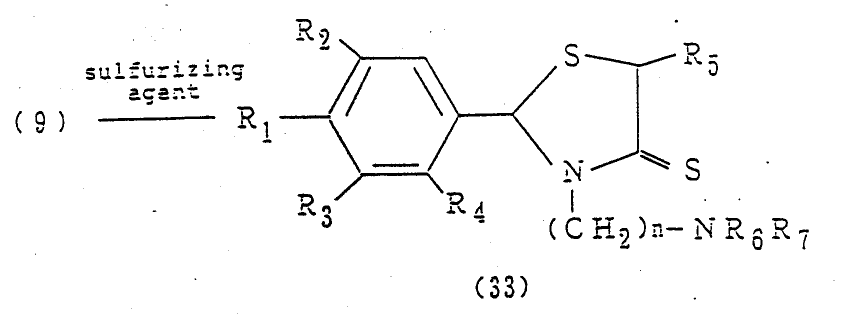

In the scheme of process J, R₁, R₂, R₃, R₄, R₅, R₆, R₇, and n are the same as defined previously.

### Process K:

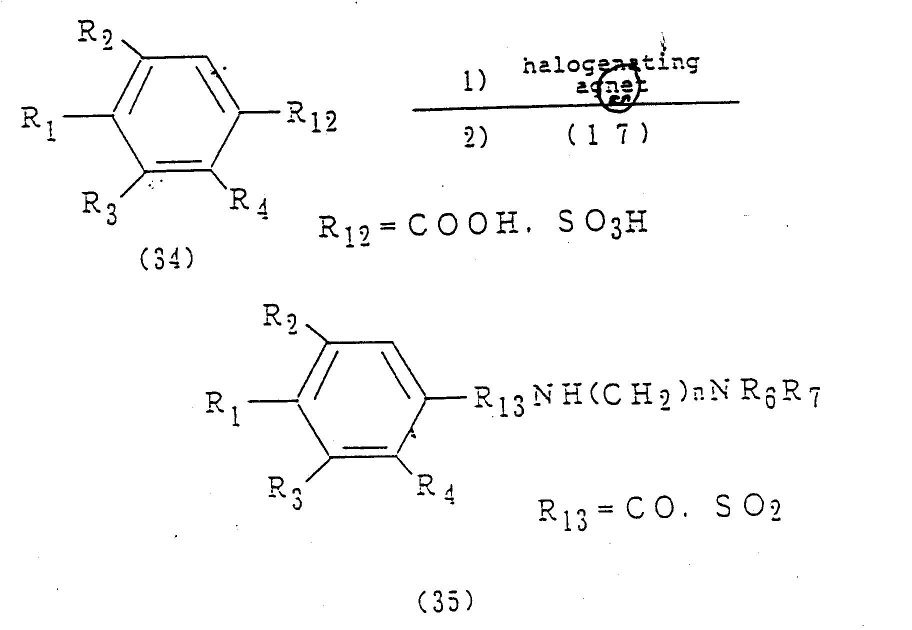

In the scheme of process K, R₁, R₂, R₃, R₄, R₅, R₆, R₇, and n are the same as defined previously; R₁₂ represents a carboxyl group or a sulfonyl group; and R₁₃ represents a carbonyl group or a sulfonyl group.

### Process L:

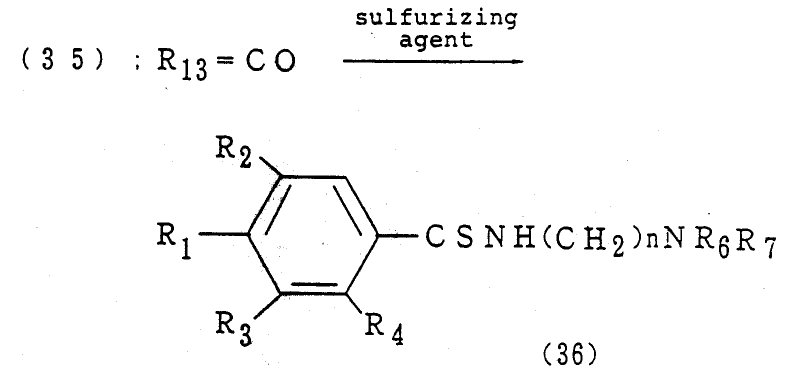

In the scheme of process L, R₁, R₂, R₃, R₄, R₅, R₆, R₇, and n are the same as defined previously; and R₁₃ represents a carbonyl group.

### Process M:

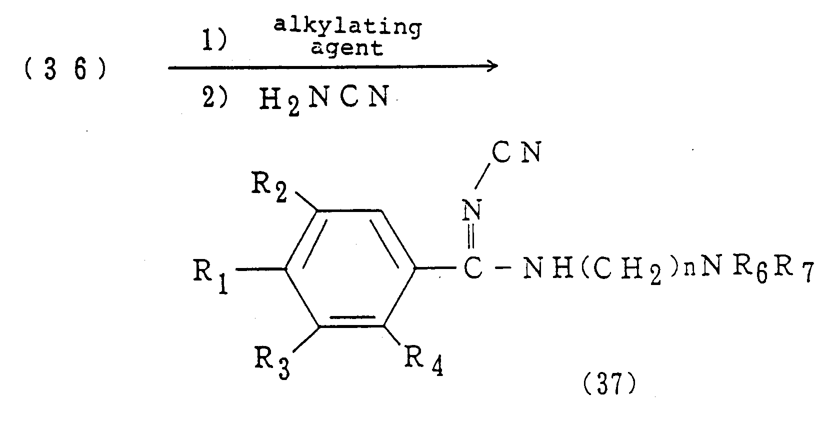

In the scheme of process M, R₁, R₂, R₃, R₄, R₅, R₆, R₇, and n are the same as defined previously.

### Process N:

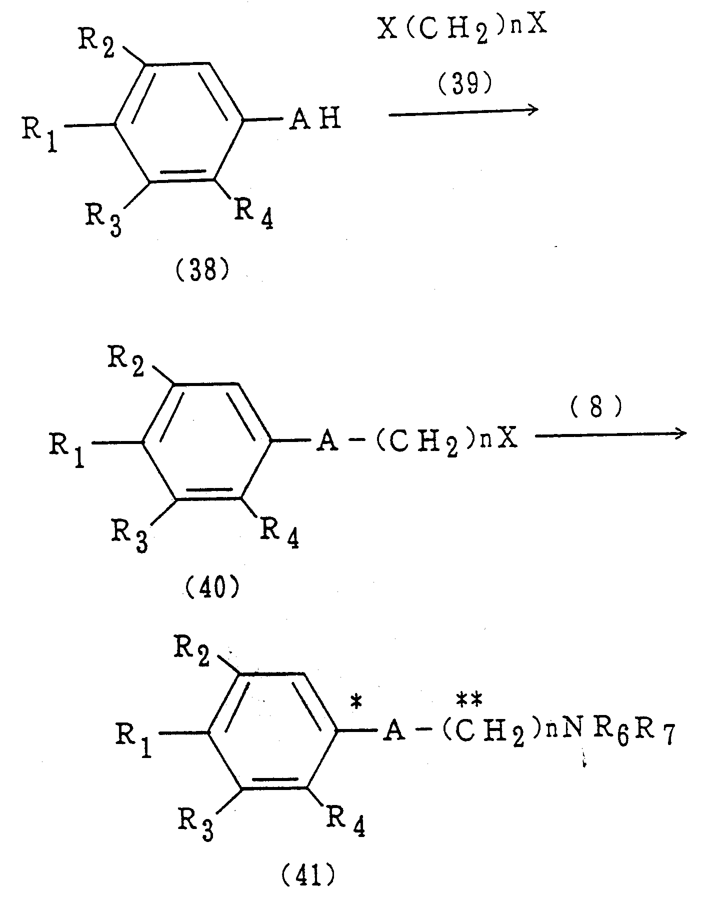

In the scheme of process N, R₁, R₂, R₃, R₄, R₅, R₆, R₇, and n are the same as defined previously; A represents a fragment selected from the group of following fragments represented by formulae (VI), (VIII), (XI), (XV), and (XVI): 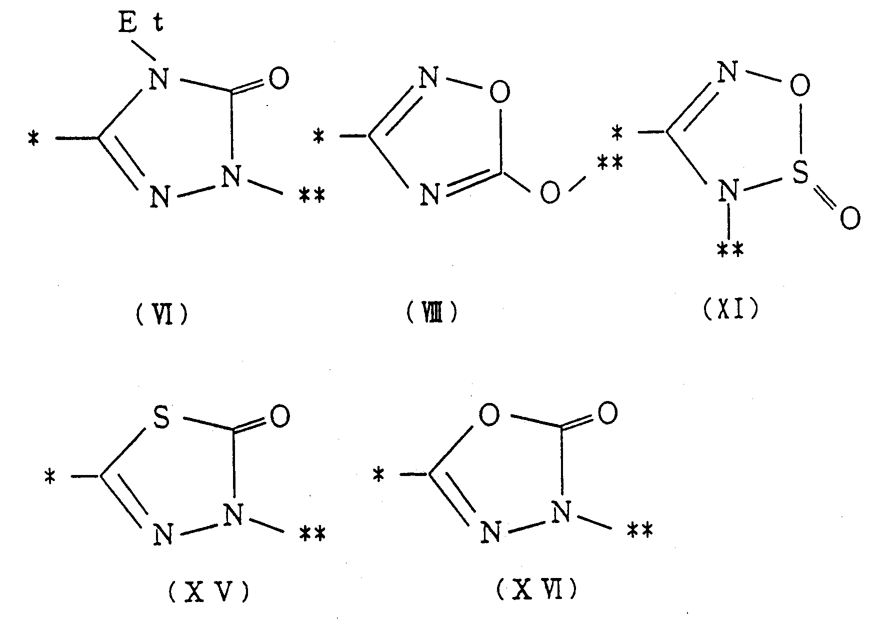 and X represents a chlorine atom or a bromine atom.

### Process O:

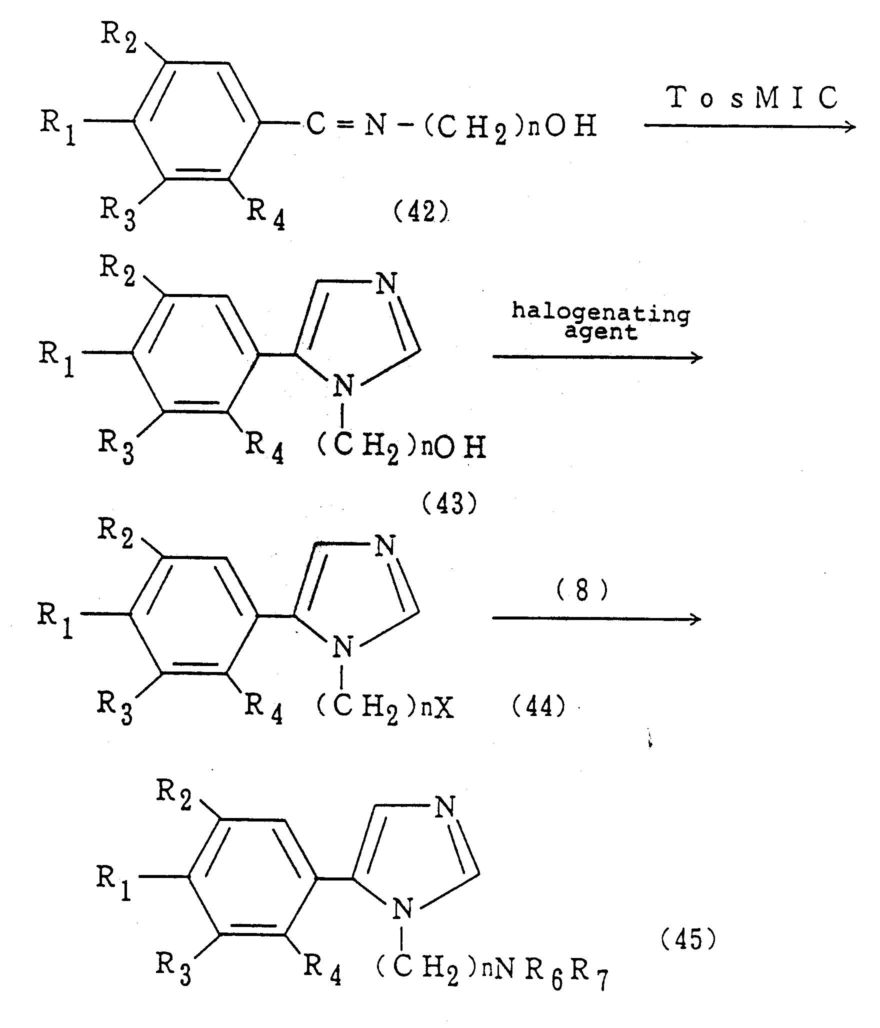

In the scheme of process O, R₁, R₂, R₃, R₄, R₅, R₆, R₇, n, and X are the same as defined previously.

### Process P:

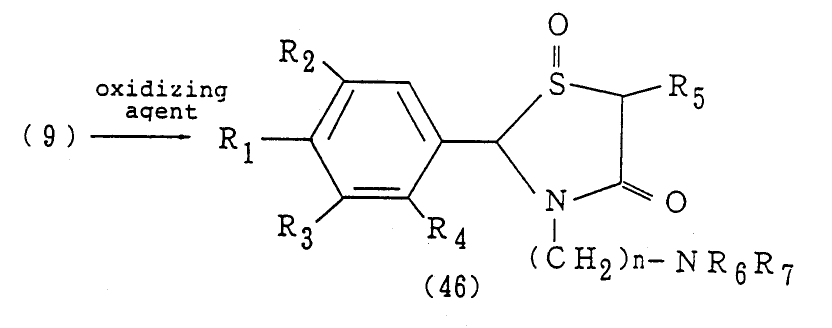

In the scheme of process P, R₁, R₂, R₃, R₄, R₅, R₆, R₇, and n are the same as defined previously.

### Process Q:

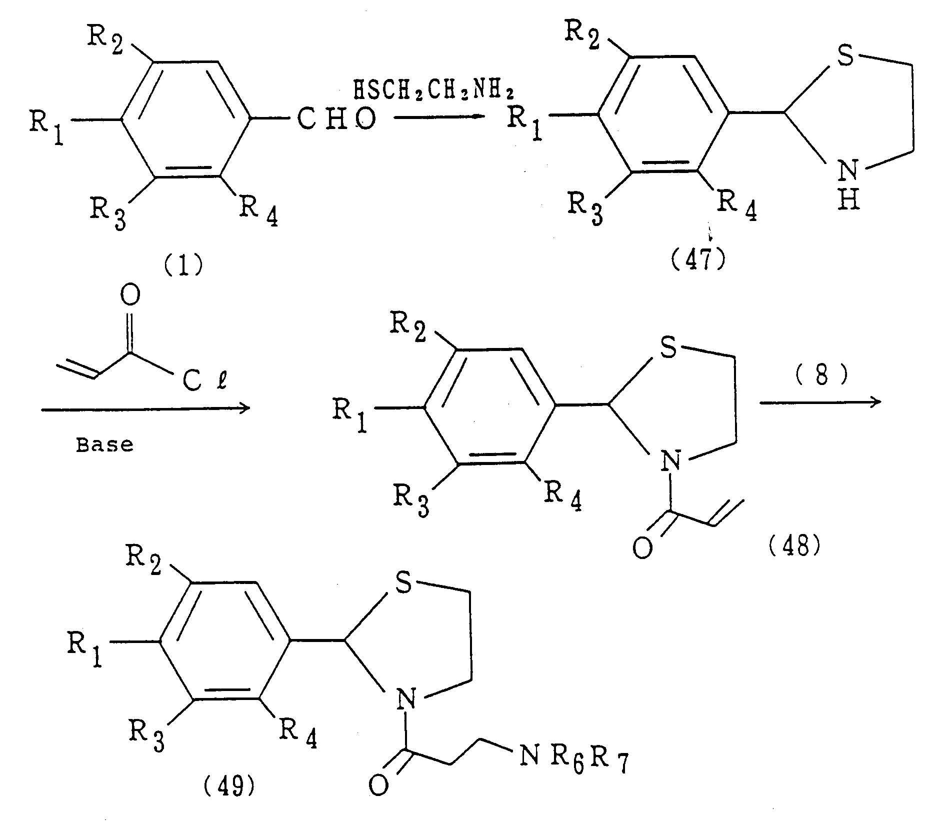

In the scheme of process Q, R₁, R₂, R₃, R₄, R₅, R₆, and R₇ are the same as defined previously.

The reaction conditions adopted in each of processes A to Q are tabulated below.

### Process A:

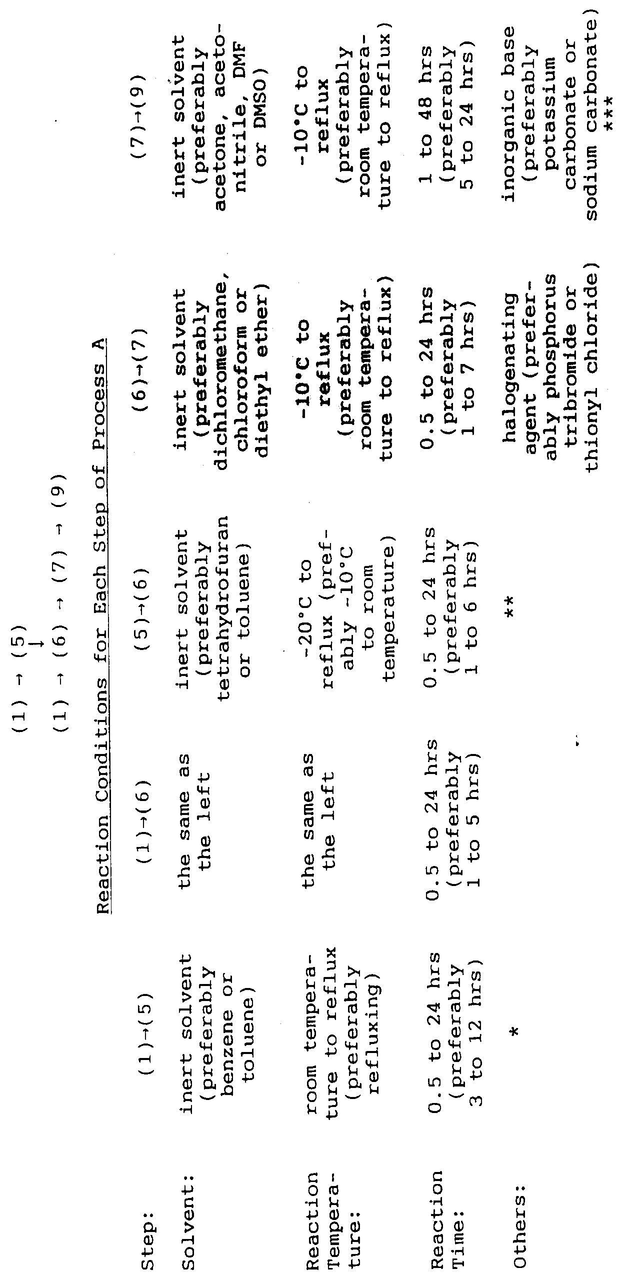

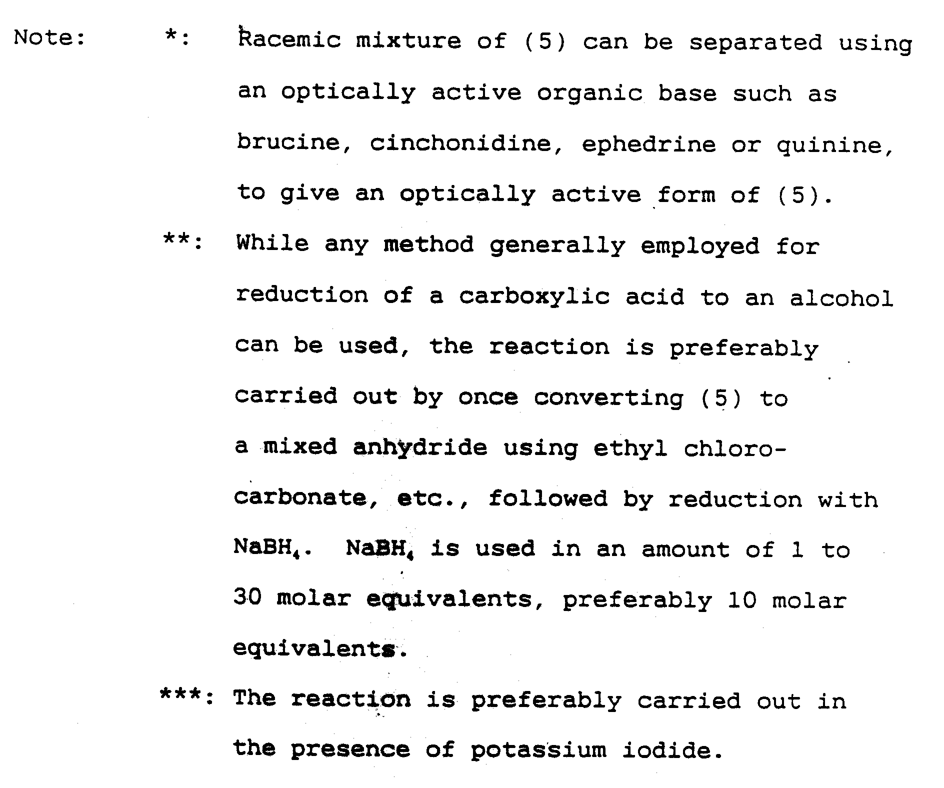

### Process B:

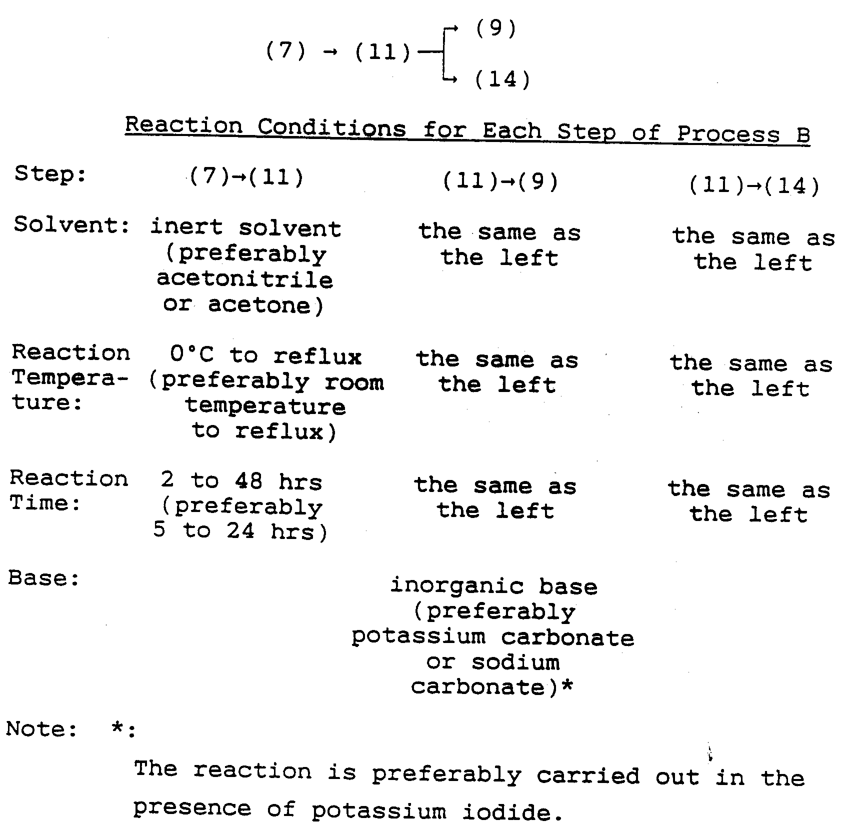

### Process C:

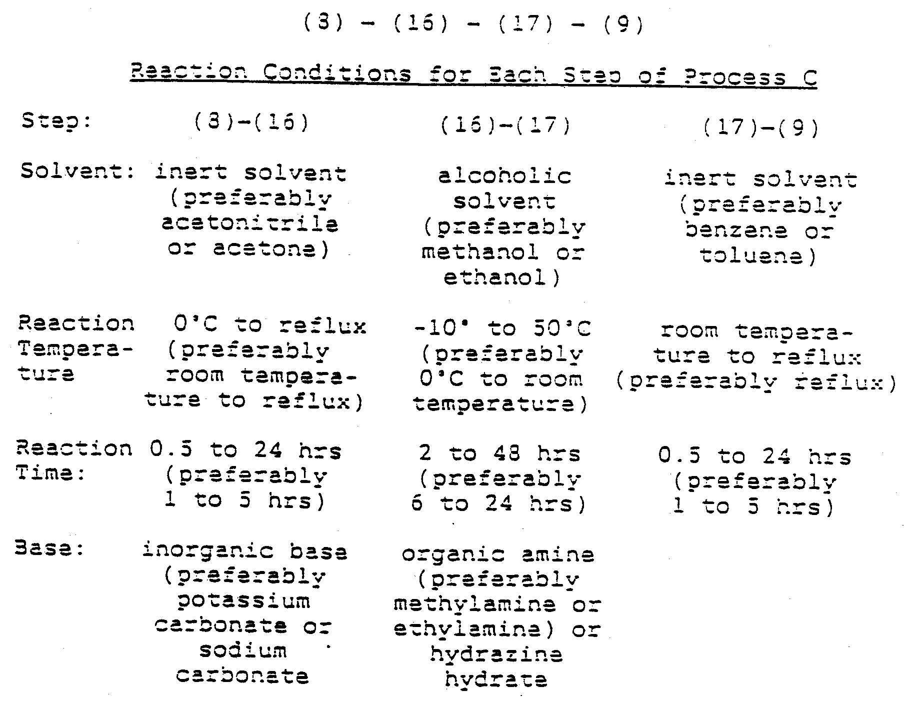

### Process D:

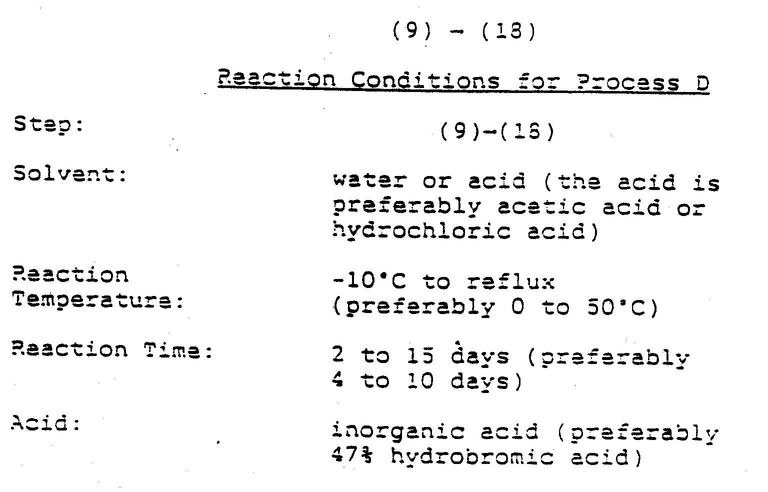

### Process E:

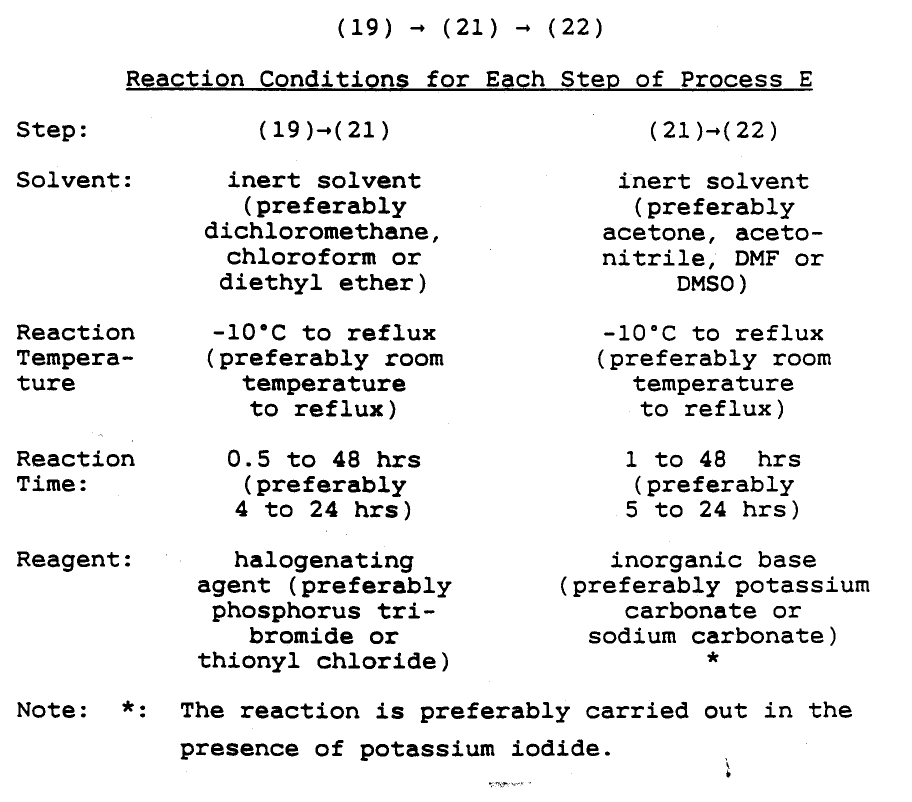

### Process F:

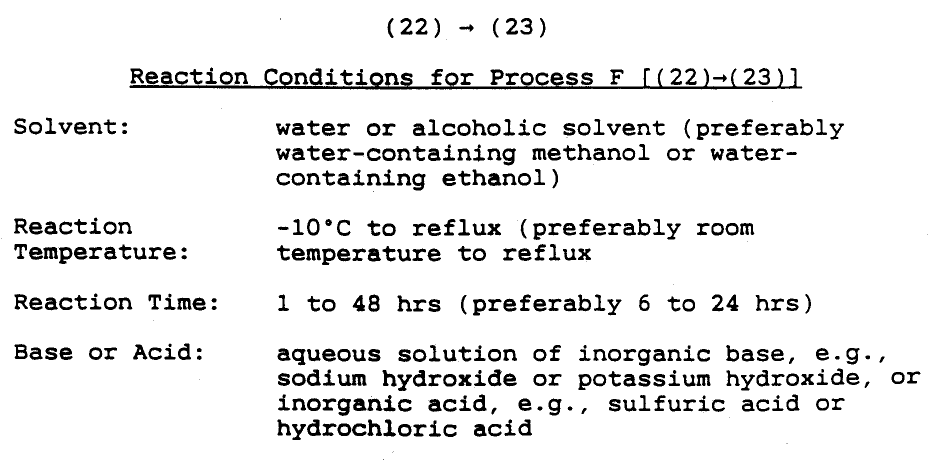

### Process G:

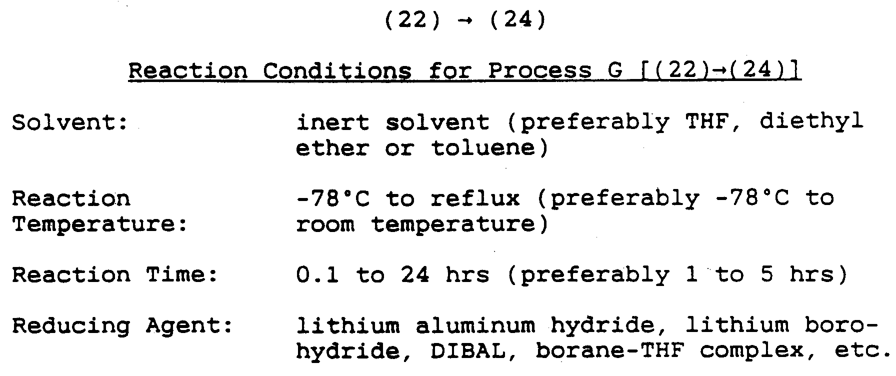

### Process H:

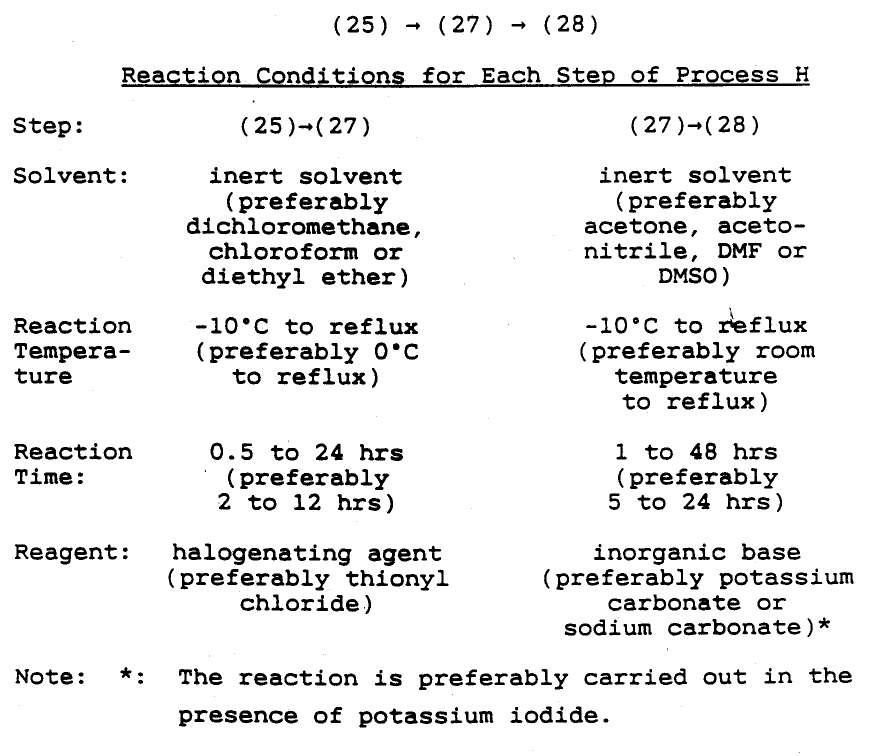

### Process I:

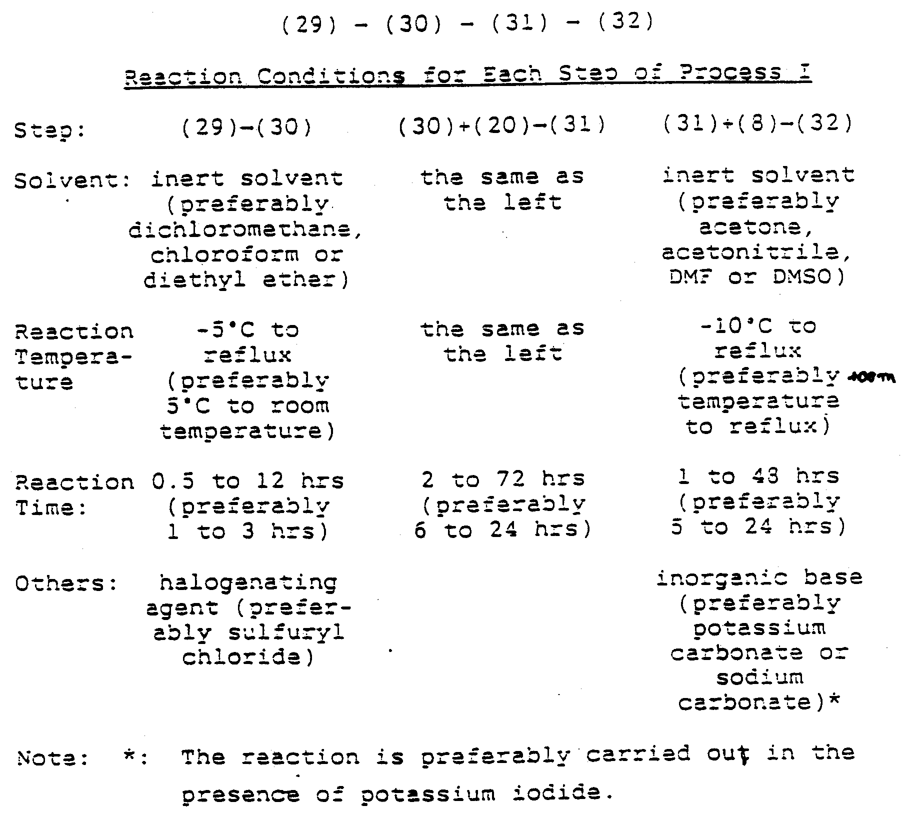

### Process J:

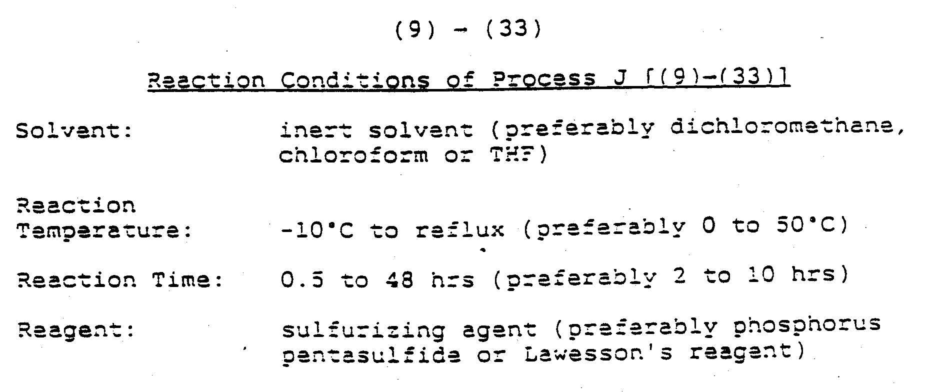

### Process K:

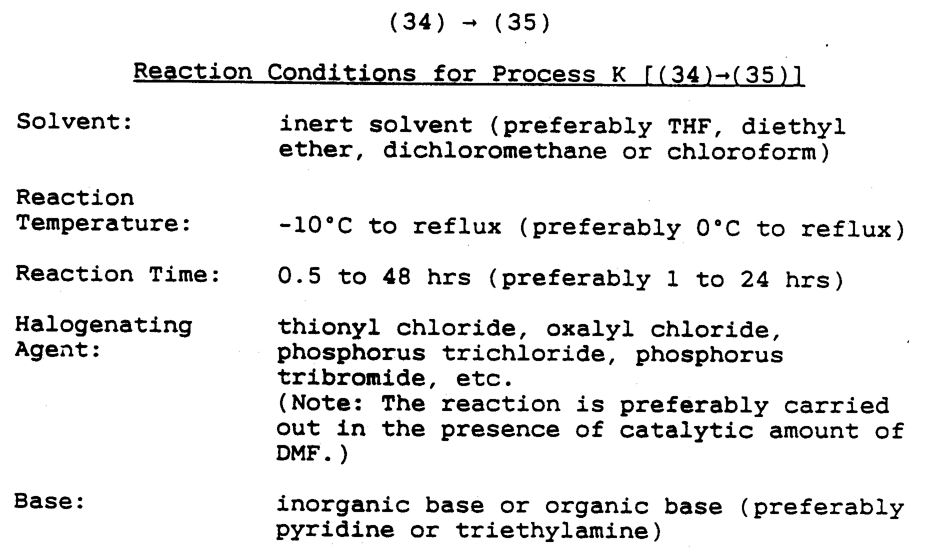

### Process L:

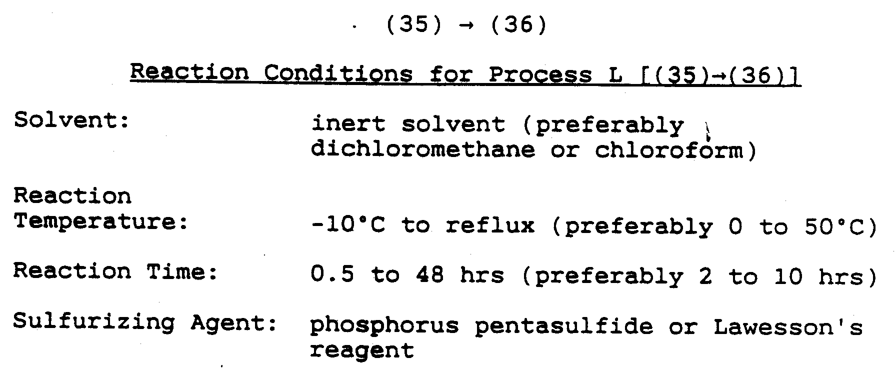

### Process M:

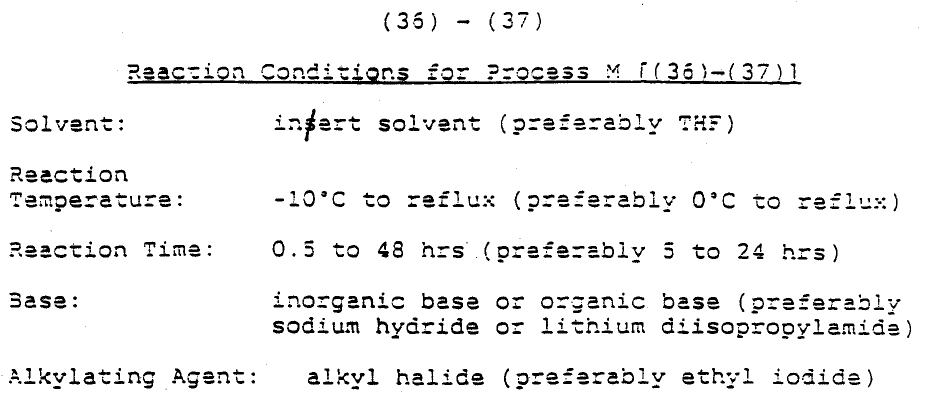

### Process N:

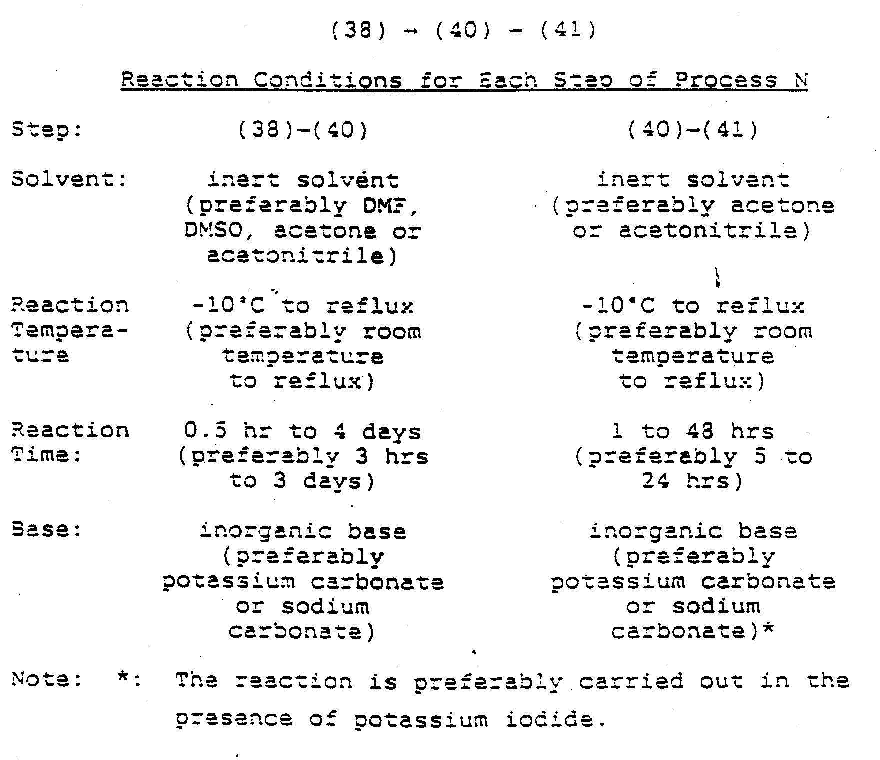

### Process O:

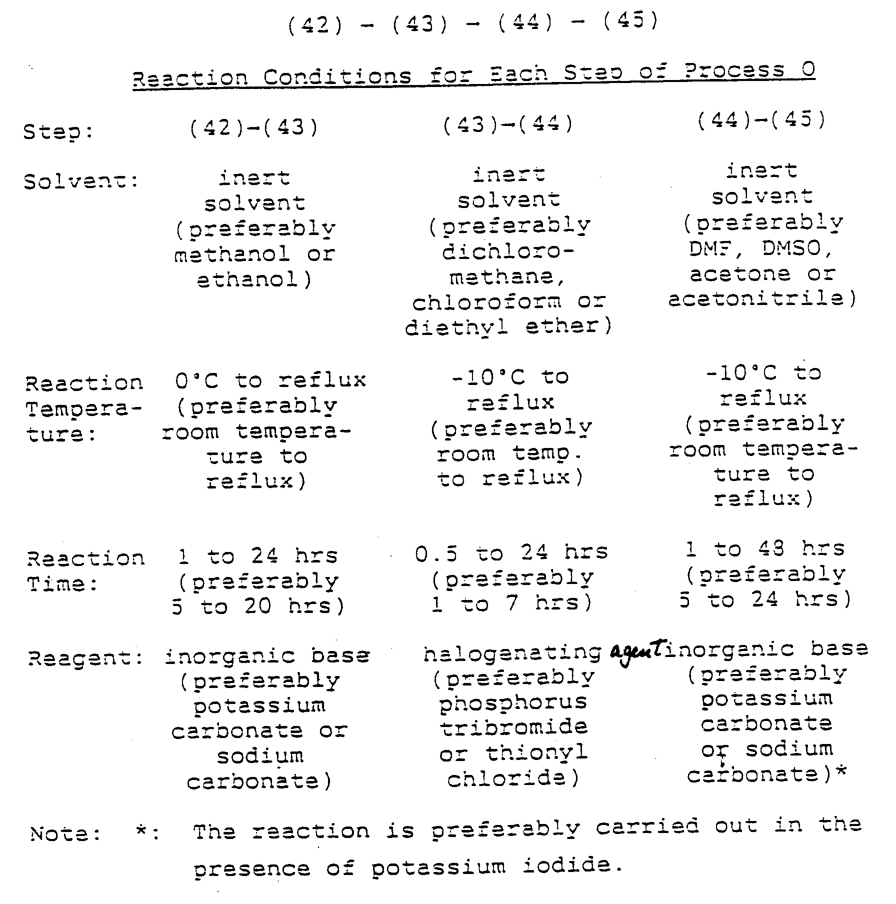

### Process P:

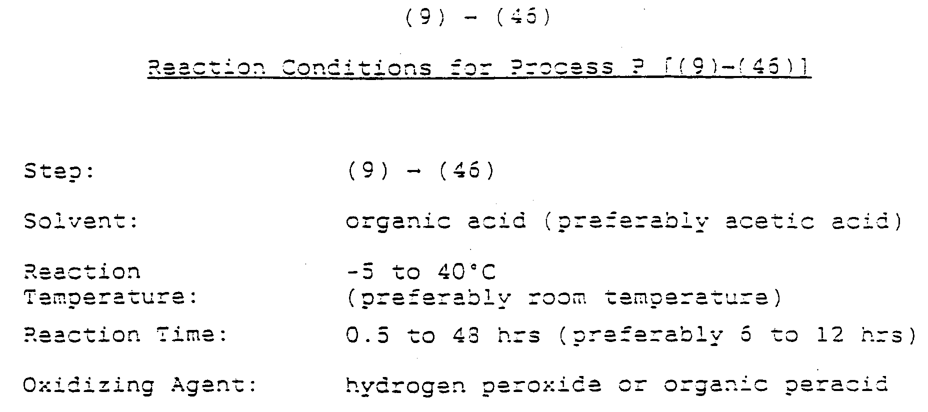

### Process Q:

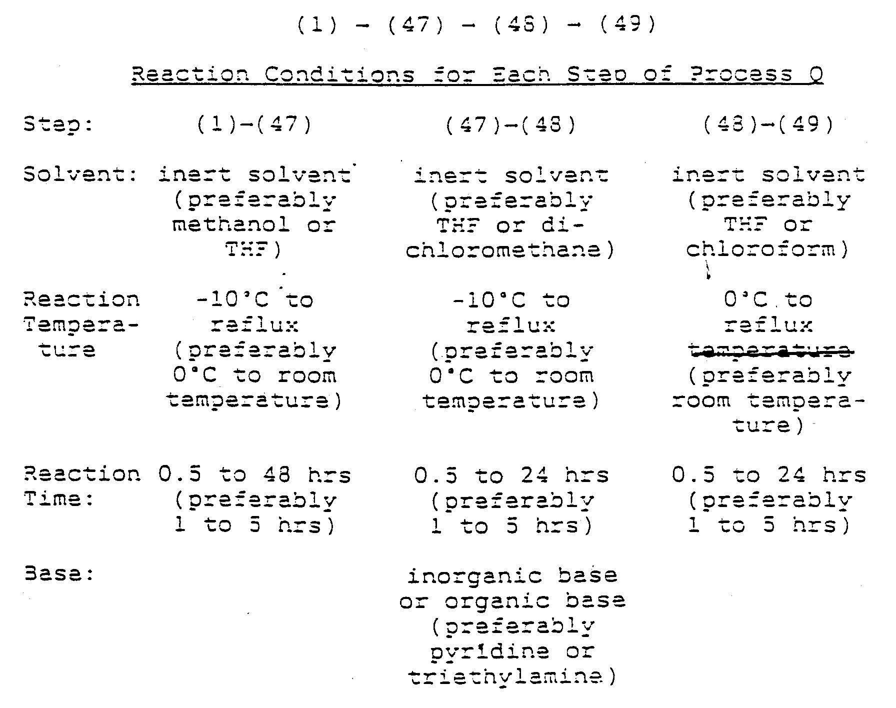

The compound of formula (I) according to the present invention contains one or two asymmetric carbon atoms in the structure thereof, and the pure stereoisomers or optical isomers can be obtained by methods known in the art. For example, each enantiomer can be separated by chromatography using a HPLC column for optical resolution or by fractional crystallization utilizing an optically active acid, preferably (R)-(-)-1,1'-binaphthyl-2,2'-diyl hydrogen phosphate. The above-mentioned techniques for separation of optical isomers apply to not only a final product but also an intermediate having a carboxyl group. In the latter case, commonly used optically active bases such as brucine can be employed. Similarly, diastereomeric mixtures comprising both cis- and trans-stereoisomers can be separated to each optical isomer, i.e., cis(+), cis(-), trans(+) and trans(-), by conventional methods known to those skilled in the art.

As a matter of course, the stereoisomers and optical isomers of the compound of formula (I) are also included in the scope of the present invention.

### Examples

The present invention will now be illustrated in greater detail by referring to reference examples, examples, and pharmacological test examples.

In the following reference examples, examples, and tables 1 to 24 inclusive the NMR data were measured with JEOL JNM-FX200 or JEOL JNM-EX270 except those with asterisk, in which case the measurements were carried out on Hitachi R-24B (60 MHz).

### REFERENCE EXAMPLE 1

### Preparation of 2-(3,5-Diisopropyl-4-hydroxyphenyl)-3-(3-hydroxypropyl)-1,3-thiazolidin-4-one

In benzene (50 ml) were suspended 3,5-diisopropyl-4-hydroxybenzaldehyde (5.00 g) and 3-aminopropanol (1.82 g) in a nitrogen atmosphere. A Dean-Stark trap was fitted to the reactor, and the suspension was refluxed for 1.5 hours. After allowing the mixture to cool, α-mercaptoacetic acid (2.23 g) was added, then the mixture was further refluxed for 2 hours. After removal of benzene by evaporation, water (50 ml) was added to the residue, and the mixture was extracted with chloroform. The organic layer was dried over anhydrous sodium sulfate, and the solvent was removed under reduced pressure. The residue was purified by silica gel column chromatography (eluent; chloroform-methanol, 99:1) to afford 1.97 g (24%) of the title compound as a colorless oil.
- NMR (CDCl₃, 60 MHz):: 1.23 (12H, d, J=6.6Hz), 1.0-1.8 (2H, m), 2.5-3.8 (7H, m), 3.73 (2H, brs), 5.50 (2H, brs), 6.92 (2H, s)

Each alcohol shown in Tables 1 to 3 was prepared according to the procedure for Reference Example 1, using an appropriate substituted benzaldehyde and a corresponding ω-aminoalkyl alcohol instead of 3,5-diisopropyl-4-hydroxybenzaldehyde and 3-aminopropanol in each case.

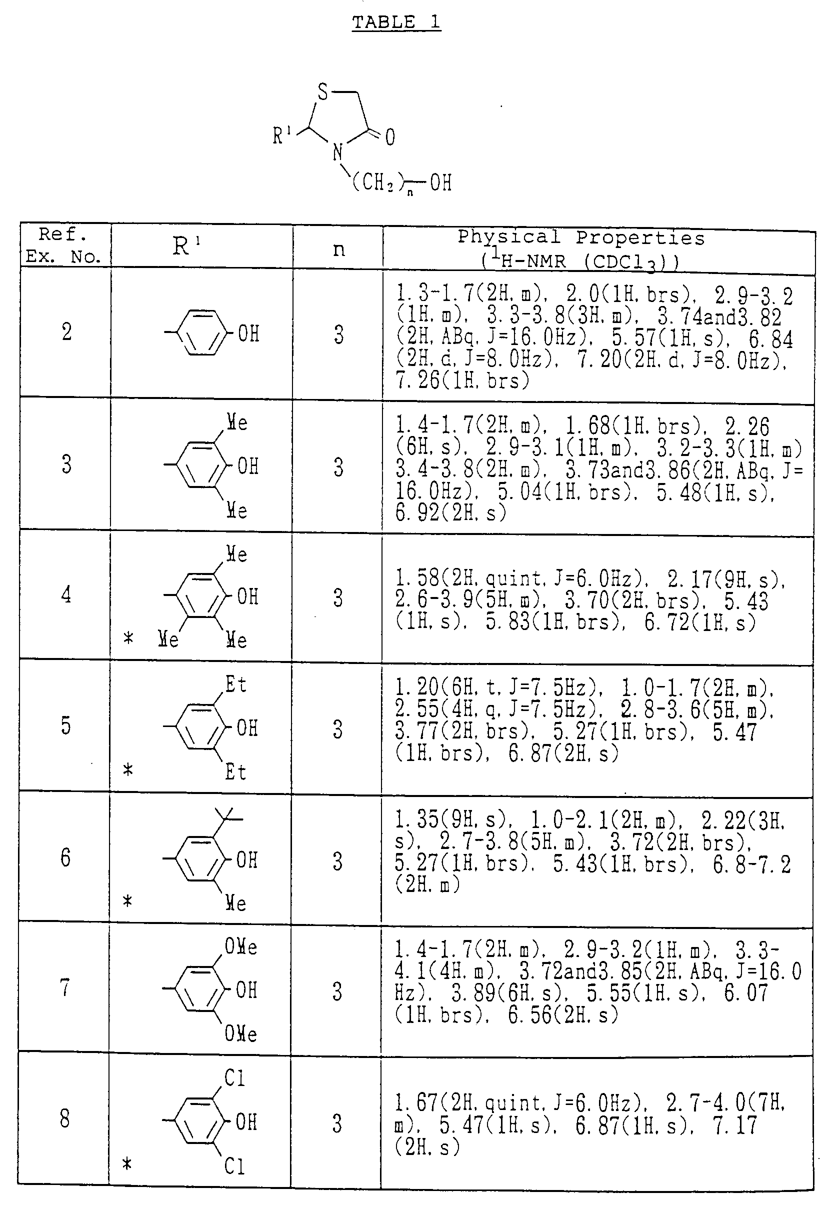

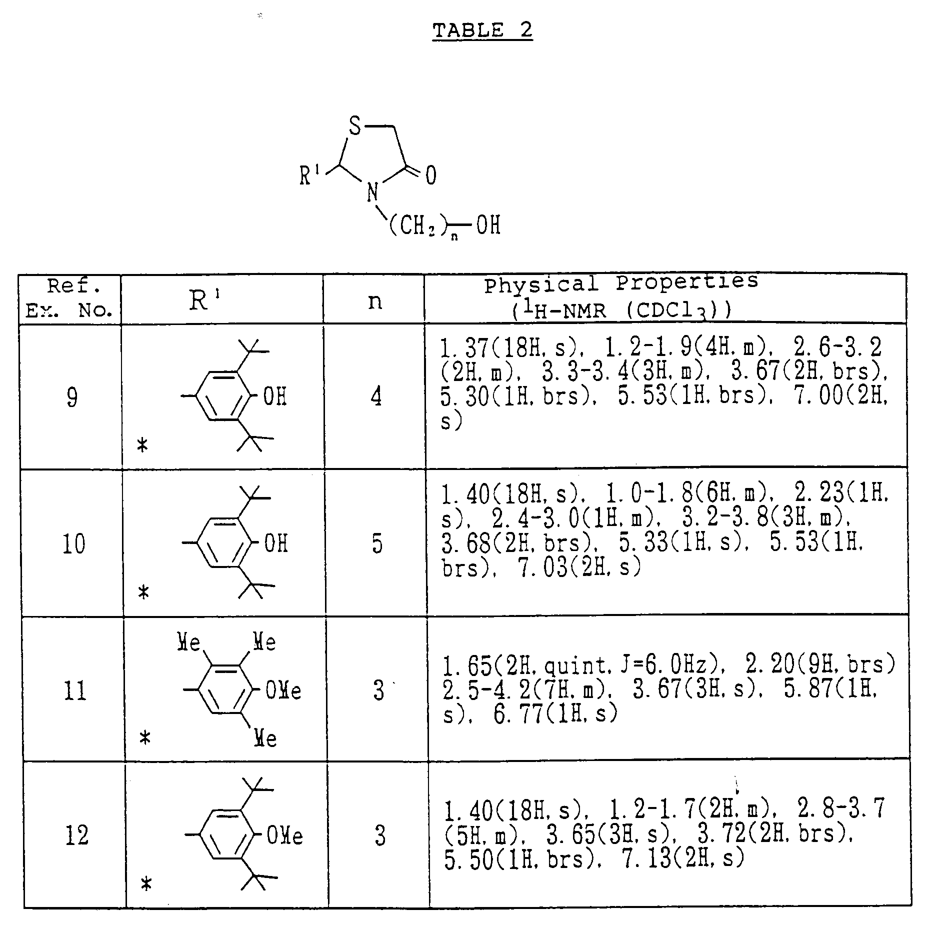

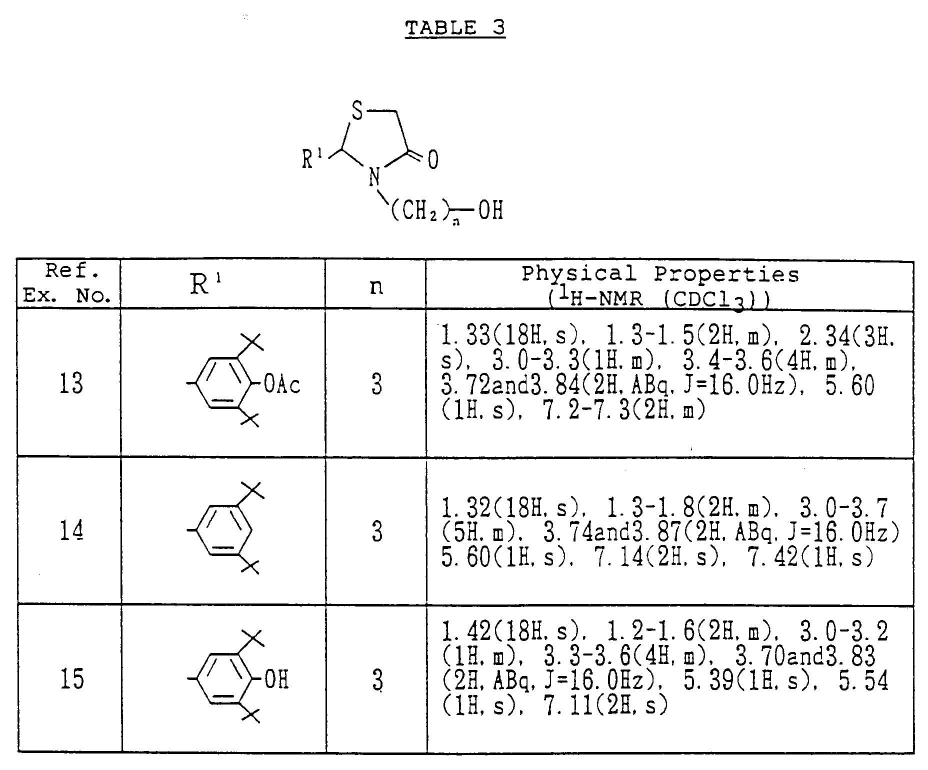

### REFERENCE EXAMPLE 16

### Preparation of 2-(3,5-Di-tert-butyl-4-hydroxyphenyl)-3-(3-hydroxypropyl)-1,3-thiazolidin-4-one

In benzene (500 ml) were suspended 3,5-di-tert-butyl-4-hydroxybenzaldehyde (50.0 g) and β-alanine (20.0 g) under a nitrogen atmosphere. A Dean-Stark trap was fitted to the reactor, and the suspension was refluxed for 1 hour. After allowing the mixture to cool, α-mercaptoacetic acid (23.6 g) was added, then the mixture was further refluxed for 24 hours. After removal of benzene by evaporation, water (500 ml) was added to the residue, and the mixture was extracted with chloroform. The organic layer was dried over anhydrous sodium sulfate, and the solvent was removed under reduced pressure. The residue was purified by silica gel column chromatography (eluent; chloroform-methanol, 98:2) to afford 54.6 g (67%) of 2-(3,5-di-tert-butyl-4-hydroxyphenyl)-3-(2-carboxyethyl)-1,3-thiazolidin-4-one as colorless crystals. mp 164-165°C.
- NMR (CDCl₃, 200 MHz) δ:: 1.42 (18H, s), 2.2-2.5 (1H, m), 2.5-2.8 (1H, m), 3.0-3.3 (1H, m), 3.5-4.0 (3H, m), 5.33 (1H, s), 5.64 (1H, s), 7.09 (2H, s), 8.5 (1H, brs)

To a solution of the resulting 2-(3,5-di-tert-butyl-4-hydroxyphenyl)-3-(2-carboxyethyl)-1,3-thiazolidin-4-one (100 mg) in tetrahydrofuran (3 ml) were added dropwise triethylamine (27 mg) and ethyl chloroformate (28 mg) at -10°C under a nitrogen atmosphere, followed by stirring at -10 to -5°C for 1 hour. To the mixture was added sodium borohydride (100 mg), the mixture was stirred at room temperature for 3 hours, then poured into ice-water and extracted with ethyl acetate. The organic layer was dried over anhydrous sodium sulfate, and the solvent was evaporated under reduced pressure. The residue was purified by silica gel column chromatography (eluent; chloroform-methanol, 98:2) to afford 70 mg (73%) of the product which was identical with that of Reference Example 15.

### REFERENCE EXAMPLE 17

### Preparation of 2-(3,5-Diisopropyl-4-hydroxyphenyl)-3-(3-chloropropyl)-1,3-thiazolidin-4-one

To a dichloromethane (50 ml) solution of the 2-(3,5-diisopropyl-4-hydroxyphenyl)-3-(3-hydroxypropyl)-1,3-thiazolidin-4-one (1.97 g) obtained in Reference Example 1 was added thionyl chloride (1.04 g) under a nitrogen atmosphere, then the mixture was refluxed for 1 hour. The solvent was evaporated under reduced pressure, and to the residue were added brine and chloroform. The organic layer was separated and dried over anhydrous sodium sulfate, and the solvent was evaporated under reduced pressure. The residue was purified by silica gel column chromatography (eluent; dichloromethane) to afford 1.25 g (60%) of the title compound as pale yellow crystals. mp 105-106°C.
- NMR (CDCl₃, 60 MHz) δ:: 1.23 (12H, d, J=6.6Hz), 1.5-2.1 (2H, m), 2.6-3.8 (6H, m), 3.67 (2H, brs), 5.20 (1H, s), 5.50 (1H, brs), 6.88 (2H, s)

### REFERENCE EXAMPLES 18 TO 28

Each compound shown in Tables 4 and 5 was prepared according to the procedure for Reference Example 17 using an appropriate alcohol in each case.

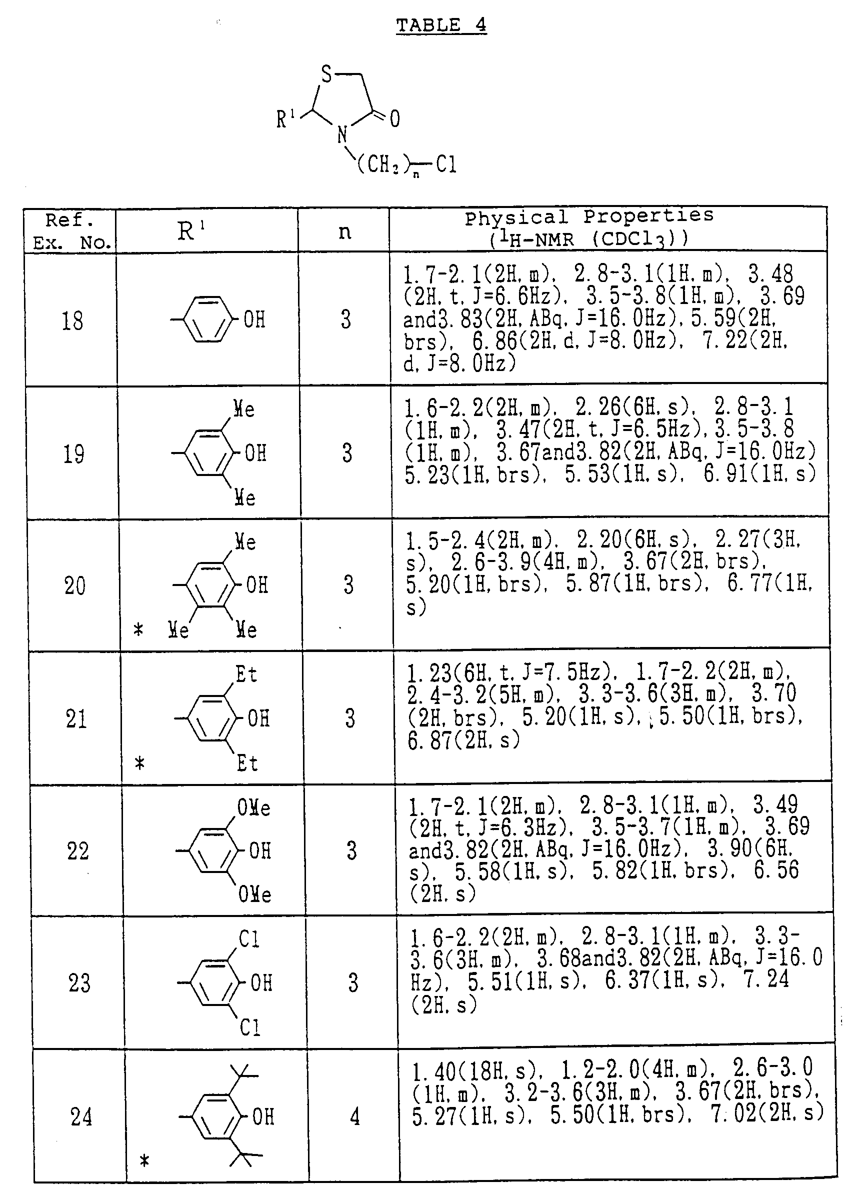

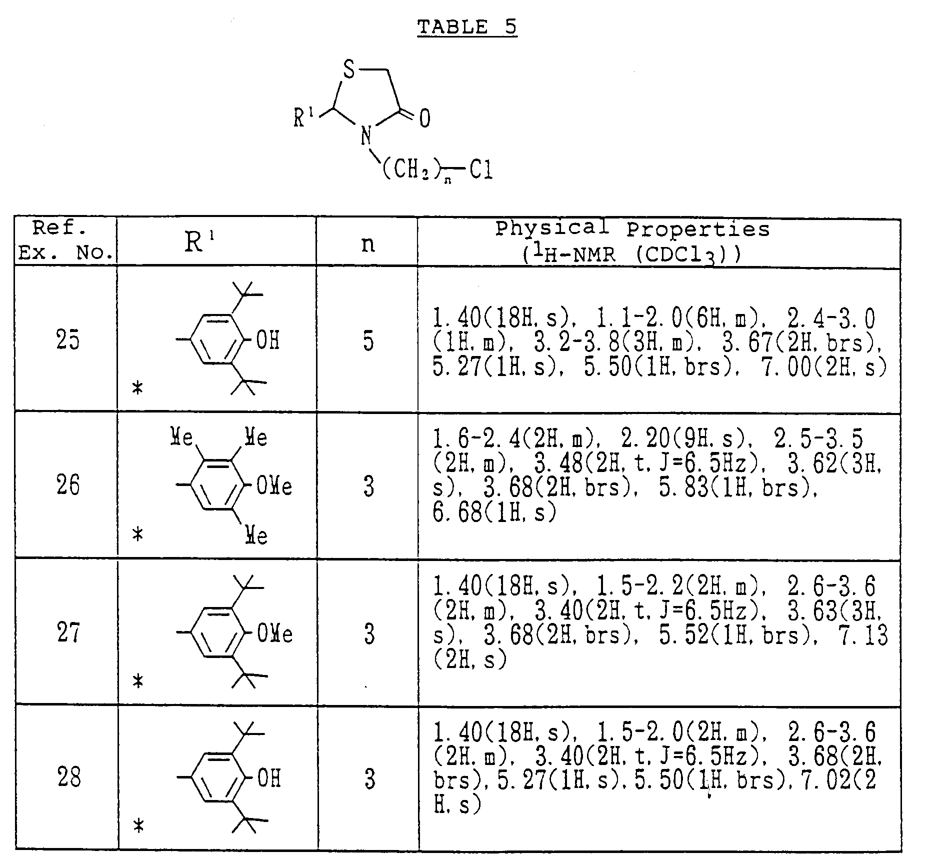

### REFERENCE EXAMPLE 29

### Preparation of 2-(3,5-Di-tert-butyl-4-hydroxyphenyl)-3-(3-bromopropyl)-1,3-thiazolidin-4-one

To a solution of 2-(3,5-di-tert-butyl-4-hydroxyphenyl)-3-(3-hydroxypropyl)-1,3-thiazolidin-4-one (2.00 g) obtained in Reference Example 15 in diethyl ether (20 ml) was added phosphorus tribromide (0.74 g) under a nitrogen atmosphere, then the mixture was stirred at room temperature for 6 hours. After completion of the reaction, the mixture was poured into ice-water (100 ml), and the product was extracted with diethyl ether. The organic layer was washed with brine and dried over anhydrous sodium sulfate. The solvent was evaporated under reduced pressure, and the residue was purified by silica gel column chromatography (eluent; chloroform) to afford 1.31 g (56%) of the title compound as pale yellow crystals. mp 130-131°C.
- NMR (CDCl₃, 60 MHz) δ:: 1.43 (18H, s), 1.6-2.2 (2H, m), 2.6-3.6 (2H, m), 3.28 (2H, t, J=6.5Hz), 3.70 (2H, brs), 5.28 (1H, s), 5.53 (1H, brs), 7.05 (2H, s)

Each bromide shown in Table 6 was prepared according to the procedure for Reference Example 29, using an appropriate alcohol in each case.

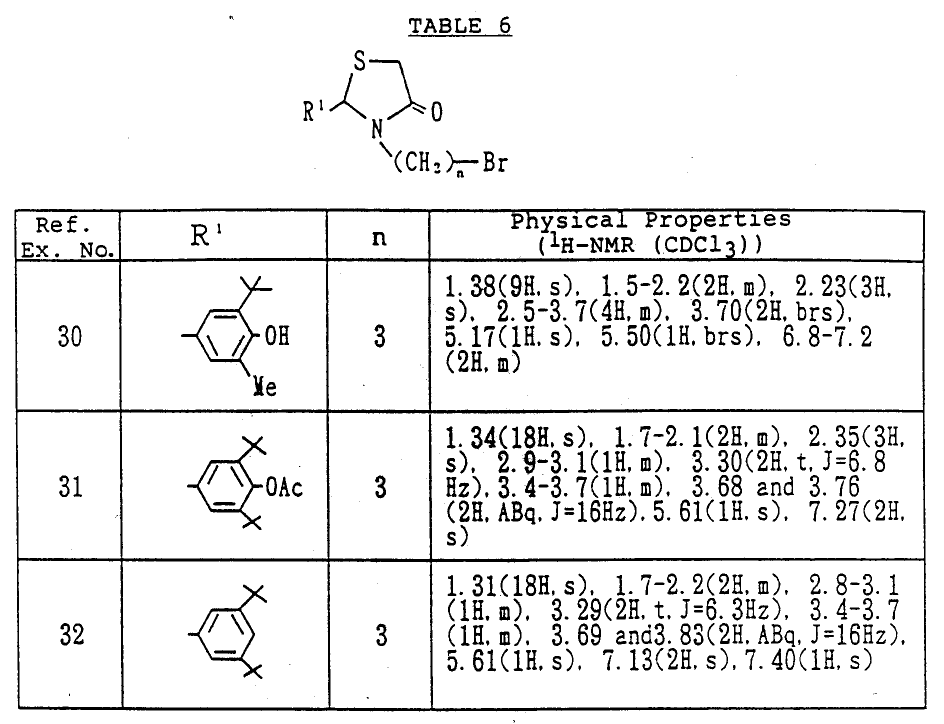

### EXAMPLE 1

### Preparation of 2-(3,5-Diisopropyl-4-hydroxyphenyl)-3-[3-[N-methyl-N-[2-(3,4-methylenedioxyphenoxy)ethyl]amino]propyl]-1,3-thiazolidin-4-one

To a solution of 2-(3,5-diisopropyl-4-hydroxyphenyl)-3-(3-chloropropyl)-1,3-thiazolidin-4-one (0.50 g) obtained in Reference Example 17 and N-methyl-N-[2-(3,4-methylenedioxyphenoxy)ethyl]amine (0.30 g) in dimethylformamide (10 ml) were added sodium carbonate (0.29 g) and potassium iodide (0.30 g) under a nitrogen atmosphere, and the mixture was stirred at 80°C for 24 hours. The solvent was removed by evaporation under reduced pressure, water (20 ml) was added to the residue, and the mixture was extracted with chloroform. The organic layer was washed successively with water and brine, then dried over anhydrous sodium sulfate. The solvent was evaporated under reduced pressure, and the residue was purified by silica gel column chromatography (eluent; chloroform-methanol, 97:3) to afford 0.26 g (36%) of the title compound as a pale brown oil.
- NMR (CDCl₃, 60 MHz) δ:: 1.23 (12H, d, J=6.6Hz), 1.4-1.9 (2H, m), 2.17 (3H, s), 2.3-3.8 (8H, m), 3.67 (2H, brs), 3.87 (2H, t, J=5.7Hz), 5.00 (1H, brs), 5.57 (1H, s), 5.80 (2H, s), 6.0-6.7 (3H, m), 6.90 (2H, s)

### EXAMPLES 2 TO 25

Each compound shown in Tables 7 to 10 was prepared according to the procedure for Example 1, using a corresponding chloride and an appropriate amine in each case.

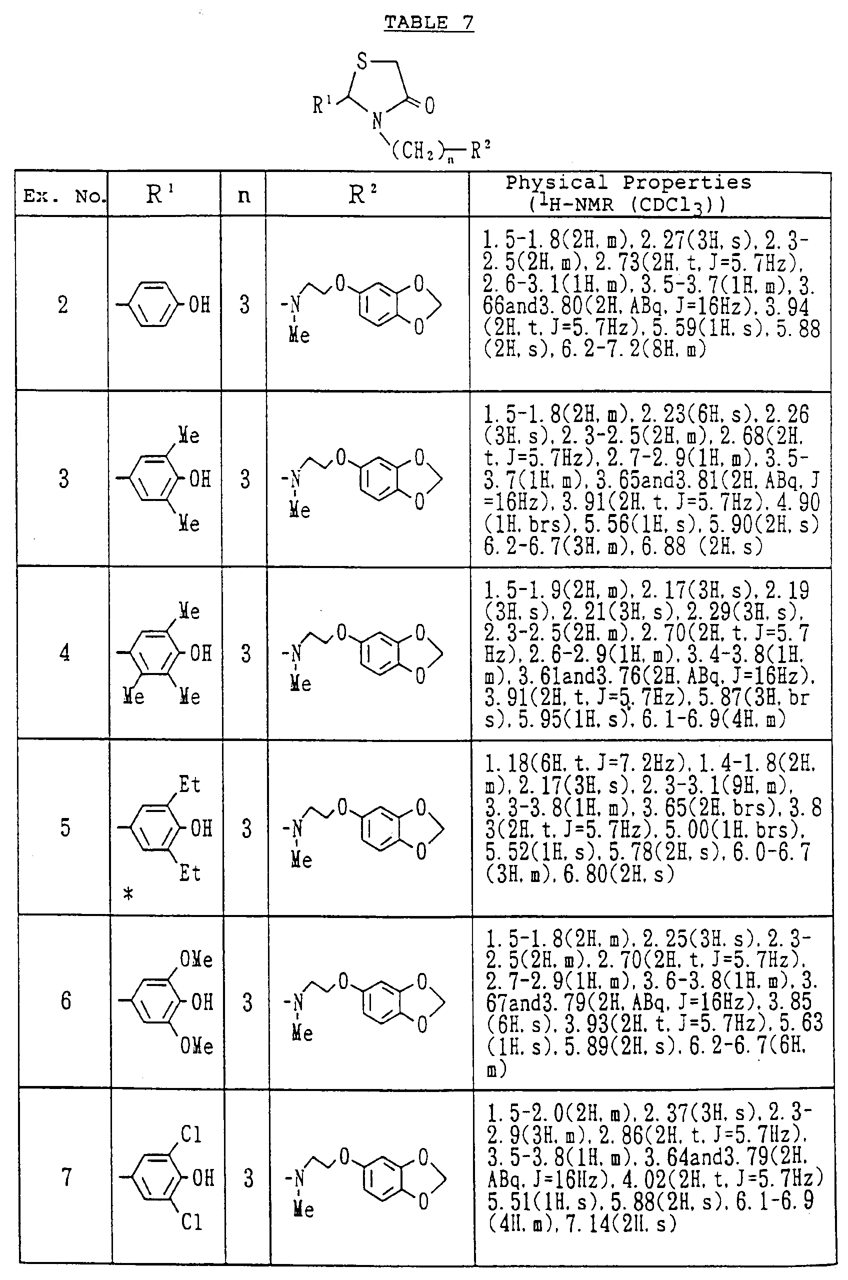

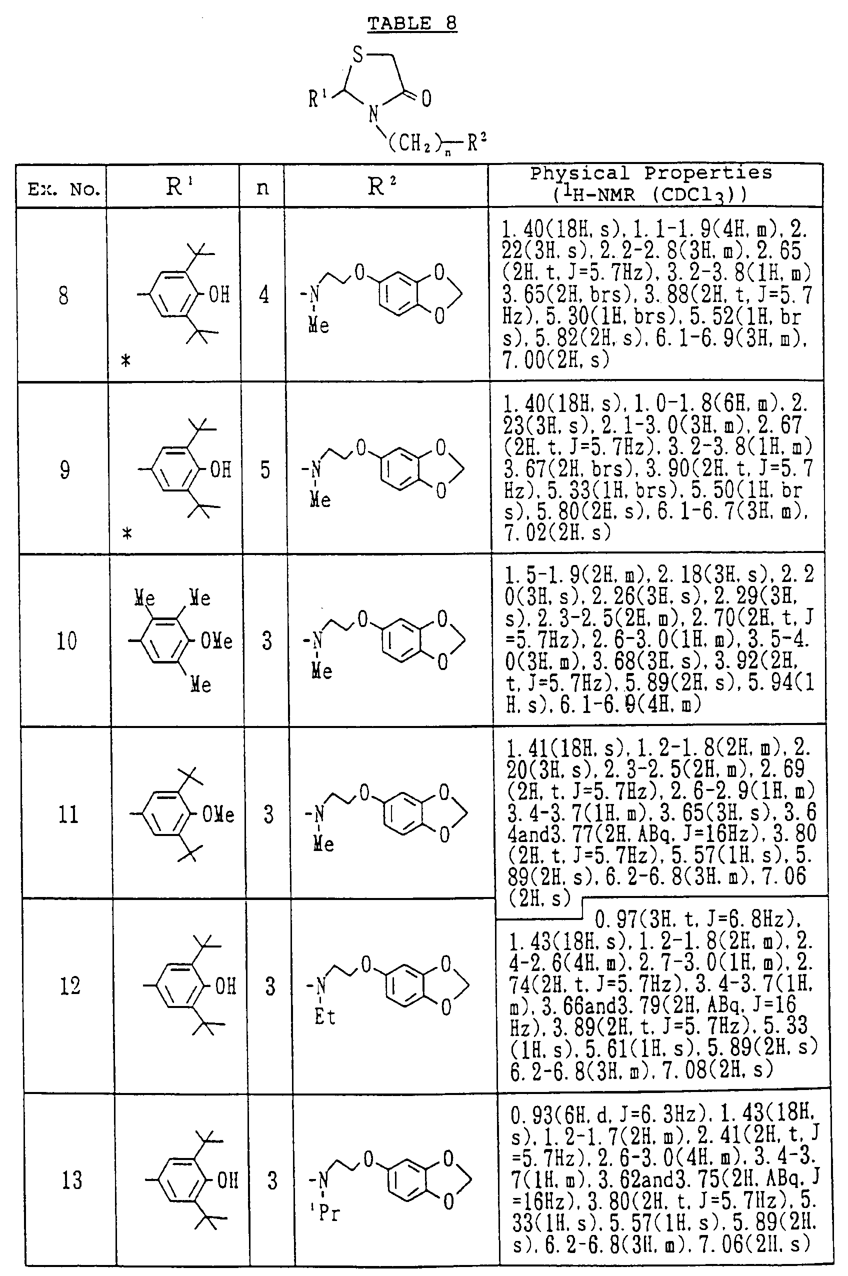

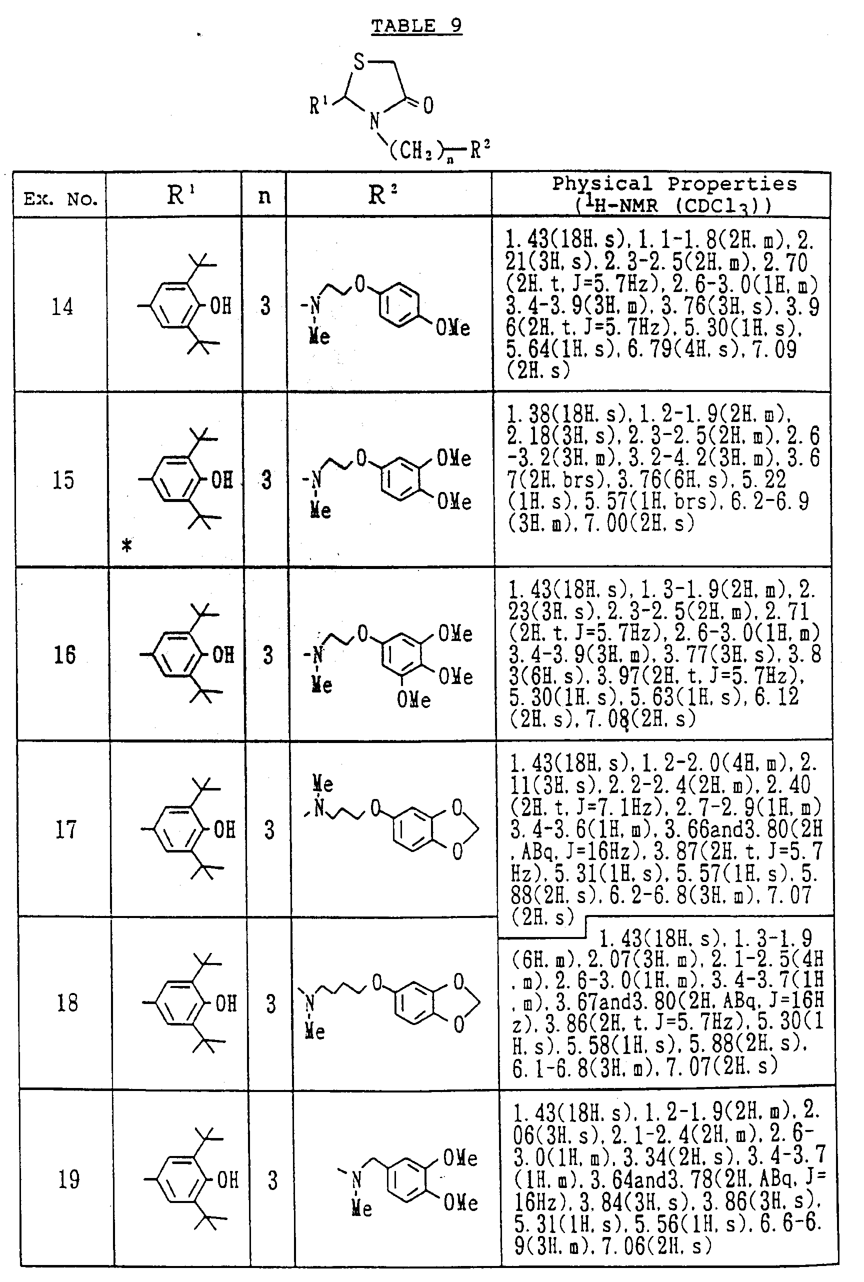

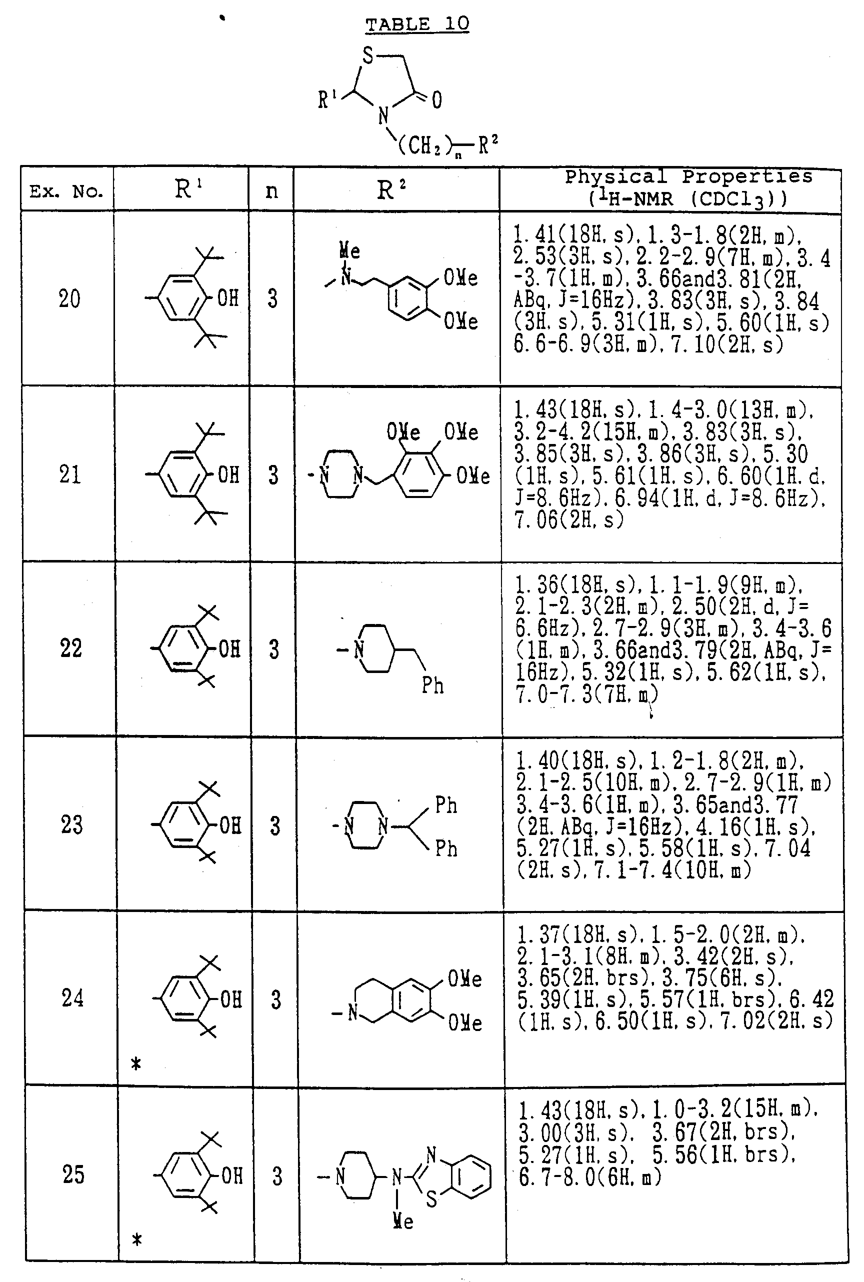

### EXAMPLE 26-A

### Preparation of 2-(3,5-Di-tert-butyl-4-hydroxyphenyl)-3-[3-[N-methyl-N-[2-(3,4-methylenedioxyphenoxy)ethyl]amino]propyl]-1,3-thiazolidin-4-one

To a solution of the 2-(3,5-di-tert-butyl-4-hydroxyphenyl)-3-(3-bromopropyl)-1,3-thiazolidin-4-one (89.3 mg) obtained in Reference Example 29 and N-methyl-N-[2-(3,4-methylenedioxyphenoxy)ethyl]amine (48.8 mg) in acetone (5 ml) was added potassium carbonate (34.6 mg) under a nitrogen atmosphere, and the mixture was refluxed for 10 hours. After allowing the mixture to cool, inorganic matter was removed by filtration, and the filtrate was concentrated under reduced pressure. The residue was purified by silica gel column chromatography (eluent; chloroform-methanol, 97:3) to afford 67.4 mg (60%) of the title compound as colorless crystals. mp 70-71°C.
- NMR (CDCl₃, 200 MHz) δ:: 1.42 (18H, s), 1.4-1.7 (2H, m), 2.20 (3H, s), 2.3-2.5 (2H, m), 2.68 (2H, t, J=5.9Hz), 2.7-2.9 (1H, m), 3.5-3.6 (1H, m), 3.66 and 3.80 (2H, ABq, J=16.0Hz), 3.92 (2H, t, J=5.9Hz), 5.32 (1H, s), 5.66 (1H, s), 5.90 (2H, s), 6.2-6.7 (3H, m), 7.09 (2H, s)

### EXAMPLES 27 TO 34

Each compound shown in Tables 11 and 12 was prepared according to the procedure for Example 26-A, using a corresponding bromide and an appropriate amine in each case.

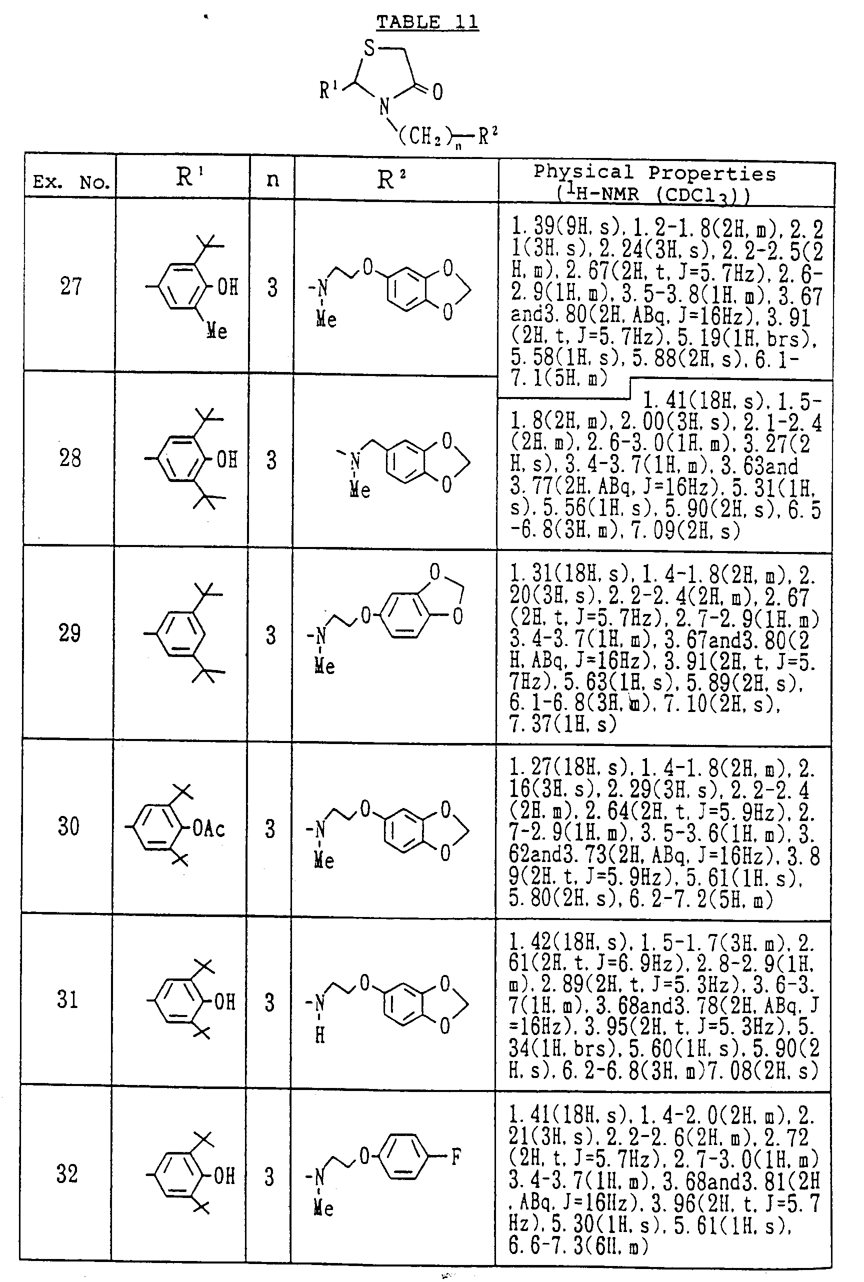

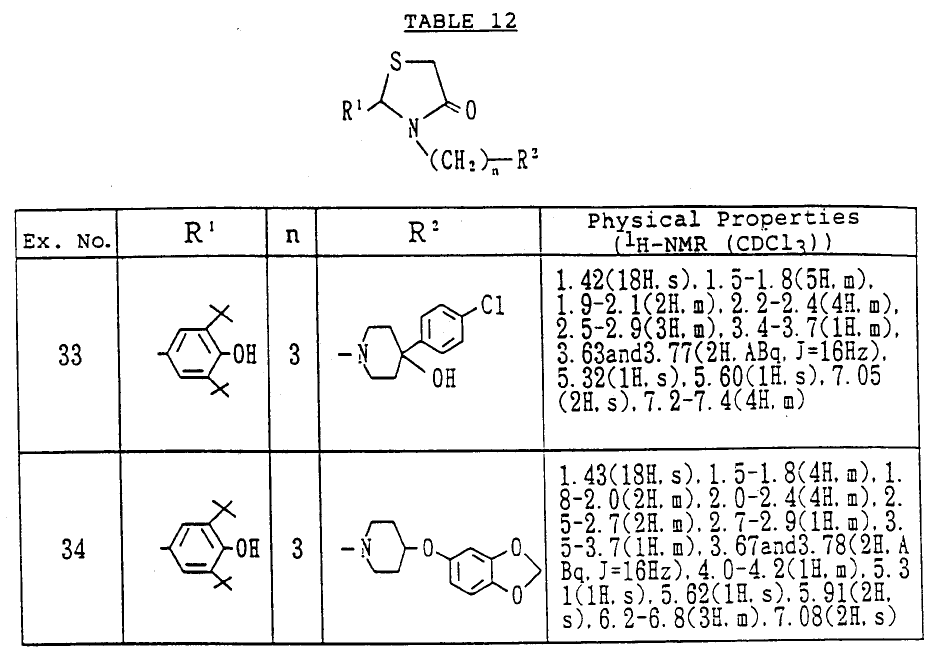

### REFERENCE EXAMPLE 33

### Preparation of 2-(3,5-Di-tert-butyl-4-hydroxyphenyl)-3-[3-(N-methylamino)propyl]-1,3-thiazolidin-4-one Hydrobromide

A mixture of 2-(3,5-di-tert-butyl-4-hydroxyphenyl)-3-(3-bromopropyl)-1,3-thiazolidin-4-one (1.10 g) obtained in Reference Example 29, 40% methanolic solution of methylamine (20 ml), and acetonitrile (15 ml) was stirred at room temperature for 15 hours under a nitrogen atmosphere. After completion of the reaction, the solvent and excess methylamine were removed under reduced pressure. The residue was purified by silica gel column chromatography (eluent; chloroform-methanol, 95:5) to afford 0.90 g (76%) of the title compound as pale orange crystals. mp 195-196°C.
- NMR (CDCl₃, 60 MHz) δ:: 1.42 (18H, s), 1.6-2.2 (2H, m), 2.67 (3H, s), 2.6-3.6 (4H, m), 3.77 (2H, brs), 5.33 (1H, s), 5.63 (1H, brs), 7.08 (2H, s)

Each compound shown in Table 13 was prepared according to the procedure for Reference Example 33, using an appropriate amine instead of methylamine in each case.

In Reference Example 35, chromatographic purification was performed on a column of silica gel using chloroform-methanol, 95:5 containing 1% triethylamine as an eluent.

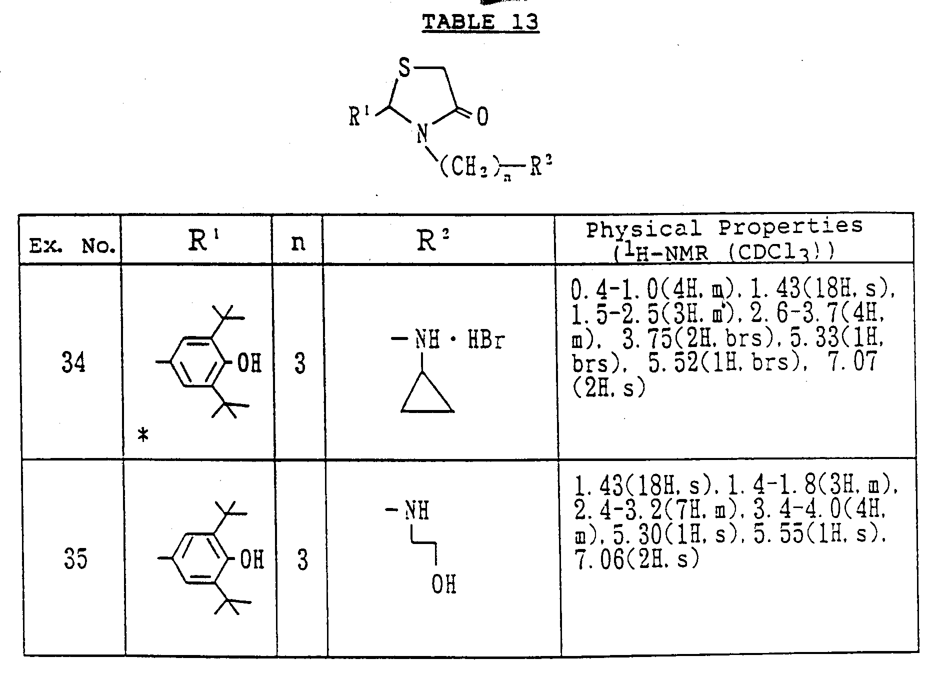

### EXAMPLE 26-B

To a solution of the 2-(3,5-di-tert-butyl-4-hydroxyphenyl)-3-[3-(N-methylamino)propyl]-1,3-thiazolidin- 4-one hydrobromide (380 mg) obtained in Reference Example 33 and 2-(3,4-methylenedioxyphenoxy)ethyl bromide (260 mg) in acetone (10 ml) was added potassium carbonate (300 mg) and the mixture was refluxed for 10 hours under a nitrogen atmosphere. After allowing the reaction mixture to cool, insoluble matter was removed by filtration, and the filtrate was concentrated under reduced pressure. The residue was purified by silica gel column chromatography (eluent; chloroform-methanol, 97:3) to afford 350 mg (64%) of the same compound as obtained in Example 26-A.

### EXAMPLES 35 TO 37

Each compound of Table 14 was prepared according to the procedure for Example 26-B, reacting each of the compounds shown in Table 13 with an appropriate bromide in each case.

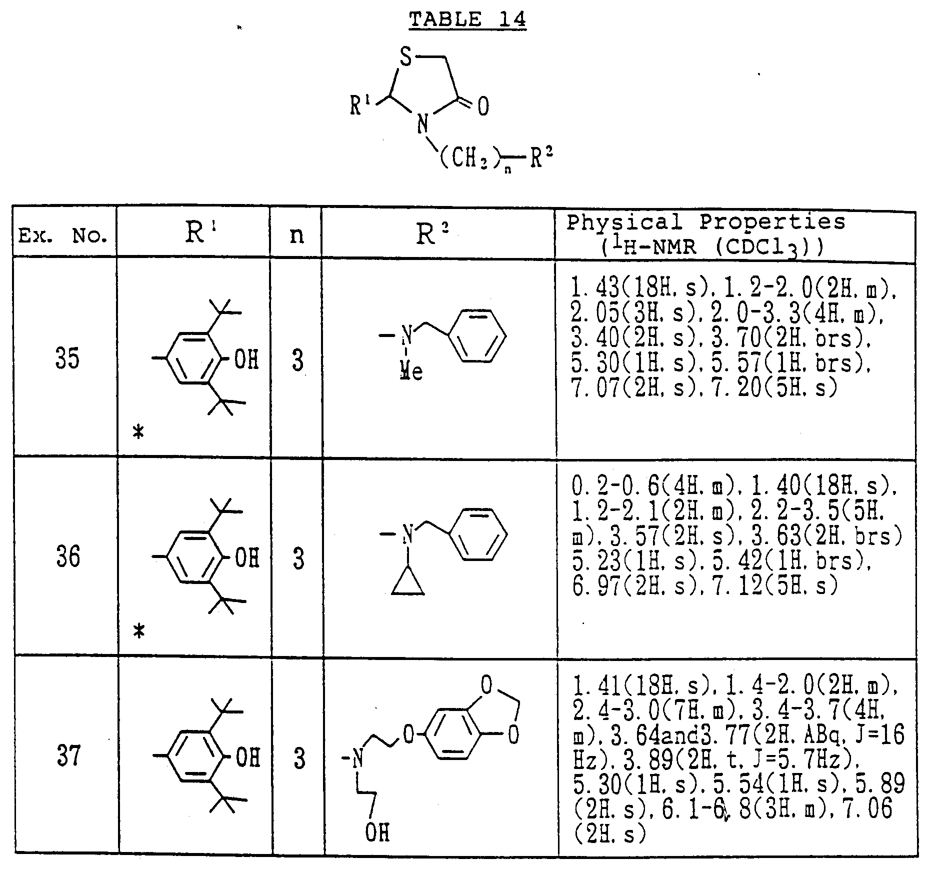

### EXAMPLE 38

### Preparation of 2-(3,5-Di-tert-butyl-4-hydroxyphenyl)-3-[3-[N-methyl-N-[2-hydroxy-3-(3,4-methylenedioxyphenoxy)propyl]amino]propyl]-1,3-thiazolidin-4-one

To a solution of the 2-(3,5-di-tert-butyl-4-hydroxyphenyl)-3-[3-(N-methylamino)propyl]-1,3-thiazolidin-4-one (0.50 g) obtained in Reference Example 33 in acetonitrile (10 ml) was added 2,3-epoxypropyl-3,4-methylenedioxyphenyl ether (0.26 g) at room temperature, and the mixture was refluxed for 8 hours. After allowing to cool, the mixture was poured into ice-water and extracted with ethyl acetate. The organic layer was washed with brine and dried over anhydrous sodium sulfate. The solvent was evaporated under reduced pressure, and the residue was purified by silica gel column chromatography (eluent; chloroform-methanol, 98:2) to afford 0.72 g (95%) of the title compound as a colorless oil.
- NMR (CDCl₃, 270 MHz) δ:: 1.43 (18H, s), 1.4-1.8 (2H, m), 2.17 (3H, s), 2.2-2.6 (4H, m), 2.7-3.0 (1H, m), 3.4-3.7 (1H, m), 3.67 and 3.80 (2H, ABq, J=16.0Hz), 3.8-4.1 (4H, m), 5.33 (1H, s), 5.57 (1H, s), 5.91 (2H, s), 6.2-6.8 (3H, m), 7.09 (2H, s)

### EXAMPLES 39 AND 40

Each compound shown in Table 15 was prepared according to the procedure for Example 38, using an appropriate epoxide in each case.

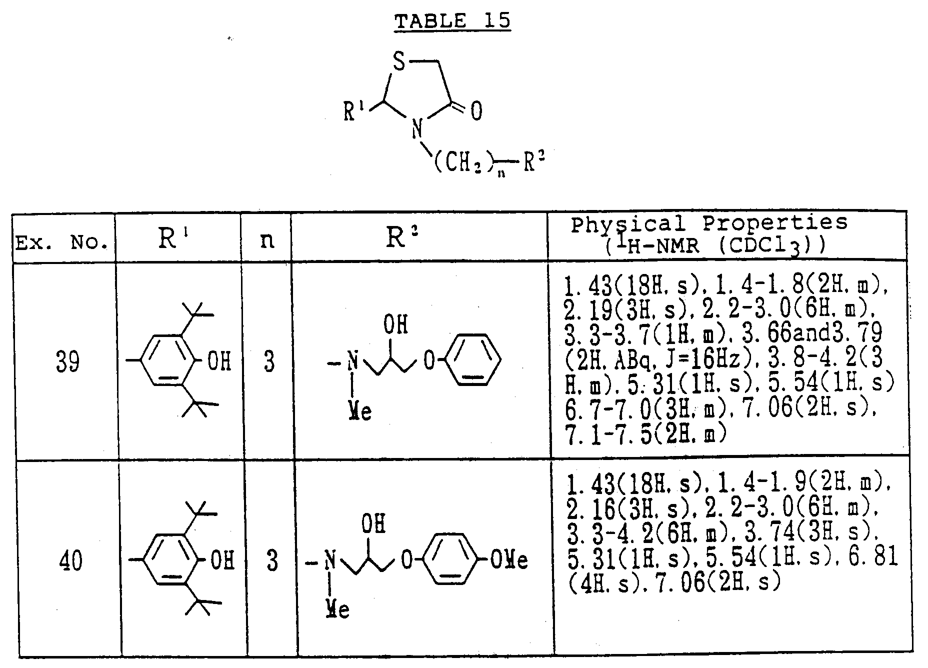

### REFERENCE EXAMPLE 36

### Preparation of 3-[N-Methyl-N-[2-(3,4-methylenedioxyphenoxy)ethyl]amino]propylamine

In acetone (20 ml) were suspended N-methyl-N-[2-(3,4-methylenedioxyphenoxy)ethyl]amine (1.0 g), N-(3-bromopropyl)phthalimide (1.51 g), and potassium carbonate (0.78 g), and the suspension was refluxed for 3 hours. After allowing to cool, the mixture was filtered to remove inorganic matter, and the filtrate was concentrated. The residue was purified by silica gel column chromatography (eluent; chloroform-methanol, 10:1) to afford 1.82 g (93%) of N-[3-[N'-methyl-N'-[2-(3,4-methylenedioxyphenoxy)ethyl]amino]propyl]phthalimide as a brown oil.
- NMR (CDCl₃, 60 MHz) δ:: 1.5-2.2 (2H, m), 2.28 (3H, s), 2.3-2.9 (4H, m), 3.72 (2H, t, J=7.0Hz), 3.90 (2H, t, J=6.0Hz), 5.82 (2H, s), 6.0-6.8 (3H, m), 7.4-8.0 (4H, m)

The resulting N-[3-[N'-methyl-N'-[2-(3,4-methylenedioxyphenoxy)ethyl]amino]propyl]phthalimide (1.82 g) was dissolved in 40% methanolic solution of methylamine (10 ml), and the mixture was stirred at room temperature overnight. After completion of the reaction, the solvent and excess methylamine were removed under reduced pressure. The residue was purified by silica gel column chromatography (eluent; chloroform-methanol, 1:1 containing 1% triethylamine) to afford 0.74 g (62%) of the title compound as a pale brown oil.
- NMR (CDCl₃) (60 MHz) δ:: 1.33 (2H, s), 1.1-2.1 (2H, m), 2.27 (3H, s), 2.2-3.0 (6H, m), 3.90 (2H, t, J=6.0Hz), 5.77 (2H, s), 6.0-6.8 (3H, m)

### REFERENCE EXAMPLE 37

### Preparation of 2-[N-Methyl-N-[2-(3,4-methylenedioxyphenoxy)ethyl]amino]ethylamine

The title compound was prepared according to the procedure for Reference Example 36, using N-(2-bromoethyl)phthalimide instead of N-(3-bromopropyl)phthalimide.
- NMR (CDCl₃) (60 MHz) δ:: 2.34 (3H, s), 2.0-3.2 (8H, m), 3.90 (2H, t, J=6.0Hz), 5.85 (2H, s), 6.0-6.9 (3H, m)

### EXAMPLE 41 (Method-A)

### Preparation of 2-(3,5-Di-tert-butyl-4-hydroxyphenyl)-3-[3-[N-methyl-N-[2-(3,4-methylenedioxyphenoxy)ethyl]amino]propyl]-5-methyl-1,3-thiazolidin-4-one

The title compound was prepared according to the procedure for Reference Example 1, using 3-[N-methyl-N-[2-(3,4-methylenedioxyphenoxy)ethyl]amino]propylamine and 2-mercaptopropionic acid instead of 3-aminopropanol and α-mercaptoacetic acid.
- NMR (CDCl₃) (270 MHz) δ:: 1.42 (18H, s), 1.2-1.8 (2H, m), 1.58 (3x2/5H, d, J=6.9Hz), 1.65 (3x3/5H, d, J=6.9Hz), 2.20 (3x3/5H, s), 2.23 (3x2/5H, s), 2.2-2.5 (2H, m), 2.6-2.9 (3H, m), 3.4-3.7 (1H, m), 3.8-4.1 (3H, m), 5.29 (2/5H, s), 5.30 (3/5H, s), 5.56 (3/5H, s), 5.57 (2/5H, brs), 5.90 (2H, s), 6.2-6.8 (3H, m), 7.05 (2x2/5H, s), 7.11 (2x3/5H, s)

### EXAMPLE 42

### Preparation of 2-(3,5-Di-tert-butyl-4-hydroxyphenyl)-3-[2-[N-methyl-N-[2-(3,4-methylenedioxyphenoxy)ethyl]amino]ethyl]-1,3-thiazolidin-4-one

The title compound was prepared according to the procedure for Reference Example 1, using 2-[N-methyl-N-[2-(3,4-methylenedioxyphenoxy)ethyl]amino]ethylamine instead of 3-aminopropanol.
- NMR (CDCl₃) (60 MHz) δ:: 1.40 (18H, s), 2.20 (3H, s), 2.5-3.0 (5H, m), 3.3-4.1 (3H, m), 3.65 (2H, brs), 5.23 (1H, s), 5.73 (1H, s), 5.82 (2H, s), 6.0-6.8 (3H, m), 7.00 (2H, s)

### EXAMPLE 43

### Preparation of 2-(3-tert-Butyl-4-hydroxyphenyl)-3-[3-[N-methyl-N-[2-(3,4-methylenedioxyphenoxy)ethyl]amino]propyl]-1,3-thiazolidin-4-one

In acetic acid (5 ml) was dissolved 2-(3,5-di-tert-butyl-4-hydroxyphenyl)-3-[3-[N-methyl-N-[2-(3,4-methylenedioxyphenoxy)ethyl]amino]propyl]-1,3-thiazolidin-4-one (0.35 g), and 47% hydrobromic acid (5 ml) was added, then the mixture was stirred at room temperature for 7 days. After completion of the reaction, the mixture was poured into an ice-cooled 5% aqueous solution of sodium carbonate and extracted with chloroform. The organic layer was dried over anhydrous sodium sulfate, and the solvent was evaporated under reduced pressure. The residue was purified by silica gel column chromatography (eluent; chloroform-methanol, 98:2) to afford 0.05 g (16%) of the title compound as a pale brown oil.
- NMR (CDCl₃) (200 MHz) δ:: 1.37 (9H, s), 1.2-1.9 (2H, m), 2.26 (3H, s), 2.1-2.5 (2H, m), 2.71 (2H, t, J=5.7Hz), 2.6-3.0 (1H, m), 3.4-3.8 (1H, m), 3.67 and 3.80 (2H, ABq, J=16.0Hz), 3.94 (2H, t, J=5.7Hz), 5.60 (1H, s), 5.82 (1H, s), 5.88 (2H, s), 6.1-7.0 (5H, m), 7.14 (1H, s)

### REFERENCE EXAMPLE 38

2-(3,5-Di-tert-butyl-4-hydroxyphenyl)-3-(3-hydroxypropyl)-5-methyl-1,3-thiazolidin-4-one was prepared according to the procedure for Reference Example 1, using 2-mercaptopropionic acid instead of α-mercaptoacetic acid.
- NMR (CDCl₃) (200 MHz) δ:: 1.43 (18H, s), 1.1-1.7 (2H, m), 1.60 (3x1/4H, d, J=6.9Hz), 1.67 (3x3/4H, d, J=6.9Hz), 3.0-3.3 (1H, m), 3.3-3.7 (4H, m), 3.9-4.2 (1H, m), 5.34 (1/4H, s), 5.36 (3/4H, s), 5.49 (3/4H, s), 5.52 (1/4H, s), 7.07 (2x1/4H, s), 7.12 (2x3/4H, s)

### REFERENCE EXAMPLES 39 AND 40

Each compound shown in Table 16 was prepared according to the procedure for Reference Example 38, using an appropriate α-mercaptocarboxylic acid instead of 2-mercaptopropionic acid in each case.

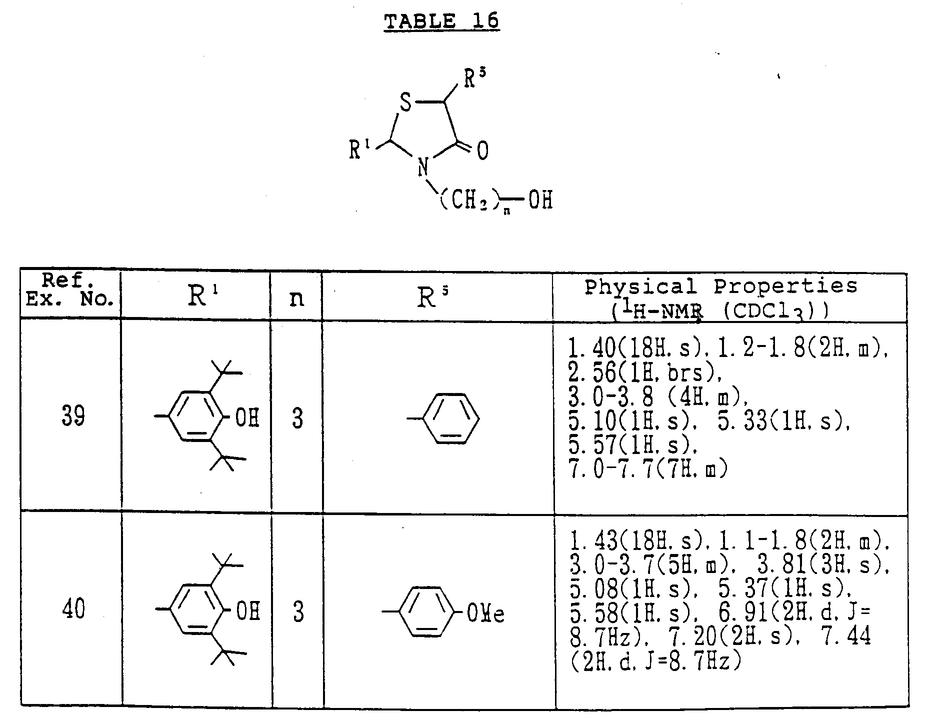

### REFERENCE EXAMPLE 41

### Preparation of 2-(3,5-Di-tert-butyl-4-hydroxyphenyl)-3-(3-hydroxypropyl)-5-carboxymethyl-1,3-thiazolidin-4-one

In benzene (200 ml) were suspended 3,5-di-tert-butyl-4-hydroxybenzaldehyde (23.4 g) and 3-aminopropanol (9.01 g) under a nitrogen atmosphere. After fitting a Dean-Stark trap to the reactor, the suspension was refluxed for 2 hours. After allowing the mixture to cool, thiomalic acid (19.52 g) was added thereto, then the mixture was refluxed for an additional period of 3 hours. Benzene was removed by evaporation, and the resulting white solid was recrystallized from water-containing methanol to afford 10.5 g (25%) of the title compound as colorless crystals. mp 227-228°C.
- NMR (d₆-DMSO) (200 MHz) δ:: 1.37 (18H, s), 1.2-1.8 (2H, m), 2.3-2.8 (1H, m), 3.0-3.6 (5H, m), 4.0-4.2 (1H, m), 4.37 (1H, brs), 5.73 (1H, s), 7.10 (2H, s), 7.13 (1H, s)

### REFERENCE EXAMPLE 42

2-(3,5-Di-tert-butyl-4-hydroxyphenyl)-3-(3-chloropropyl)-5-methyl-1,3-thiazolidin-4-one was prepared from 2-(3,5-di-tert-butyl-4-hydroxyphenyl)-3-(3-hydroxypropyl)-5-methyl-1,3-thiazolidin-4-one according to the procedure for Reference Example 17.
- NMR (CDCl₃) (60 MHz) δ:: 1.42 (18H, s), 1.1-2.2 (5H, m), 2.7-4.2 (5H, m), 5.23 (1H, s), 5.43 (1H, brs), 6.97 (2H, brs)

### REFERENCE EXAMPLES 43 AND 44

Each compound shown in Table 17 was prepared from each of the compounds prepared in Reference Examples 39 and 40, respectively, according to the procedure for Reference Example 29.

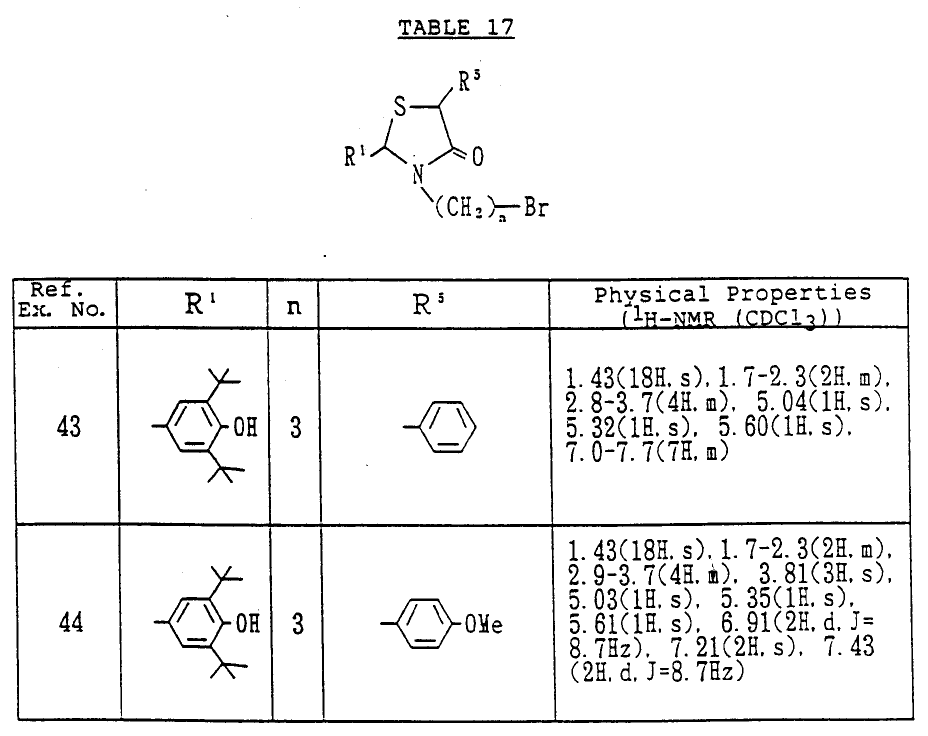

### REFERENCE EXAMPLE 45

### Preparation of 2-(3,5-Di-tert-butyl-4-hydroxyphenyl)-3-(3-bromopropyl)-5-ethoxycarbonylmethyl-1,3-thiazolidin-4-one

To a suspension of 2-(3,5-di-tert-butyl-4-hydroxyphenyl)-3-(3-hydroxypropyl)-5-carboxymethyl-1,3-thiazolidin-4-one (2.45 g) in diethyl ether (30 ml) was added phosphorus tribromide (3.45 g), and the mixture was stirred at room temperature for 3 hours. To the mixture was added dropwise ethanol (30 ml) under cooling with ice, the mixture was stirred at room temperature overnight. After completion of the reaction, the reaction mixture was poured into 100 ml of ice-water, and the product was extracted with ethyl acetate. The organic layer was washed with brine and dried over anhydrous sodium sulfate. The solvent was removed by evaporation under reduced pressure, and the residue was purified by silica gel column chromatography (eluent; ethyl acetate:n-hexane, 20:80) and then recrystallized from chloroform/n-hexane to afford 1.50 g (51%) of the title compound as colorless crystals. mp 154-155°C.
- NMR (CDCl₃) (200 MHz) δ:: 1.26 (3H, t, J=7.1Hz), 1.43 (18H, s), 1.6-2.2 (2H, m), 2.6-3.1 (2H, m), 3.2-3.6 (4H, m), 4.17 (2H, q, J=7.1Hz), 4.1-4.4 (1H, m), 5.33 (1H, s), 5.53 (1H, s), 7.14 (2H, s)

### REFERENCE EXAMPLE 46

2-(3,5-Di-tert-butyl-4-hydroxyphenyl)-3-(3-bromopropyl)-5-isopropoxycarbonylmethyl-1,3-thiazolidin-4-one was prepared as colorless crystals according to the procedure for Reference Example 45, using isopropyl alcohol instead of ethanol. mp 170-171°C.
- NMR (CDCl₃) (200 MHz) δ:: 1.24 (6H, d, J=5.7Hz), 1.43 (18H, s), 1.6-2.2 (2H, m), 2.6-3.7 (6H, m), 4.1-4.4 (1H, m), 4.8-5.2 (1H, m), 5.33 (1H, s), 5.53 (1H, s), 7.14 (2H, s)

### EXAMPLE 41 (Method-B)

### Preparation of 2-(3,5-Di-tert-butyl-4-hydroxyphenyl)-3-[3-[N-methyl-N-[2-(3,4-methylenedioxyphenoxy)ethyl]amino]propyl]-5-methyl-1,3-thiazolidin-4-one

The title compound was prepared according to the procedure for Example 1, using 2-(3,5-di-tert-butyl-4-hydroxyphenyl)-3-(3-chloropropyl)-5-methyl-1,3-thiazolidin-4-one prepared in Reference Example 42. The data of instrumental analyses on the resulting compound were identical with those of the compound prepared in Example 41-A.

### EXAMPLES 44 AND 45

Each compound shown in Table 18 was prepared according to the procedure for Example 26-A, using respective compounds obtained in Reference Examples 43 and 44, instead of 2-(3,5-di-tert-butyl-4-hydroxyphenyl)-3-(3-bromopropyl)-1,3-thiazolidin-4-one.

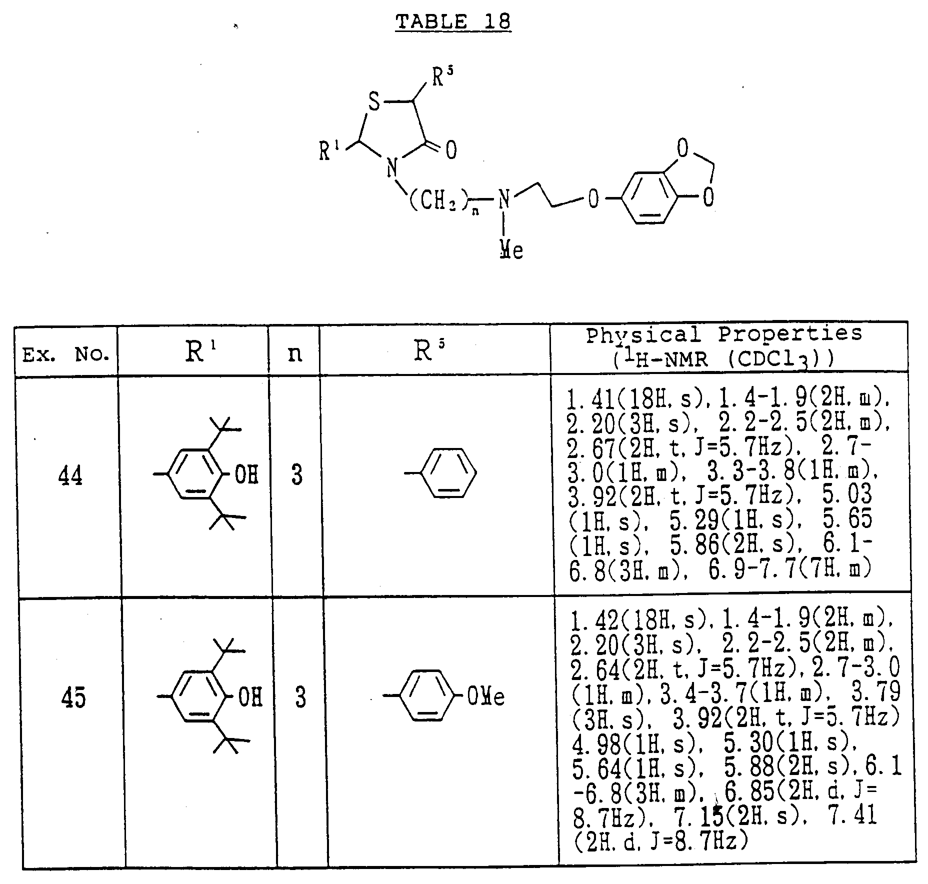

### EXAMPLE 46

### Preparation of 2-(3,5-Di-tert-butyl-4-hydroxyphenyl)-3-[3-[N-methyl-N-[2-(3,4-methylenedioxyphenoxy)ethyl]amino]propyl]-5-ethoxycarbonylmethyl-1,3-thiazolidin-4-one

According to the procedure for Example 26-A, using 2-(3,5-di-tert-butyl-4-hydroxyphenyl)-3-(3-bromopropyl)-5-ethoxycarbonylmethyl-1,3-thiazolidin-4-one obtained in Reference Example 45 instead of 2-(3,5-di-tert-butyl-4-hydroxyphenyl)-3-(3-bromopropyl)-1,3-thiazolidin-4-one, the title compound was prepared as colorless crystals. mp 88-89°C.
- NMR (CDCl₃) (200 MHz) δ:: 1.26 (3H, t, J=7.1Hz), 1.41 (18H, s), 1.4-1.9 (2H, m), 2.19 (3H, s), 2.3-2.5 (2H, m), 2.67 (2H, t, J=5.7Hz), 2.7-3.0 (2H, m), 3.2-3.7 (2H, m), 3.91 (2H, t, J=5.7Hz), 4.17 (2H, q, J=7.1Hz), 4.1-4.4 (1H, m), 5.29 (1H, s), 5.58 (1H, s), 5.89 (2H, s), 6.1-6.8 (3H, m), 7.09 (2H, s)

### EXAMPLE 47

According to the procedure for Example 46, using 2-(3,5-di-tert-butyl-4-hydroxyphenyl)-3-(3-bromopropyl)-5-isopropoxycarbonylmethyl-1,3-thiazolidin-4-one instead of 2-(3,5-di-tert-butyl-4-hydroxyphenyl)-3-(3-bromopropyl)-5-ethoxycarbonylmethyl-1,3-thiazolidin-4-one, 2-(3,5-di-tert-butyl-4-hydroxyphenyl)-3-[3-[N-methyl-N-[2-(3,4-methylenedioxyphenoxy)ethyl]amino]propyl]-5-isopropoxycarbonylmethyl-1,3-thiazolidin-4-one was prepared as colorless crystals. mp 81-82°C.
- NMR (CDCl₃) (200 MHz) δ:: 1.23 (6H, d, J=5.7Hz), 1.41 (18H, s), 1.4-2.0 (2H, m), 2.20 (3H, s), 2.2-2.5 (2H, m), 2.5-3.0 (4H, m), 3.1-3.7 (2H, m), 3.93 (2H, t, J=5.7Hz), 4.1-4.4 (1H, m), 4.8-5.2 (1H, m), 5.30 (1H, s), 5.57 (1H, s), 5.89 (2H, s), 6.1-6.8 (3H, m), 7.10 (2H, s)

### EXAMPLE 48

### Preparation of 2-(3,5-Di-tert-butyl-4-hydroxyphenyl)-3-[3-[N-methyl-N-[2-(3,4-methylenedioxyphenoxy)ethyl]amino]propyl]-5-carboxymethyl-1,3-thiazolidin-4-one

In ethanol (5 ml) was dissolved 2-(3,5-di-tert-butyl-4-hydroxyphenyl)-3-[3-[N-methyl-N-[2-(3,4-methylenedioxy phenoxy)ethyl]amino]propyl]-5-ethoxycarbonylmethyl-1,3-thiazolidin-4-one (100 mg) prepared in Example 46, and a solution of sodium hydroxide (200 mg) in 20% water-containing ethanol was added, then the mixture was stirred at room temperature overnight. After completion of the reaction, the mixture was neutralized with 1N hydrochloric acid and concentrated under reduced pressure. To the residue was added a mixture of water (50 ml) and chloroform (50 ml), and the mixture was stirred. The organic layer was separated, washed with brine, and dried over anhydrous sodium sulfate. The solvent was evaporated under reduced pressure, and the residue was triturated with n-hexane to afford 80 mg (84%) of the title compound as a pale yellow solid.
- NMR (CDCl₃) (200 MHz) δ:: 1.41 (18/2H, s), 1.42 (18/2H, s), 1.4-2.4 (2H, m), 2.85 (3/2H, s), 2.90 (3/2H, s), 2.7-3.8 (8H, m), 4.1-4.7 (3H, m), 5.2-5.4 (1H, m), 5.66 (1/2H, brs), 5.81 (1/2H, brs), 5.89 (2H, s), 6.2-6.7 (3H, m), 7.13 (2/2H, s), 7.14 (2/2H, s)

### EXAMPLE 49

### Preparation of 2-(3,5-Di-tert-butyl-4-hydroxyphenyl)-3-[3-[N-methyl-N-[2-(3,4-methylenedioxyphenoxy)ethyl]amino]propyl]-5-(2-hydroxyethyl)-1,3-thiazolidin-4-one

To a solution of 2-(3,5-di-tert-butyl-4-hydroxyphenyl)-3-[3-[N-methyl-N-[2-(3,4-methylenedioxyphenoxy)ethyl]amino]propyl]-5-ethoxycarbonylmethyl-1,3-thiazolidin-4-one (80 mg) prepared in Example 46 in dry tetrahydrofuran (5 ml) was added lithium aluminum hydride (20 mg) at -78°C, and the mixture was stirred at 0°C for 3 hours. To the mixture was added water-containing tetrahydrofuran (5 ml), followed by stirring at that temperature for 1 hour. The mixture was neutralized with 1N hydrochloric acid, concentrated under reduced pressure. The residue was purified by silica gel column chromatography (eluent; chloroform-methanol, 95:5) to afford 68 mg (86%) of the title compound as a colorless oil.
- NMR (CDCl₃) (200 MHz) δ:: 1.41 (18H, s), 1.3-1.8 (4H, m), 2.19 (3H, s), 2.2-2.5 (3H, m), 2.67 (2H, t, J=5.7Hz), 2.7-2.9 (1H, m), 3.4-3.6 (1H, m), 3.8-4.0 (2H, m), 3.91 (2H, t, J=5.7Hz), 4.04 (1H, t, J=5.7Hz), 5.33 (1H, s), 5.60 (1H, s), 5.90 (2H, s), 6.2-6.7 (3H, m), 7.12 (2H, s)

### REFERENCE EXAMPLE 47

### Preparation of 2-(3,5-Di-tert-butyl-4-hydroxyphenyl)-3-(3-chloropropyl)-5-(1-pyrrolidinecarbonylmethyl)-1,3-thiazolidin-4-one

To a suspension of 2-(3,5-di-tert-butyl-4-hydroxyphenyl)-3-(3-hydroxypropyl)-5-carboxymethyl-1,3-thiazolidin-4-one (0.61 g) obtained in Reference Example 41 in dichloromethane (20 ml) were added thionyl chloride (0.38 g) and a catalytic amount of dimethylformamide, and the mixture was refluxed for 2 hours. After allowing to cool, the mixture was added dropwise to a solution of pyrrolidine (1.02 g) in dichloromethane (20 ml) under cooling with ice, and stirred at that temperature for 1 hour. After completion of the reaction, the reaction mixture was poured into ice-water and extracted with chloroform. The organic layer was washed successively with 1N hydrochloric acid and brine, and dried over anhydrous sodium sulfate. The solvent was evaporated under reduced pressure, and the residue was purified by silica gel column chromatography (eluent; chloroform-methanol, 97:3) and then recrystallized from chloroform/n-hexane to yield 0.65 g (92%) of the title compound as colorless crystals. mp 193-194°C.
- NMR (CDCl₃) (200 MHz) δ:: 1.43 (18H, s), 1.4-2.2 (6H, m), 2.5-3.1 (2H, m), 3.2-3.7 (8H, m), 4.2-4.5 (1H, m), 5.29 (1H, s), 5.53 (1H, s), 7.08 (2H, s)

### REFERENCE EXAMPLES 48 TO 50

Each compound shown in Table 19 was prepared according to the procedure for Reference Example 47, using an appropriate amine in each case.

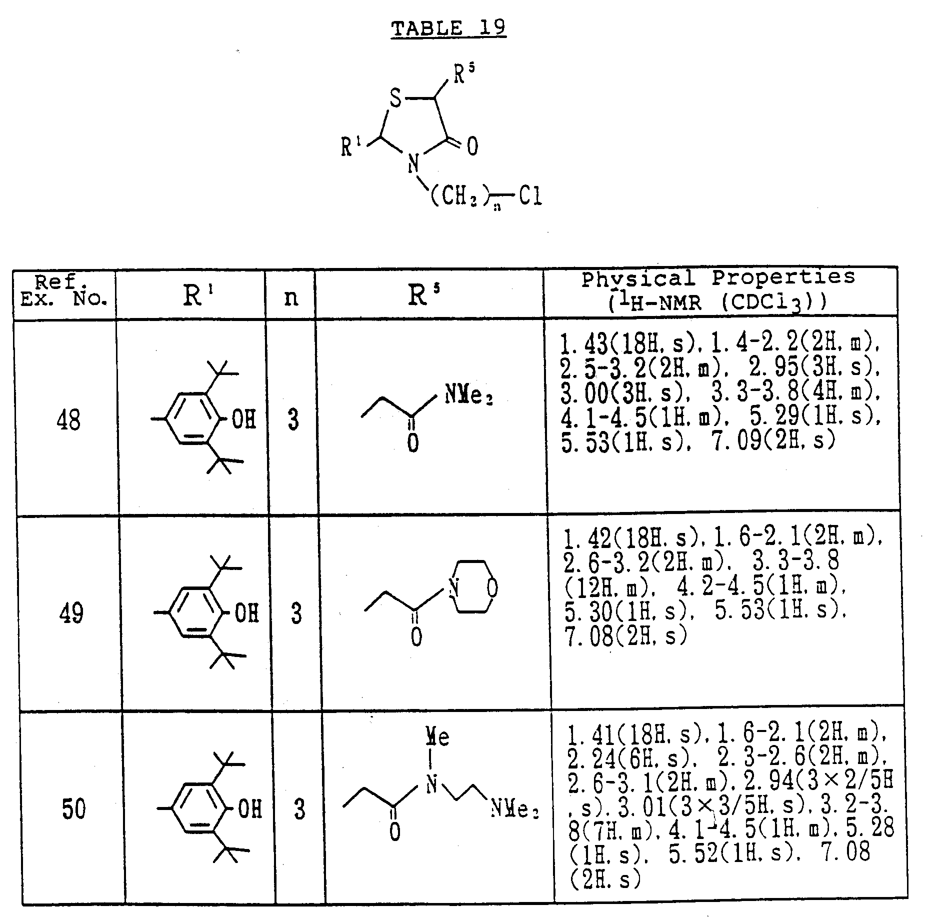

### EXAMPLE 50

### Preparation of 2-(3,5-Di-tert-butyl-4-hydroxyphenyl)-3-[3-[N-methyl-N-[2-(3,4-methylenedioxyphenoxy)ethyl]amino]propyl]-5-(1-pyrrolidinecarbonylmethyl)-1,3-thiazolidin-4-one

The title compound was obtained as a pale brown oil according to the procedure for Example 1, using 2-(3,5-di-tert-butyl-4-hydroxyphenyl)-3-(3-chloropropyl)-5-(1-pyrrolidinecarbonylmethyl)-1,3-thiazolidin-4-one obtained in Reference Example 47 instead of 2-(3,5-diisopropyl-4-hydroxyphenyl)-3-(3-chloropropyl)-1,3-thiazolidin-4-one.
- NMR (CDCl₃) (200 MHz) δ:: 1.41 (18H, s), 1.4-2.1 (6H, m), 2.20 (3H, s), 2.2-2.5 (2H, m), 2.5-3.1 (4H, m), 3.2-3.8 (6H, m), 3.93 (2H, t, J=5.7Hz), 4.2-4.5 (1H, m), 5.29 (1H, s), 5.57 (1H, s), 5.88 (2H, s), 6.1-6.8 (3H, m), 6.9-7.2 (2H, m)

### EXAMPLES 51 TO 53

Each compound shown in Table 20 was prepared according to the procedure for Example 50, using each of the compounds prepared in Reference Examples 48 to 50 instead of 2-(3,5-di-tert-butyl-4-hydroxyphenyl)-3-(chloropropyl)-5-(1-pyrrolidinecarbonylmethyl)-1,3-thiazolidin-4-one.

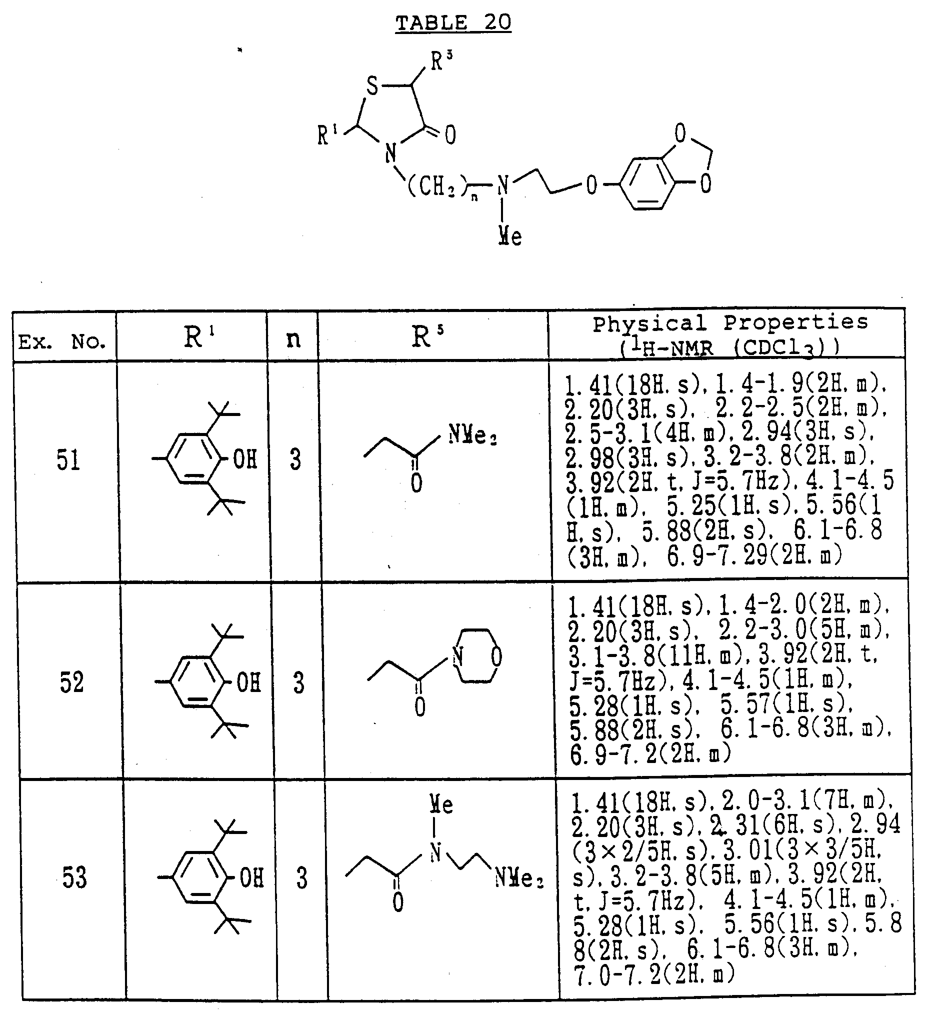

### REFERENCE EXAMPLE 51

### Preparation of 2-(3,5-Di-tert-butyl-4-hydroxyphenyl)-3-(3-bromopropyl)-5-methoxy-1,3-thiazolidin-4-one

To a solution of 2-(3,5-di-tert-butyl-4-hydroxyphenyl)-3-(3-bromopropyl)-1,3-thiazolidin-4-one (1.0 g) prepared in Reference Example 29 in dichloromethane (15 ml) was added dropwise sulfuryl chloride (0.36 g) under cooling with ice-water, and the mixture was stirred at room temperature for 1.5 hours. The solvent was evaporated under reduced pressure, and methanol (10 ml) was added to the residue, and the mixture was stirred at room temperature overnight. The mixture was concentrated under reduced pressure, and the residue was purified by silica gel column chromatography (eluent; chloroform) to afford 0.59 g (48%) of the title compound as a pale brown oil.
- NMR (CDCl₃) (200 MHz) δ:: 1.42 (18H, s), 1.5-2.3 (2H, m), 2.8-3.6 (4H, m), 3.46 (3x2/5H, s), 3.52 (3x3/5H, s), 5.2-6.1 (3H, m), 7.08 (2x2/5H, s), 7.12 (2x3/5H, s)

### REFERENCE EXAMPLES 52 AND 53

Each compound shown in Table 21 was prepared according to the procedure for Reference Example 51, using ethylene glycol instead of methanol.

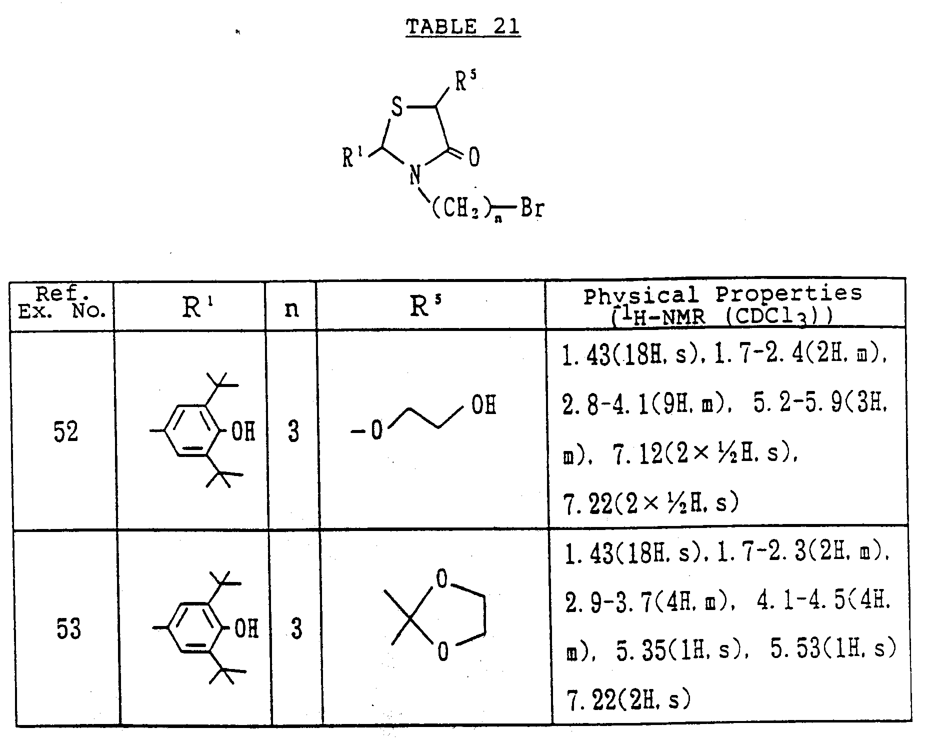

### EXAMPLE 54

### Preparation of 2-(3,5-Di-tert-butyl-4-hydroxyphenyl)-3-[3-[N-methyl-N-[2-(3,4-methylenedioxyphenoxy)ethyl]amino]propyl]-5-methoxy-1,3-thiazolidin-4-one

The title compound was obtained as a pale yellow oil according to the procedure for Example 26-A, using 2-(3,5-di-tert-butyl-4-hydroxyphenyl)-3-(3-bromopropyl)-5-methoxy-1,3-thiazolidin-4-one prepared in Reference Example 51 instead of 2-(3,5-di-tert-butyl-4-hydroxyphenyl)-3-(3-bromopropyl)-1,3-thiazolidin-4-one.
- NMR (CDCl₃) (270 MHz) δ:: 1.41 (18H, s), 1.4-1.9 (2H, m), 2.18 (3x2/5H, s), 2.22 (3x3/5H, s), 2.2-3.1 (5H, m), 3.46 (3x2/5H, s), 3.51 (3x3/5H, s), 3.5-3.7 (1H, m), 3.8-4.1 (2H, m), 5.2-5.8 (3H, m), 5.90 (2H, s), 6.1-6.8 (3H, m), 7.21 (2x2/5H, s), 7.26 (2x3/5H, s)

### EXAMPLES 55 AND 56

Each compound shown in Table 22 was prepared according to the procedure for Example 54, using each of the compounds prepared in Reference Examples 52 and 53 instead of 2-(3,5-di-tert-butyl-4-hydroxyphenyl)-3-(3-bromopropyl)-5-methoxy-1,3-thiazolidin-4-one.

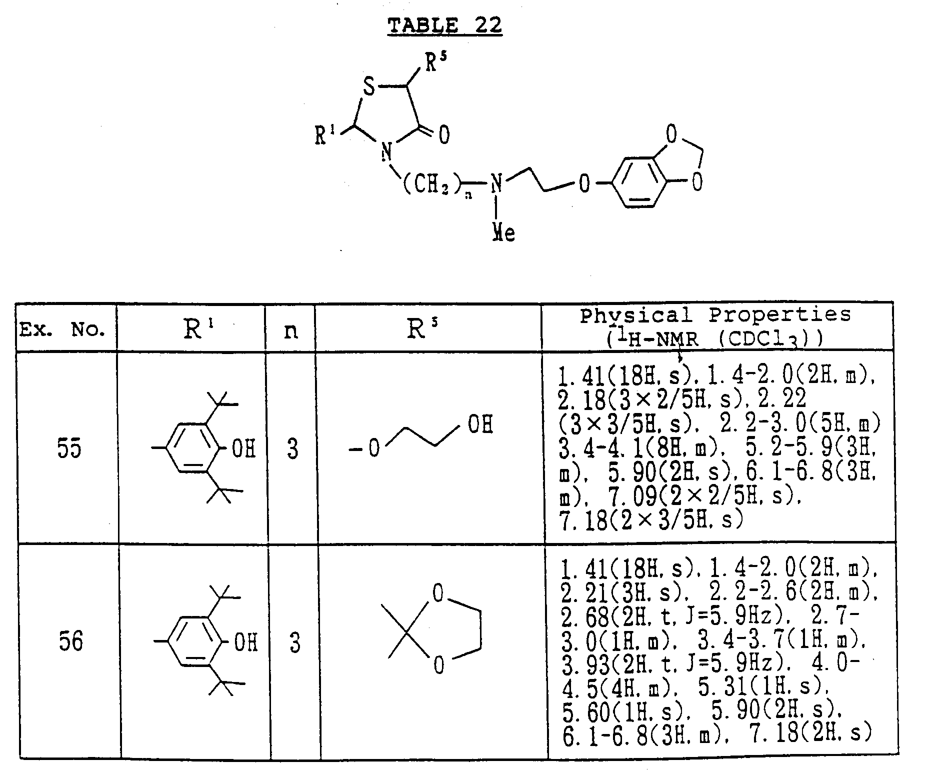

### EXAMPLE 57

### Preparation of 2-(3,5-Di-tert-butyl-4-hydroxyphenyl)-3-[3-[N-methyl-N-[2-(3,4-methylenedioxyphenoxy)ethyl]amino]propyl]-1,3-thiazolidin-4-thione

In THF (5 ml) were suspended 2-(3,5-di-tert-butyl-4-hydroxyphenyl)-3-[3-[N-methyl-N-[2-(3,4-methylenedioxyphenoxy)ethyl]amino]propyl]-1,3-thiazolidin-4-one (217 mg) and Lawesson's reagent (194 mg), and the suspension was stirred at room temperature for 5 hours. The solvent was evaporated under reduced pressure, and to the residue was added water (20 ml), then the mixture was extracted with chloroform. The organic layer was washed successively with water and brine, and dried over anhydrous sodium sulfate. The solvent was removed by evaporation under reduced pressure, and the residue was purified by silica gel column chromatography (eluent; chloroform-methanol, 99:1) to afford 181 mg (81%) of the title compound as a pale yellow oil.
- NMR (CDCl₃) (200 MHz) δ:: 1.41 (18H, s), 1.3-1.8 (2H, m), 2.20 (3H, s), 2.3-2.5 (2H, m), 2.70 (2H, t, J=5.7Hz), 3.1-3.3 (1H, m), 3.93 (2H, t, J=5.7Hz), 3.9-4.1 (1H, m), 4.26 and 4.40 (2H, ABq, J=16.0Hz), 5.34 (1H, s), 6.18 (2H, s), 6.04 (1H, s), 6.2-7.0 (3H, m), 7.07 (2H, s)

### EXAMPLE 58

### Preparation of N-[3-[N-Methyl-N-[2-(3,4-methylenedioxyphenoxy)ethyl]amino]propyl]-3,5-di-tert-butyl-4-hydroxybenzamide

To a suspension of 3,5-di-tert-butyl-4-hydroxybenzoic acid (1.00 g) in tetrahydrofuran (17 ml) were added oxalyl chloride (0.76 g) and a catalytic amount of dimethylformamide at 0°C, the mixture was stirred at room temperature for 1 hour. The solvent and excess oxalyl chloride were removed under reduced pressure, and tetrahydrofuran (8 ml) was added to the residue. The resultant solution was added dropwise to a solution of 3-[N-methyl-N-[2-(3,4-methylenedioxyphenoxy)ethyl]amino]propylamine (1.01 g) and triethylamine (0.41 g) in tetrahydrofuran (13 ml) at 0°C, and the mixture was stirred at room temperature for 6 hours. The reaction mixture was poured into cold water, and the product was extracted with chloroform. The organic layer was washed with brine and dried over anhydrous sodium sulfate. The solvent was evaporated under reduced pressure, and the residue was purified by silica gel column chromatography (eluent; chloroform-methanol, 97:3) to afford 1.35 g (70%) of the title compound as a pale brown oil.
- NMR (CDCl₃) δ:: 1.34 (18x1/3H, s), 1.43 (18x2/3H, s), 1.7-1.9 (2H, m), 2.41 (3H, s), 2.6-2.7 (2H, m), 2.8-2.9 (2H, m), 3.5-3.6 (2H, m), 3.9-4.1 (2H, m), 5.48 (1H, s), 5.86 (2x2/3H, s), 5.88 (2x1/3H, s), 6.1-6.7 (3H, m), 7.64 (2x2/3H, s), 7.79 (2x1/3H, s), 7.8-8.0 (1H, brs)

### EXAMPLE 59

According to the procedure for Example 58, 3,5-di-tert-butyl-4-hydroxybenzenesulfonyl chloride was prepared from 3,5-di-tert-butyl-4-hydroxybenzenesulfonic acid and thionyl chloride instead of 3,5-di-tert-butyl-4-hydroxybenzoic acid and oxalyl chloride, and then converted to N-[3-[N-methyl-N-[2-(3,4-methylenedioxyphenoxy)ethyl]amino]propyl]-3,5-di-tert-butyl-4-hydroxybenzenesulfonamide to afford a colorless oil.
- NMR (CDCl₃) (200 MHz) δ:: 1.43 (18H, s), 1.3-1.8 (2H, m), 2.25 (3H, s), 2.51 (2H, t, J=6.3Hz), 2.72 (2H, t, J=6.3Hz), 3.07 (2H, t, J=6.3Hz), 3.97 (2H, t, J=6.3Hz), 5.66 (1H, s), 5.90 (2H, s), 6.2-6.7 (3H, m), 7.67 (2H, s)

### EXAMPLE 60

### Preparation of N-[3-[N-Methyl-N-[2-(3,4-methylenedioxyphenoxy)ethyl]amino]propyl]-3,5-di-tert-butyl-4-hydroxybenzothioamide

The title compound was obtained as pale yellow crystals according to the procedure for Example 57, using N-[3-[N-methyl-N-[2-(3,4-methylenedioxyphenoxy)ethyl]amino]propyl]-3,5-di-tert-butyl-4-hydroxybenzamide prepared in Example 58 instead of 2-(3,5-di-tert-butyl-4-hydroxyphenyl)-3-[3-[N-methyl-N-[2-(3,4-methylenedioxyphenoxy)ethyl]amino]propyl]-1,3-thiazolidin-4-one. mp 114-115°C.
- NMR (CDCl₃) (200 MHz) δ:: 1.42 (18H, s), 1.6-2.1 (2H, m), 2.21 (3H, s), 2.5-2.9 (4H, m), 3.80 (2H, t, J=5.7Hz), 3.8-4.1 (2H, m), 5.44 (1H, s), 5.82 (2H, s), 5.8-6.7 (3H, m), 7.5-7.8 (3H, m)

### EXAMPLE 61

### Preparation of N-Cyano-N'-[3-[N-methyl-N-[2-(3,4-methylenedioxyphenoxy)ethyl]amino]propyl]-3,5-di-tert-butyl-4-hydroxybenzamidine

To a solution of N-[3-[N-methyl-N-[2-(3,4-methylenedioxyphenoxy)ethyl]amino]propyl]-3,5-di-tert-butyl-4-hydroxybenzothioamide (1.0 g) obtained in Example 60 in dry tetrahydrofuran was added sodium hydride (0.16 g) at 0°C under a nitrogen atmosphere, and the mixture was stirred at the same temperature for 1 hour. To the mixture was added ethyl iodide (0.31 g), followed by stirring at room temperature for 2 hours. The mixture was again cooled to 0°C, and cyanamide (0.84 g) was added thereto, followed by stirring at room temperature for 15 hours. After completion of the reaction, the mixture was poured into ice-water, and the product was extracted with chloroform. The organic layer was washed with brine and dried over anhydrous sodium sulfate. The solvent was removed by evaporation under reduced pressure, and the residue was purified by silica gel column chromatography (eluent; chloroform-methanol, 99:1) to afford 0.70 g (69%) of the title compound as pale yellow crystals. mp 129-130°C.
- NMR (CDCl₃) (200 MHz) δ:: 1.45 (18H, s), 1.6-2.1 (2H, m), 2.20 (3H, s), 2.6-3.0 (4H, m), 3.6-3.8 (2H, m), 3.83 (2H, t, J=5.7Hz), 5.56 (1H, s), 5.84 (2H, s), 5.8-6.8 (3H, m), 7.48 (2H, s), 8.44 (1H, brs)

### REFERENCE EXAMPLE 54

### Preparation of 3-(3-Bromopropyl)-5-(3,5-di-tert-butyl-4-hydroxyphenyl)-1,3,4-oxadiazol-2(3H)-one

To a solution of 5-(3,5-di-tert-butyl-4-hydroxyphenyl)-1,3,4-oxadiazol-2(3H)-one (0.50 g) in dimethylformamide (8 ml) were added sodium carbonate (0.36 g) and dibromopropane (1.74 g), followed by stirring at room temperature for 5 hours. After completion of the reaction, the reaction mixture was poured into ice-water, and the product was extracted with ethyl acetate. The organic layer was dried over anhydrous sodium sulfate, and the solvent was evaporated under reduced pressure. The residue was purified by silica gel column chromatography (eluent; chloroform) and then recrystallized from chloroform/n-hexane to afford 0.45 g (63%) of the title compound as colorless crystals. mp 130-131°C.
- NMR (CDCl₃) (200 MHz) δ:: 1.46 (18H, s), 2.1-2.6 (2H, m), 3.46 (2H, t, J=6.6Hz), 3.93 (2H, t,J=6.6Hz), 5.60 (1H, s), 7.63 (2H, s)

### REFERENCE EXAMPLES 55 TO 58

Each compound shown in Table 23 was prepared according to the procedure for Reference Example 54, using an appropriate 5-membered heterocyclic compound instead of 5-(3,5-di-tert-butyl-4-hydroxyphenyl)-1,3,4-oxadiazol-2(3H)-one in each case.

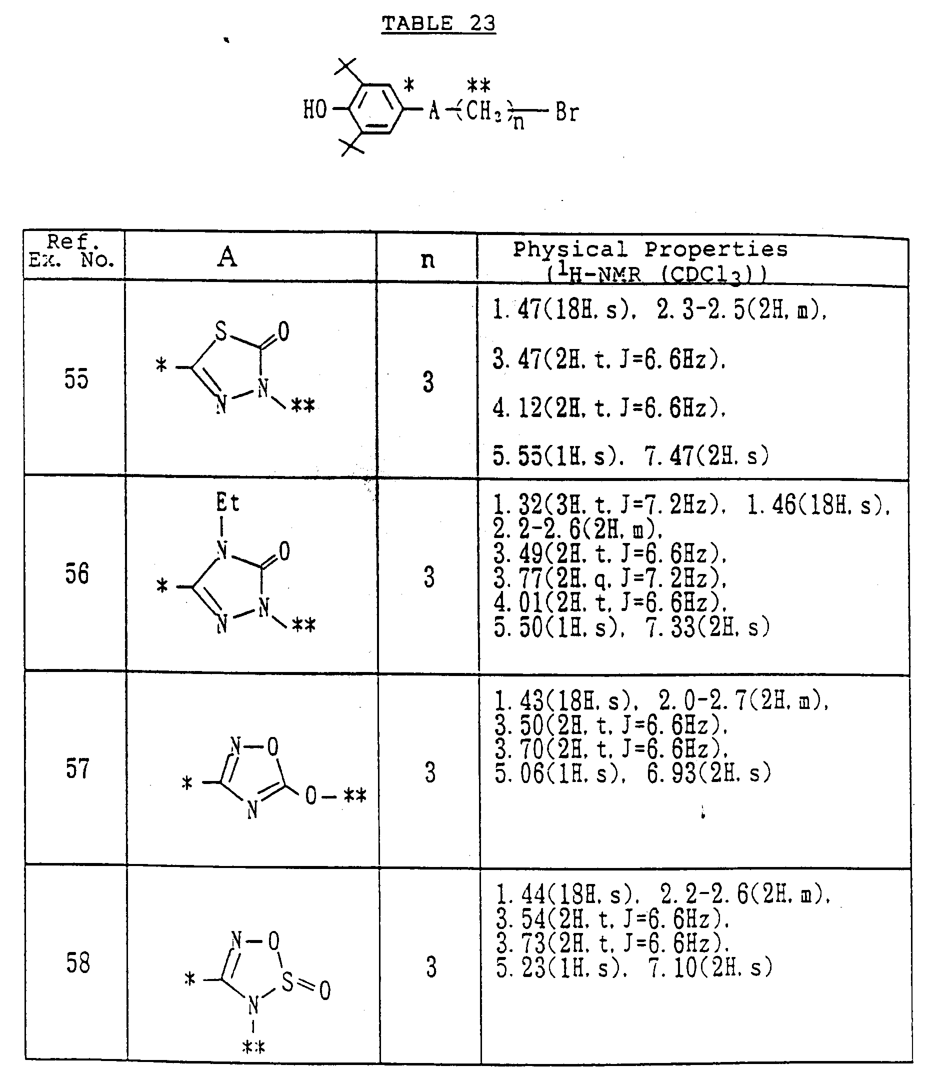

### EXAMPLE 62

### Preparation of 5-(3,5-Di-tert-butyl-4-hydroxyphenyl)-3-[3-[N-methyl-N-[2-(3,4-methylenedioxyphenoxy)ethyl]amino]propyl]-1,3,4-oxadiazol-2(3H)-one

The title compound was obtained as a colorless oil according to the procedure for Example 26-A, using 3-(3-bromopropyl)-5-(3,5-di-tert-butyl-4-hydroxyphenyl)-1,3,4- oxadiazol-2(3H)-one obtained in Reference Example 54 instead of 2-(3,5-di-tert-butyl-4-hydroxyphenyl)-3-(3-bromopropyl)-1,3-thiazolidin-4-one.
- NMR (CDCl₃) (270 MHz) δ:: 1.45 (18H, s), 1.8-2.2 (2H, m), 2.35 (3H, s), 2.4-3.0 (4H, m), 3.7-4.2 (4H, m), 5.60 (1H, s), 5.88 (2H, s), 6.1-6.8 (3H, m), 7.65 (2H, s)

### EXAMPLES 63 TO 66

Each compound shown in Table 24 was prepared according to the procedure for Example 62, using each of the compounds shown in Table 23 instead of 3-(3-bromopropyl)-5-(3,5-di-tert-butyl-4-hydroxyphenyl)-1,3,4-oxadiazol-2(3H)-one.

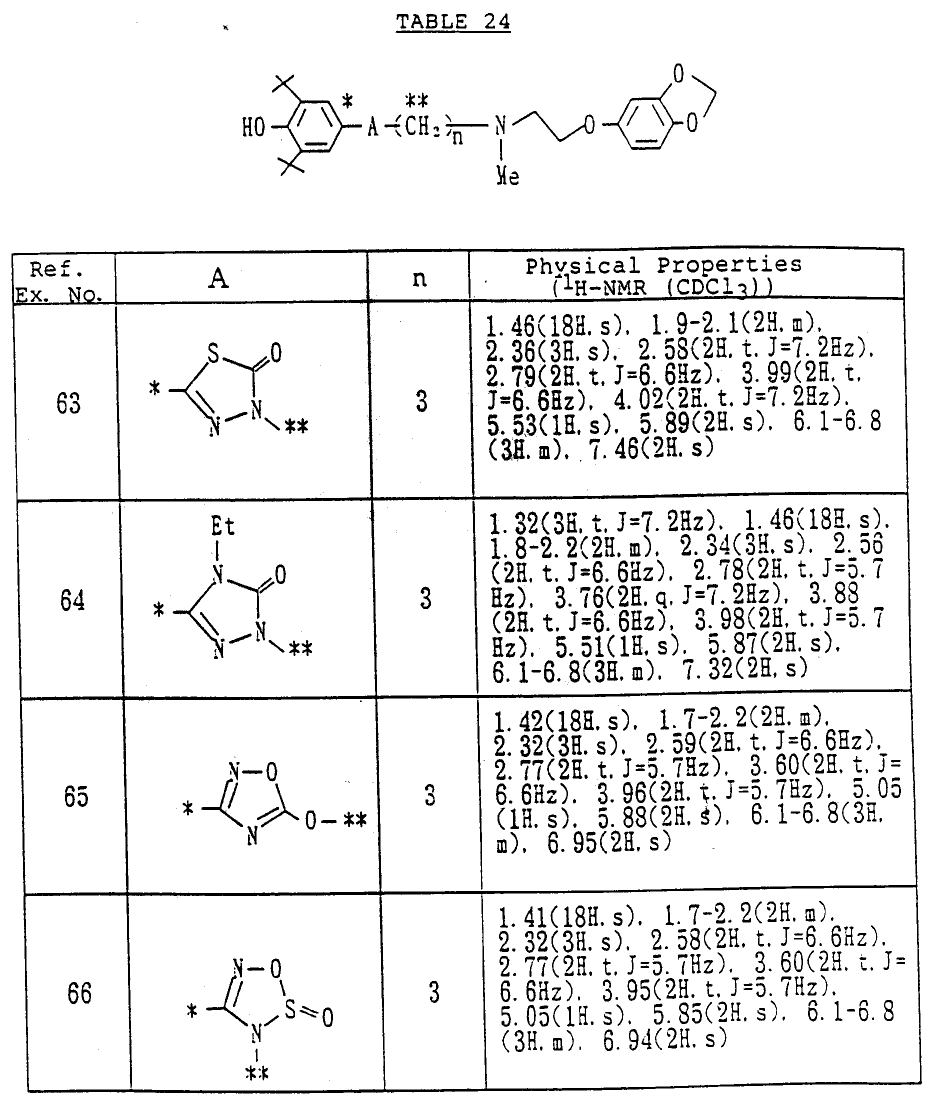

### REFERENCE EXAMPLE 59

### Preparation of 5-(3,5-Di-tert-butyl-4-hydroxyphenyl)-1-(3-hydroxypropyl)imidazole

To a solution of 3-hydroxy-N-(3,5-di-tert-butyl-4-hydroxybenzylidene)propylamine (1.50 g) in methanol (50 ml) were added tosylmethyl isocyanide (1.68 g) and potassium carbonate (1.43 g), and the mixture was stirred at room temperature for 16 hours. After completion of the reaction, the reaction mixture was poured into ice-water and extracted with dichloromethane. The organic layer was dried over anhydrous sodium sulfate, and the solvent was evaporated under reduced pressure. The resulting crude crystals were recrystallized from a chloroform/diethyl ether to afford 1.41 g (82%) of the title compound as pale yellow crystals. mp 166-167°C.
- NMR (CDCl₃) δ:: 1.43 (18H, s), 1.5-2.3 (3H, m), 3.53 (2H, t,J=6.6Hz), 4.08 (2H, t, J=6.6Hz), 5.30 (1H, brs), 6.92 (1H, brs), 7.10 (2H, s), 7.50 (1H, s)

### REFERENCE EXAMPLE 60

### Preparation of 5-(3,5-Di-tert-butyl-4-hydroxyphenyl)-1-(3-chloropropyl)imidazole

To a solution of 5-(3,5-di-tert-butyl-4-hydroxyphenyl)-1-(3-hydroxypropyl)imidazole (1.20 g) obtained in Reference Example 59 in dichloromethane (30 ml) were added thionyl chloride (0.65 g) and a catalytic amount of dimethylformamide, and the mixture was refluxed for 2 hours. After allowing to cool, the reaction mixture was poured into a 5% aqueous solution of sodium carbonate cooled with ice-water, and the product was extracted with dichloromethane. The organic layer was dried over anhydrous sodium sulfate, and the solvent was evaporated under reduced pressure. The resulting crude product was recrystallized from a dichloromethane/diethyl ether to afford 1.09 g (86%) of the title compound as pale yellow crystals. mp 178-179°C.
- NMR (CDCl₃) (60 MHz) δ:: 1.43 (18H, s), 1.6-2.8 (2H, m), 3.35 (2H, t,J=6.6Hz), 4.15 (2H, t, J=6.6Hz), 5.45 (1H, brs), 6.90 (1H, s), 7.05 (2H, s), 7.43 (1H, s)

### EXAMPLE 67

### Preparation of 5-(3,5-Di-tert-butyl-4-hydroxyphenyl)-1-[3-[N-methyl-N-[2-(3,4-methylenedioxyphenoxy)ethyl]amino]propyl]imidazole

The title compound was obtained as a colorless oil according to the procedure for Example 1, using 5-(3,5-di-tert-butyl-4-hydroxyphenyl)-1-(3-chloropropyl)imidazole obtained in Reference Example 60 instead of 2-(3,5-diisopropyl-4-hydroxyphenyl)-3-(3-chloropropyl)-1,3-thiazolidin-4-one.
- NMR (CDCl₃) (200 MHz) δ:: 1.43 (18H, s), 1.5-2.0 (2H, m), 2.16 (3H, s), 2.32 (2H, t, J=6.6Hz), 2.64 (2H, t,J=5.7Hz), 3.88 (2H, t, J=5.7Hz), 3.98 (2H, t, J=6.6Hz), 5.35 (1H, brs), 5.87 (2H, s), 6.1-6.8 (3H, m), 6.96 (1H, s), 7.12 (2H, s), 7.52 (1H, s)

### EXAMPLE 68

### Preparation of 2-(3,5-Di-tert-butyl-4-hydroxyphenyl)-3-[3-[N-methyl-N-[2-(3,4-methylenedioxyphenoxy)ethyl]amino]propyl]-1,3-thiazolidin-4-one-1-oxide

To a solution of 2-(3,5-di-tert-butyl-4-hydroxyphenyl)-3-[3-[N-methyl-N-[2-(3,4-methylenedioxyphenoxy)ethyl]amino]propyl]-1,3-thiazolidin-4-one (0.30 g) obtained in Example 26-A or 26-B in acetic acid (5 ml) was added 35% aqueous solution of hydrogen peroxide (0.20 g), and the mixture was stirred at room temperature overnight. After completion of the reaction, the reaction mixture was added to a mixture of ethyl acetate and a 5% aqueous solution of sodium carbonate and stirred. The organic layer was separated, dried over anhydrous sodium sulfate. The solvent was evaporated under reduced pressure, and the residue was purified by silica gel column chromatography (eluent; chloroform-methanol, 98:2) to afford 0.12 g (39%) of the title compound as a colorless oil.
- MS (m/z):: 558 (M⁺)
- NMR (CDCl₃) (200 MHz) δ:: 1.41 (18H, s), 1.4-2.0 (2H, m), 2.28 (3H, s), 2.3-2.7 (2H, m), 2.72 (2H, t, J=5.7Hz), 2.9-3.2 (1H, m), 3.37 and 3.69 (2H, ABq, J=16Hz), 3.8-4.2 (3H, m), 5.40 (1H, s), 5.61 (1H, s), 5.87 (2H, s), 6.1-6.8 (3H, m), 6.94 (2H, s)

### REFERENCE EXAMPLE 61

### Preparation of 2-(3,5-Di-tert-butyl-4-hydroxyphenyl)-1,3-thiazolidine Hydrochloride

In a mixed solvent of methanol (100 ml) and tetrahydrofuran (100 ml) was dissolved 5.0 g of 3,5-di-tert-butyl-4-hydroxybenzaldehyde (5.0 g), and solution of 2-aminoethanethiol (1.73 g) in methanol (10 ml) was added. Then the mixture was stirred at room temperature for 2 hours. After completion of the reaction, the solvent was evaporated under reduced pressure, and 200 ml of ice-water was added to the residue. The product was extracted with chloroform, and the organic layer was dried over anhydrous sodium sulfate. The solvent was evaporated under reduced pressure, and methanol-hydrochloric acid (5 ml) was added to the residue. The mixture was triturated with diethyl ether to yield 5.81 g (82%) of the title compound as a white powder.
- NMR (CDCl₃) (200 MHz) δ:: 1.43 (18H, s), 2.9-3.2 (2H, m), 3.6-3.8 (1H, m), 3.8-4.0 (1H, m), 5.20 (1H, s), 5.46 (1H, s), 7.2-7.4 (2H, m)

### REFERENCE EXAMPLE 62

### Preparation of 2-(3,5-Di-tert-butyl-4-hydroxyphenyl)-3-acryloyl-1,3-thiazolidine

To a suspension of 2-(3,5-di-tert-butyl-4-hydroxyphenyl)-1,3-thiazolidine hydrochloride (1.65 g) obtained in Reference Example 61 in tetrahydrofuran (20 ml) was added triethylamine (1.52 g), and acryloyl chloride (0.63 g) was added thereto at 0°C. The mixture was stirred at that temperature for 3 hours. After completion of the reaction, the reaction mixture was poured into ice-water, and the product was extracted with chloroform. The organic layer was washed successively with 1N hydrochloric acid, a saturated aqueous solution of sodium hydrogencarbonate, and brine, then dried over anhydrous sodium sulfate. The solvent was evaporated under reduced pressure, and the residue was purified by silica gel column chromatography (eluent; chloroform-methanol, 98:2) to afford 0.93 g (54%) of the title compound as colorless crystals. mp 147-149°C.
- NMR (CDCl₃) (200 MHz) δ:: 1.40 (18H, s), 3.0-3.2 (2H, m), 3.8-4.0 (1H, m), 4.2-4.5 (1H, m), 5.20 (1H, brs), 5.5-5.8 (1H, m), 5.9-6.6 (3H, m), 6.98 (2H, brs)

### EXAMPLE 69

### Preparation of 2-(3,5-Di-tert-butyl-4-hydroxyphenyl)-3-[3-[N-methyl-N-[2-(3,4-methylenedioxyphenoxy)ethyl]amino]propionyl]-1,3-thiazolidine

To a solution of 2-(3,5-di-tert-butyl-4-hydroxyphenyl)-3-acryloyl-1,3-thiazolidine (0.35 g) obtained in Reference Example 62 in chloroform (5 ml) was added N-methyl-2-(3,4-methylenedioxyphenoxy)ethylamine (0.20 g), and the mixture was refluxed for 3 hours. After completion of the reaction, the solvent was evaporated under reduced pressure, and the residue was purified by silica gel column chromatography (eluent; chloroform-methanol, 98:2) to afford 0.41 g (76%) of the title compound as a colorless oil.
- NMR (CDCl₃) (200 MHz) δ:: 1.41 (18H, s), 2.14 (3H, s), 2.2-2.7 (4H, m), 2.7-2.9 (2H, m), 2.9-3.2 (2H, m), 3.8-4.1 (3H, m), 4.2-4.4 (1H, m), 5.1-5.3 (1H, m), 5.89 (2H, s), 6.02 (1H, brs), 6.2-7.1 (5H, m)

### EXAMPLE 70

### Preparation of (+)-2-(3,5-Di-tert-butyl-4-hydroxyphenyl)-3-[3-[N-methyl-N-[2-(3,4-methylenedioxyphenoxy)ethyl]amino]propyl]-1,3-thiazolidin-4-one

The compound obtained in Example 26-A or 26-B and weighing 400 mg was subjected to HPLC using a column for optical resolution (CHIRALCEL OD; 2 cm (diameter) x 25 cm) in several tens of divided fractions under conditions of a mobile phase of n-hexane-isopropyl alcohol, (80:20), a flow rate of 16 ml/min, and a detection wavelength of 280 nm to recover 180 mg of the title compound. The specific rotation of the hydrochloride of the resulting compound is as follows.
[α]_{D} = +36.14 (EtOH, c=0.332)

### EXAMPLE 71

### Preparation of (-)-2-(3,5-Di-tert-butyl-4-hydroxyphenyl)-3-[3-[N-methyl-N-[2-(3,4-methylenedioxyphenoxy)ethyl]amino]propyl]-1,3-thiazolidin-4-one

The compound obtained in Examples 26-A or 26-B and weighing 400 mg was subjected to HPLC in several tens of divided fractions under the same conditions as in Example 70 to obtain 170 mg of the title compound. The hydrochloride of the resulting compound had the following specific rotation.
[α]_{D} = -36.72 (EtOH, c=0.610)

### EXAMPLE 72

### Preparation of 2,5-Trans-2-(3,5-di-tert-butyl-4-hydroxyphenyl)-3-[3-[N-methyl-[2-(3,4-methylenedioxyphenoxy)ethyl]amino]propyl]-5-methyl-1,3-thiazolidin-4-one

The compound obtained in Example 41-A or 41-B and weighing 500 mg was subjected to HPLC using a silica gel column (YMC-PackSIL SH-043-5, 2 cm (diameter) x 25 cm) in several tens of divided fractions under conditions of a mobile phase of chloroform-isopropanol, (97:3), a flow rate of 12 ml/min, and a detection wavelength of 280 nm to recover 150 mg of the title compound. The stereochemistry was assumed from the ¹H-NMR data of M.R. Johnson, et al. (M.R. Johnson, et al., J. Org. Chem., 48, 494 (1983)).
- NMR (CDCl₃) (270 MHz) δ:: 1.41 (18H, s), 1.5-1.9 (2H, m), 1.58 (3H, d, J=6.9Hz), 2.23 (3H, s), 2.3-2.5 (2H, m), 2.69 (2H, t, J=5.6Hz), 2.7-2.9 (1H, m), 3.5-3.7 (1H, m), 3.94 (2H, t, J=5.6Hz), 4.02 (1H, dq, J=1.7, 6.9Hz), 5.28 (1H, s), 5.57 (1H, d, J=1.7Hz), 5.90 (2H, s), 6.2-6.8 (3H, m), 7.05 (2H, s)

### EXAMPLE 73

### Preparation of 2,5-Cis-2-(3,5-di-tert-butyl-4-hydroxyphenyl)-3-[3-[N-methyl-N-[2-(3,4-methylenedioxyphenoxy)ethyl]amino]propyl]-5-methyl-1,3-thiazolidin-4-one

The compound obtained in Example 41-A or 41-B and weighing 500 mg was subjected to HPLC in several tens of divided fractions under the same conditions as in Example 72 to obtain 140 mg of the title compound. The stereochemistry was assumed from the ¹H-NMR data of M.R. Johnson, et al. (M.R. Johnson, et al., J. Org. Chem., 48, 494 (1983)).
- NMR (CDCl₃) (270 MHz) δ:: 1.43 (18H, s), 1.2-2.0 (2H, m), 1.65 (3H, d, J=6.9Hz), 2.20 (3H, m), 2.2-2.5 (2H, m), 2.67 (2H, t, J=5.6Hz), 2.7-3.0 (1H, m), 3.4-3.8 (1H, m), 3.92 (2H, t, J=5.6Hz), 4.02 (1H, q, J=6.9Hz), 5.30 (1H, s), 5.55 (1H, s), 5.91 (2H, s), 6.2-6.8 (3H, m), 7.12 (2H, s)

### EXAMPLE 74

### Preparation of (+)-2,5-Cis-2-(3,5-di-tert-butyl-4-hydroxyphenyl)-3-[3-[N-methyl-N-[2-(3,4-methylenedioxyphenoxy)ethyl]amino]propyl]-5-methyl-1,3-thiazolidin-4-one

The compound obtained in Example 73 was subjected to HPLC in several tens of divided fractions under the same conditions as in Example 70 to recover the title compound. The resulting compound had the following specific rotation.
[α]_{D} = +27.59 (CHCl₃, C=1.000)

### EXAMPLE 75

### Preparation of (-)-2,5-Cis-2-(3,5-di-tert-butyl-4-hydroxyphenyl)-3-[3-[N-methyl-N-[2-(3,4-methylenedioxyphenoxy)ethyl]amino]propyl]-5-methyl-1,3-thiazolidin-4-one

The compound obtained in Example 73 was subjected to HPLC in several tens of divided fractions under the same conditions as in Example 70 to recover the title compound. The resulting compound had the following specific rotation.
[α]_{D} = -28.39 (CHCl₃, c=1.000)

Test Examples are described below in order to demonstrate that the compounds having formula (I) according to the present invention exhibit the three actions, i.e., an inhibitory action on lipid peroxidation, a vasorelaxing action, and an inhibition of calcium overload.

### TEST EXAMPLE 1

### Inhibitory action on lipid peroxidation in vitro

### Method A:

A test compound was added to rabbit LDL prepared according to the method of Havel, et al. (Havel RJ et al., J. Clin. Invest., 34, 1345 (1955)), and then a soybean lipoxygenase type-IS (SLO) was added to a final concentration of 40 µg/ml. Oxydation of LDL was carried out by incubating at 37°C in a CO₂ incubator for 24 hours. The oxidized LDL solution was analyzed by gel-permeation chromatography, and the fluorescence intensity of the LDL fraction was measured at an excitation wavelength of 360 nm and an emission wavelength of 430 nm. The results of measurement expressed as a percentage of control, are shown in Table 25.

**TABLE 25**

| Compound (Example No.) | Percent (%) of Control | |
|---|---|---|
| | 5 x 10⁻⁶ M | 5 x 10⁻⁷ M |
| None (Control) | 100 | 100 |
| Compound (9) | 5.9 | 13.5 |
| Compound (12) | 6.4 | 16.8 |
| Compound (17) | 3.9 | 16.4 |
| Compound (20) | 0.6 | 8.7 |
| Compound (41) | 6.2 | 18.3 |
| Compound (57) | 11.3 | 22.8 |
| Compound (70) | 5.1 | 15.2 |
| Compound (71) | 2.8 | 12.7 |
| Diltiazem | 110.7 | 109.1 |

### Method B:

A test compound was added to rabbit LDL prepared according to the method of Havel, et al. (Havel RJ, et al., J. Clin. Invest., 34, 1345 (1955)), and then CuSO₄ was added to a final concentration of 1 µM. Oxidation of LDL was performed by shaking at 37°C for 24 hours. The generated TBARS was measured by the method of Yagi utilizing fluorometry (Yagi, K., Biochem. Med., 15, 212 (1976)). The results of measurement expressed as a percentage of control, are shown in Table 26.

**TABLE 26**

| Compound (Example No.) | % of Control 1 x 10⁻⁵ M |
|---|---|
| None (Control) | 100 |
| Compound (9) | 26.4 |
| Compound (12) | 25.9 |
| Compound (17) | 21.0 |
| Compound (20) | 21.3 |
| Compound (41) | 26.3 |
| Compound (57) | 23.0 |
| Compound (70) | 21.1 |
| Compound (71) | 23.8 |
| Compound (46) | 20.4 |
| Compound (49) | 15.7 |
| Compound (34) | 20.5 |
| Compound (55) | 20.0 |
| Compound (51) | 16.6 |
| Diltiazem | 96.9 |

### TEST EXAMPLE 2

### Vasorelaxing action in vitro

### Method:

The thoracic aorta was excised from a Sprague-Dawley (Crj) male rat weighing between 350 and 550 g, and dissected free from surrounding connective tissue. The vessel was cut into a ring having a width of 2 to 3 mm. The resulting preparation was mounted for isometric tension recording in an organ bath filled with 10 ml of a Krebs-Henseleit solution (K-H solution, pH 7.4, 37°C), which was bubbled with 95% O₂/5% CO₂. Isometric tension changes were monitored with an isometric transducer (TB-611T, manufactured by Nihon Kohden Corp.). Before starting the experiment, the preparation was given a stretched tension of 2 g and allowed to equilibrate for 30 minutes (the K-H solution was exchanged for a fresh one every 15 minutes). At first, the preparation was precontracted by changing the solution in the bath to one containing 30 mM K⁺. After the contraction was maintained for 20 minutes, the preparation was washed with K-H solution. Sixty minutes thereafter (the K-H solution was exchanged with a fresh one every 20 minutes), contraction was again induced in the same manner as described above. After the contraction stabilized, a test compound or diltiazem was added to the system in a cumulative manner in half log-unit increments to obtain a concentration-response curve.

Taking the contraction at 30 mM K⁺ as 100%, the concentration of the drug at which the contraction is relaxed to 50% was obtained as IC₅₀. The results obtained are shown in Table 27.

**TABLE 27**

| Compound (Example No.) | Vasorelaxing Activity, IC₅₀ (µM) |
|---|---|
| Compound (26) | 0.037 |
| Compound (1) | 0.17 |
| Compound (8) | 0.17 |
| Compound (9) | 0.028 |
| Compound (11) | 0.021 |
| Compound (12) | 0.063 |
| Compound (17) | 0.028 |
| Compound (19) | 0.11 |
| Compound (41) | 0.056 |
| Compound (72) | 0.098 |
| Compound (73) | 0.049 |
| Compound (70) | 0.30 |
| Compound (71) | 0.027 |
| Compound (55) | 0.021 |
| Compound (49) | 0.023 |
| Compound (74) | 0.141 |
| Compound (75) | 0.022 |
| Diltiazem | 0.11 |

### TEST EXAMPLE 3

### Inhibitory action on calcium overload in vitro

### Method A:

Incubated ventricular myocytes were prepared from the heart of male Sprague-Dawley rat weighing 300 to 500 g, using an enzyme-perfusion method. Thus obtained rod-shaped normal myocytes were treated with a test compound or diltiazem for 30 minutes, and 100 µg/ml of veratrine was added. Five minutes later, the shape of the cells was observed to obtain a survival rate thereby to evaluate the efficacy of the compound. The results obtained are shown in Table 28.

**TABLE 28**

| Compound (Example No.) | Survival Rate of Cells |
|---|---|
| Compound (26) | ++ |
| Compound (8) | ++ |
| Compound (9) | ++ |
| Compound (12) | ++ |
| Compound (20) | + |
| Diltiazem | - |

| | |
|---|---|
| Note: ++: The cells survived almost completely at 10⁻⁷ M. | |
| +: The cells survived almost completely at 10⁻⁸ M. | |
| -: The cells died almost completely at 10 M⁻⁸ M. | |

### Method B:

The efficacy was evaluated in the same manner as in Method A, except for using 50 µg/ml of veratridine in place of veratrine (100 µg/ml). The results obtained are shown in Table 29.

**TABLE 29**

| Compound (Example No.) | Concentration at Which Cells Survived Almost Completely (µM) |
|---|---|
| Compound (17) | 0.32 |
| Compound (34) | 0.32 |
| Compound (72) | 0.32 |
| Compound (74) | 0.32 |
| Compound (75) | 0.32 |
| Diltiazem | -* |

| | |
|---|---|
| Note: *: The cells died almost completely at 1 µM. | |

The compounds of the foregoing Examples are shown in Tables 30 through 37 below.

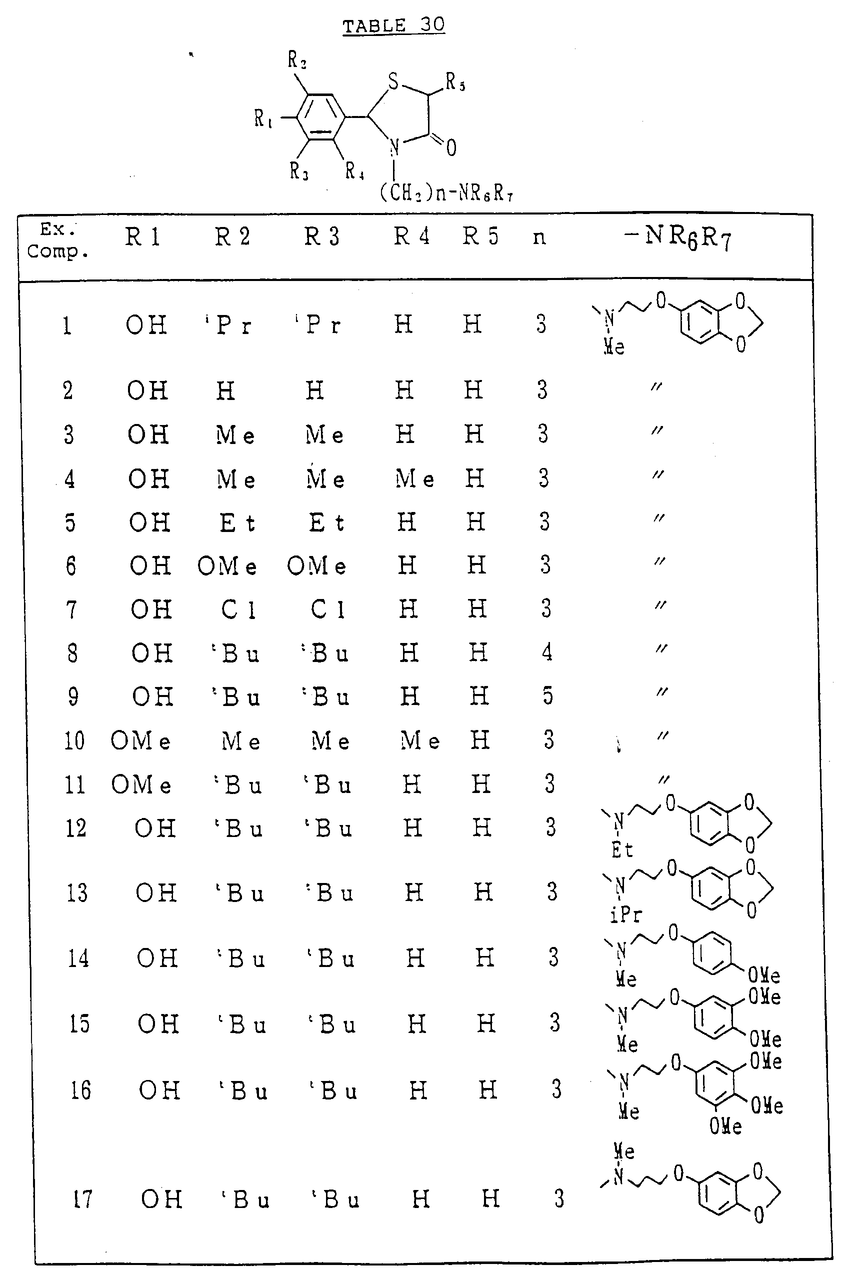

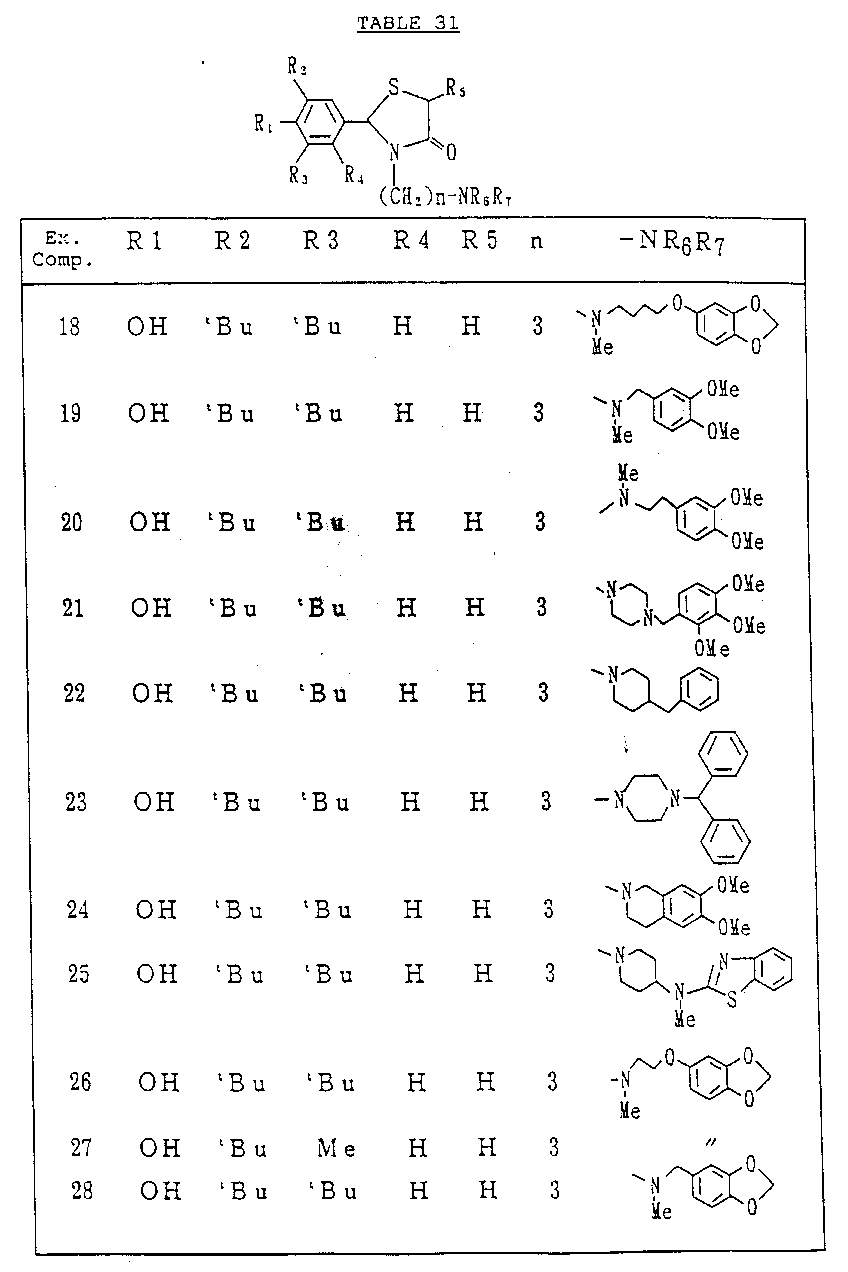

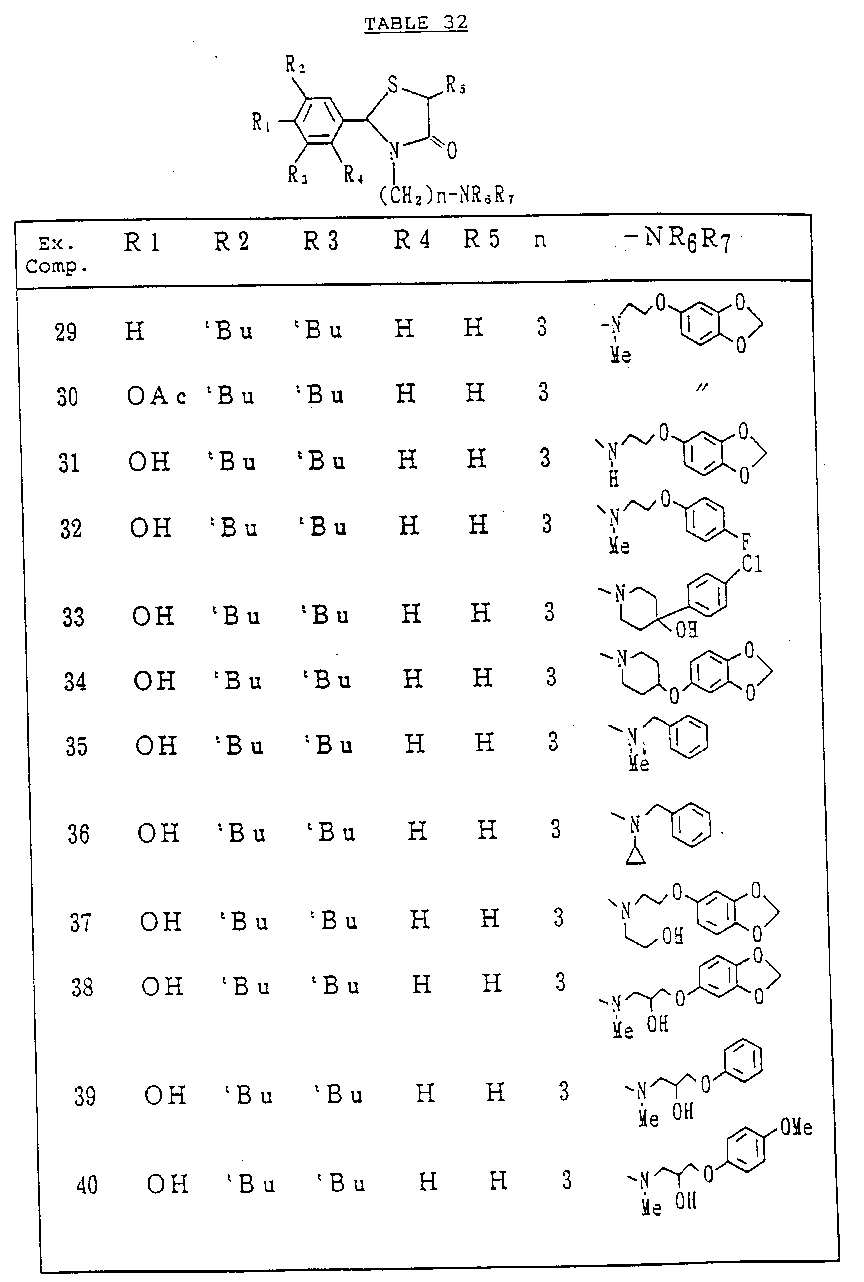

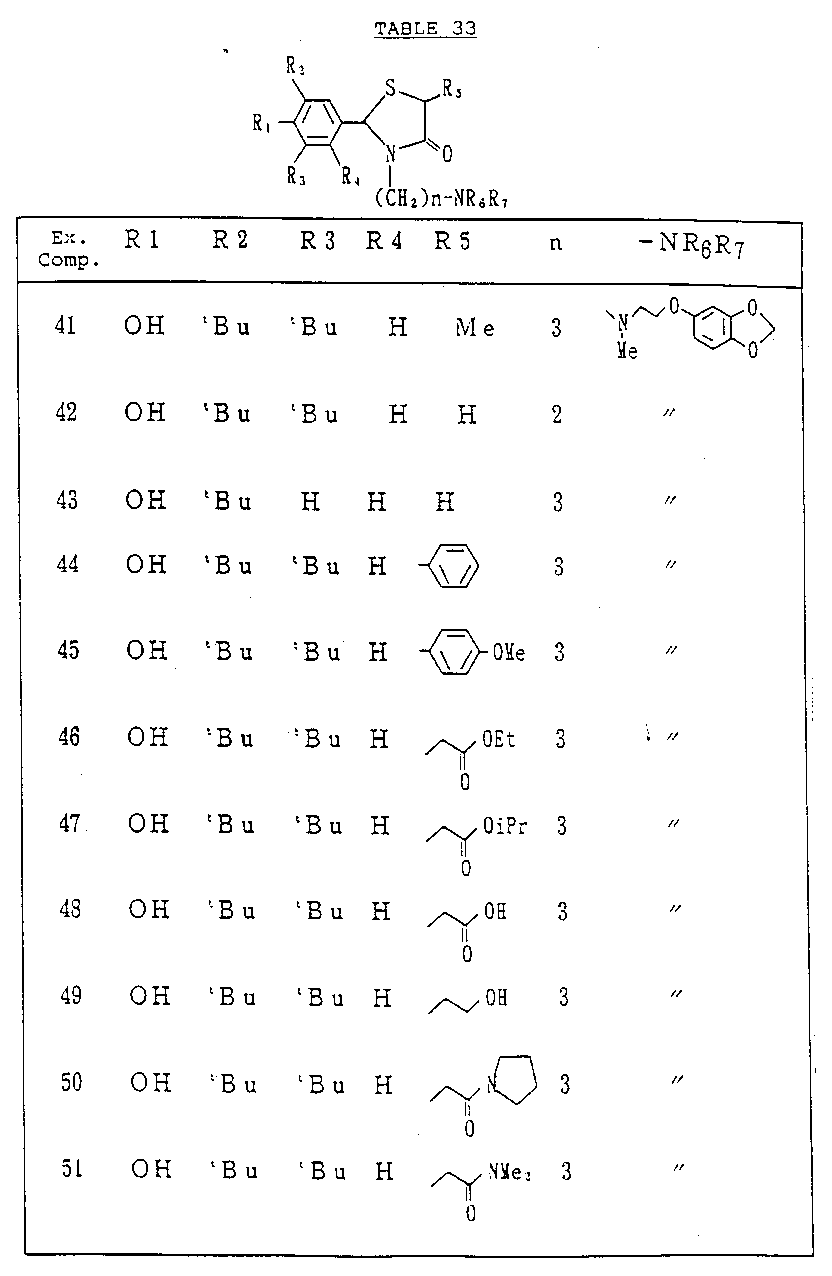

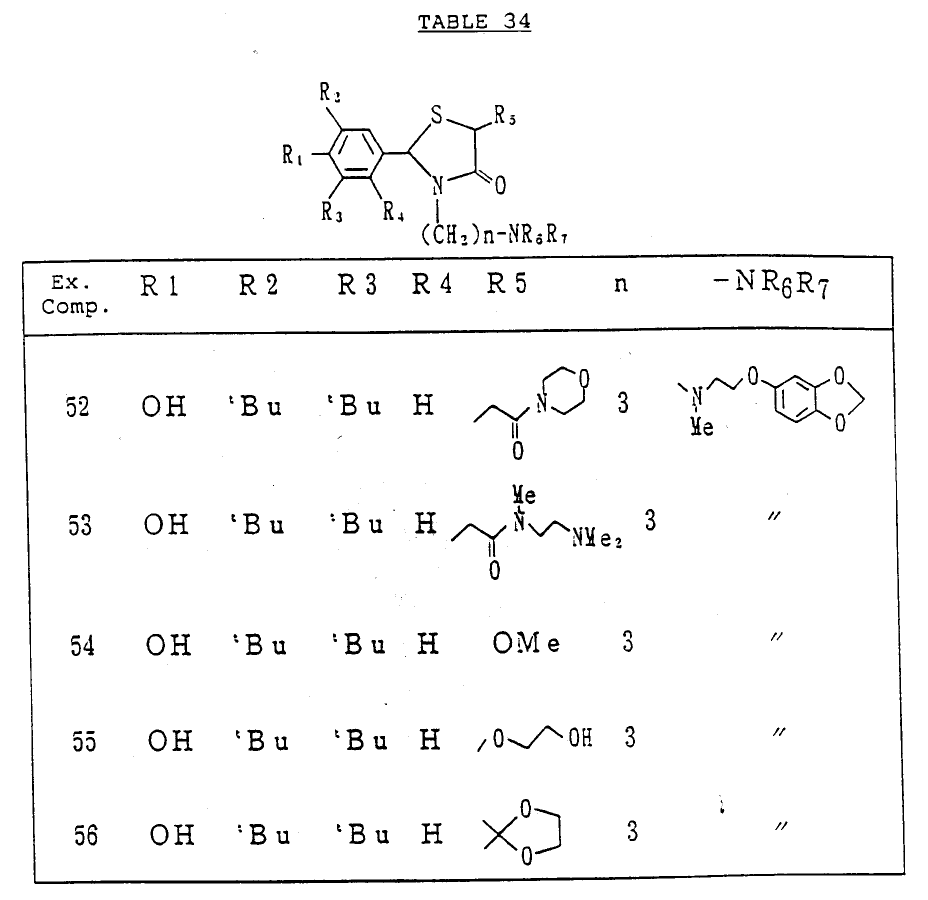

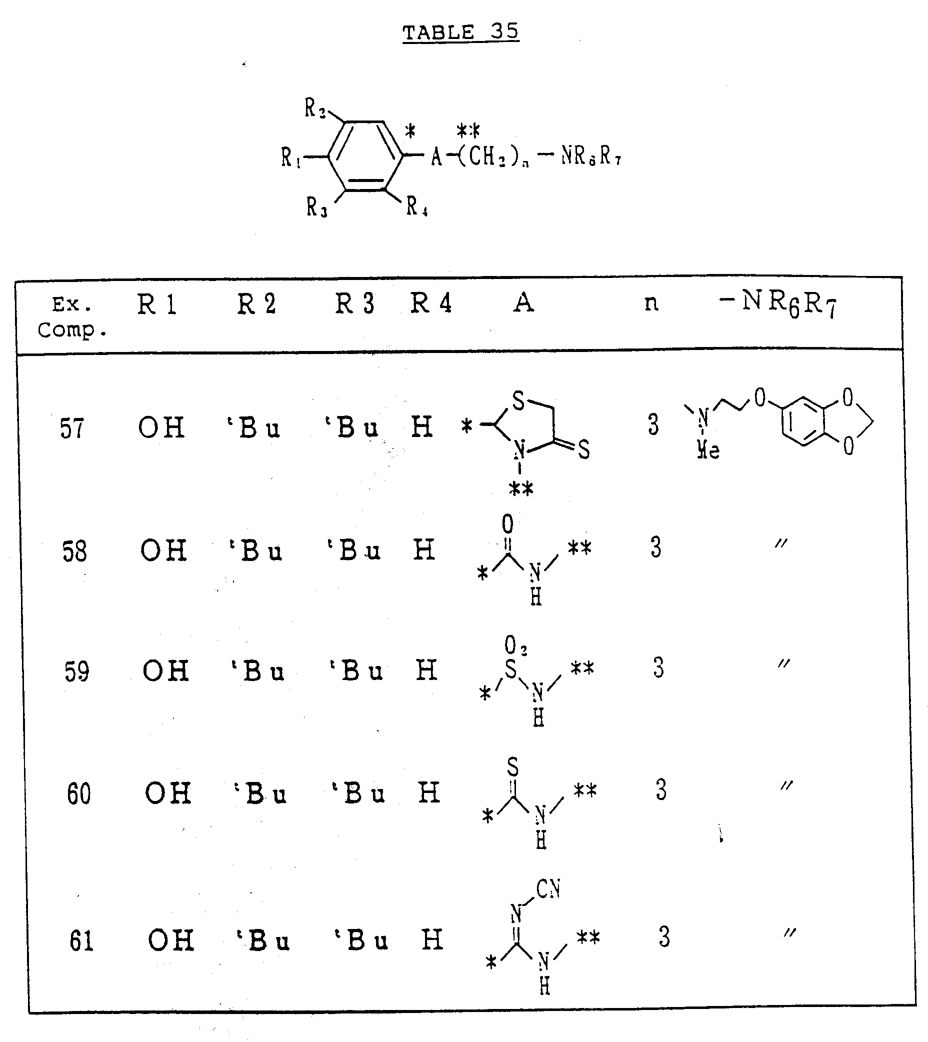

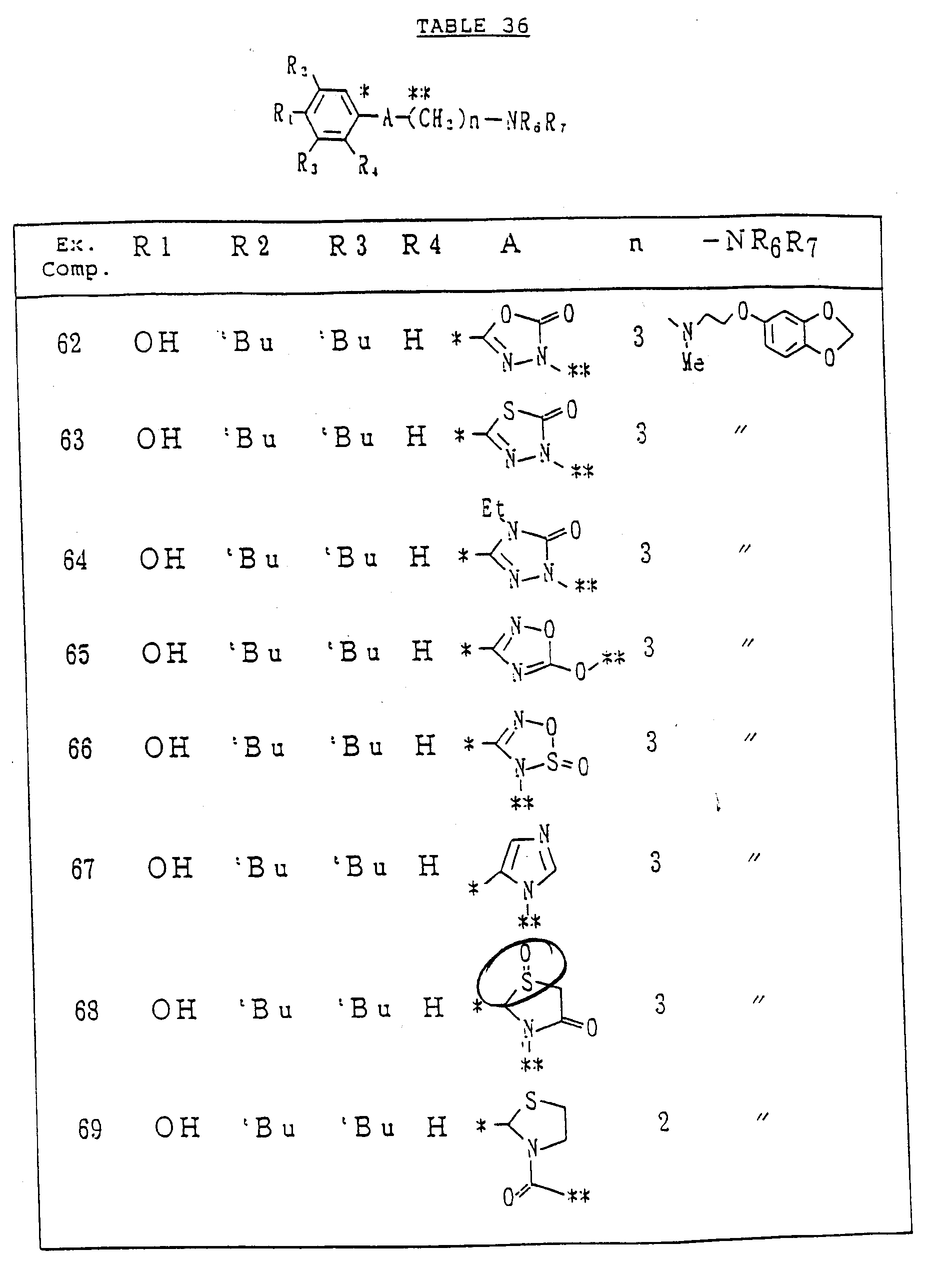

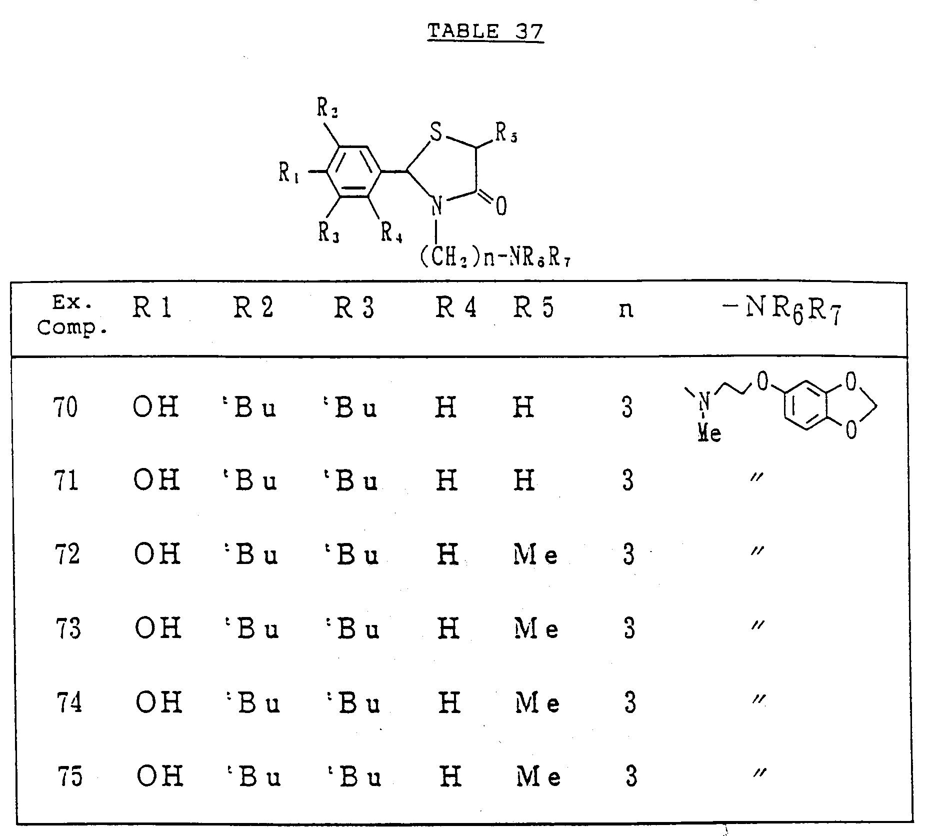

### Industrial Applicability

As has been described, the compound according to the present invention has an inhibitory action on lipid peroxidation, a vasorelaxing action, and an inhibitory action on calcium overload and can therefore be used as a preventive or treating agent for ischemic diseases and a hypertension.

## Claims

1. A compound represented by formula (I): 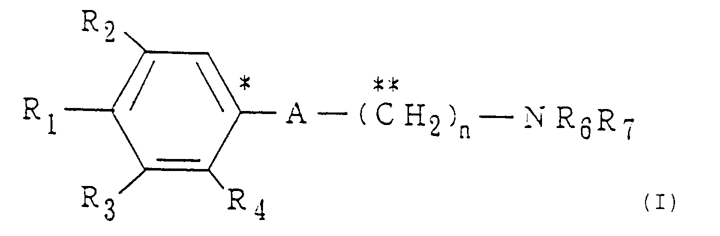 wherein
R₁ represents a hydrogen atom, a hydroxyl group, an acyloxy group having 1 to 9 carbon atoms or a lower alkoxy group having 1 to 6 carbon atoms;
R₂ and R₃, which may be the same or different, each represents a hydroxy group, an isopropyl group or a t-butyl group;
R₄ represents a hydrogen atom, a lower alkyl group having 1 to 6 carbon atoms;
A represents a fragment represented by formula (II): 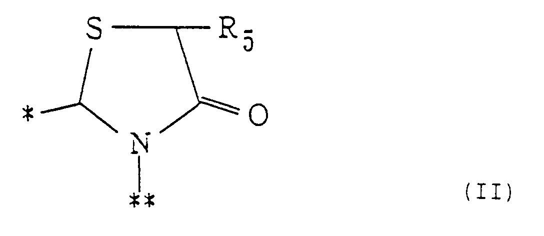 wherein
R₅ represents a hydrogen atom, a substituted or unsubstituted lower alkyl group having 1 to 6 carbon atoms, a substituted or unsubstituted lower alkenyl group having 1 to 6 carbon atoms, a substituted or unsubstituted alkoxy group having 1 to 6 carbon atoms, a substituted or unsubstituted aryl group or a substituted or unsubstituted heterocyclic group, or a 5- or 6-membered ring containing two or more oxygen atoms or sulfur atoms, in which case the carbon atom to which it is bonded is a spiro atom; or
a fragment represented by formula (III):
B (III)
wherein
B represents a fragment selected from the following group of fragments represented by formula (IV), (V), (VI), (VII), (VIII), (X), (XI), (XII), (XIII), (XIV), (XV), or (XVI): 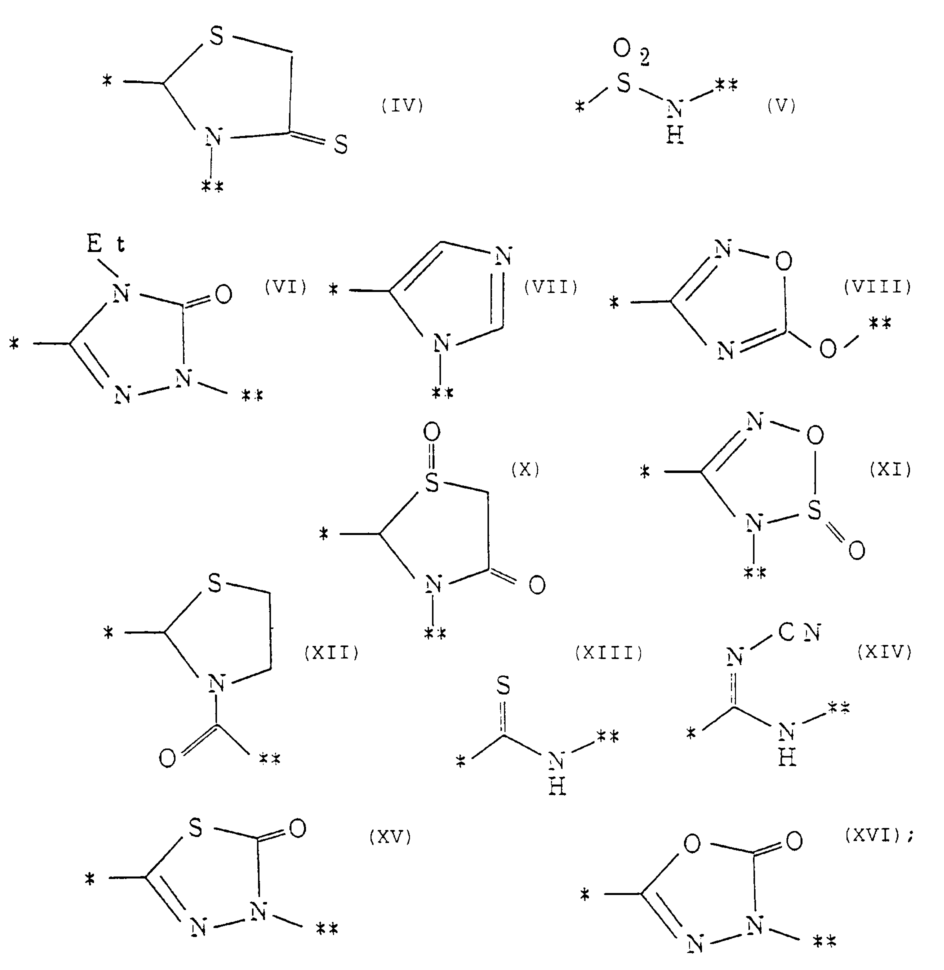
R₆ and R₇, which may be the same or different, each represents a hydrogen atom, a substituted or unsubstituted lower alkyl group having 1 to 6 carbon atoms, a substituted or unsubstituted lower alkenyl group having 1 to 6 carbon atoms, a substituted or unsubstituted aryl group or a substituted or unsubstituted heterocyclic group, provided that R₆ and R₇ do not both represent a methyl group, or
R₆ and R₇ are taken together to form a substituted or unsubstituted ring which may be a condensed ring; and
n represents an integer of 2, 3, 4, 5 or 6,
or a possible stereoisomer or optical isomer thereof or a pharmaceutically acceptable salt thereof.

2. The compound according to claim 1, wherein R₂ and R₃ both represent a t-butyl group.

3. The compound according to claim 1, wherein R₁ represents a hydroxy group, an acyloxy group having 1 to 9 carbon atoms or a lower alkoxy group having 1 to 6 carbon atoms.

4. The compound according to claim 3, wherein R₂ and R₃, which may be the same or different, each represents an isopropyl group or a t-butyl group, R₄ represents a hydrogen atom or methyl group, A represents a fragment represented by formula (II), (IV), (V), (VI), (VII) or (VIII) and n represents an integer of 2, 3, 4 or 5.

5. The compound according to claim 4, wherein A represents a fragment represented by formula (II) or (IV).

6. The compound according to claim 5, wherein R₂ and R₃ both represent a t-butyl group, R₄ represents a hydrogen atom, and A represents a fragment represented by formula (II).

7. The compound according to claim 6, wherein R₁ represents a hydroxy group or a methoxy group.

8. The compound according to claim 7, wherein R₁ represents a hydroxy group.

9. The compound according to claim 8, wherein n represents 3.

10. The compound according to claim 3, wherein R₁ represents an acyloxy group having 1 to 9 carbon atoms, R₂ and R₃ both represent a t-butyl group, R₄ represents a hydrogen atom, A represents a fragment represented by formula (II) and n represents an integer of 2,3, 4 or 5.

11. The compound according to claim 3, wherein R₁ represents a lower alkoxy group having 1 to 6 carbon atoms, R₂ and R₃ both represent a t-butyl group, R₄ represents a hydrogen atom, A represents a fragment represented by formula (II) and n represents an integer of 2, 3, 4 or 5.

12. The compound according to claim 11, wherein R₁ represents a methoxy group.

13. The compound according to claim 6, wherein R₅ represents a hydrogen atom, a substituted or unsubstituted lower alkyl group having 1 to 6 carbon atoms, a substituted or unsubstituted lower alkoxy group having 1 to 6 carbon atoms or a substituted or unsubstituted aryl group, or a 5- or 6-membered ring containing two or more oxygen atoms or sulfur atoms, in which case the carbon atom to which it is bonded is a spiro atom; R₆ and R₇, which may be the same or different, each represents a hydrogen atom or a substituted or unsubstituted lower alkyl group having 1 to 6 carbon atoms, provided that R₆ and R₇ do not both represent a methyl group or R₆ and R₇ are taken together to form a substituted or unsubstituted ring which may be a condensed ring.

14. The compound according to claim 13, wherein R₆ and R₇, which may be the same or different, each represents a substituted or unsubstituted lower alkyl group having 1 to 6 carbon atoms, provided that R₆ and R₇ do not both represent a methyl group or R₆ and R₇ are taken together to form a substituted or unsubstituted ring which may be a condensed ring.

15. The compound according to claim 14, wherein R₆ represents a substituted or unsubstituted lower alkyl group having 1 to 3 carbon atoms; R₇ represents a group selected from the set of groups consisting of a 2-(3,4-methylenedioxyphenoxy)ethyl group, a 3-(3,4-methylenedioxyphenoxy)propyl group, a 4-(3,4-methylenedioxyphenoxy)-n-butyl group, and a 3, 4-dimethoxyphenylmethyl group; or -NR₆R₇ represents a group selected from the set of groups consisting of a 4-(N-2-benzothiazolyl-N-methylamino)piperidyl group, a 4-phenylmethylpiperidyl group, a 4-(3,4-methylenedioxyphenoxy)piperidyl group, and a 4-(2,3,4-trimethoxyphenylmethyl)piperazinyl group.

16. The compound according to claim 15, wherein R₆ represents a methyl group or an ethyl group.

17. The compound according to claim 13, wherein R₅ represents a hydrogen atom, a substituted or unsubstituted lower alkyl group having 1 to 6 carbon atoms, a substituted or unsubstituted lower alkoxy group having 1 to 6 carbon atoms, or a 5- or 6-membered ring containing two or more oxygen atoms or sulfur atoms, in which case the carbon atom to which it is bonded is a spiro atom; R₆ represents a methyl group; R₇ represents a group selected from the set of groups consisting of a 2-(3,4-methylenedioxyphenoxy)ethyl group, a 3-(3,4-methylenedioxyphenoxy)propyl group, a 4-(3,4-methylenedioxyphenoxy)-n-butyl group, and a 3, 4-dimethoxyphenylmethyl group; or -NR₆R₇ represents a group selected from the set of groups consisting of a 4-(N-2-benzothiazolyl-N-methylamino)piperidyl group, a 4-phenylmethylpiperidyl group, a 4-(3,4-methylenedioxyphenoxy)piperidyl group, and a 4-(2,3,4-trimethoxyphenylmethyl)piperazinyl group.

18. The compound according to any one of claims 13-17, wherein n represents 3.

19. A compound according to claim 1, which is selected from the group of compounds consisting of 2-(3,5-di-t-butyl-4-hydroxyphenyl)-3-[3-[N-methyl-N-[2-(3,4-methylenedioxy-phenoxy)ethyl]amino]propyl]-1,3-thiazolidin-4-one, (+)-2-(3,5-di-t-butyl-4-hydroxyphenyl)-3-[3-[N-methyl-N-[2-(3,4-methylenedioxyphenoxy)ethyl]amino]propyl]-1,3-thiazolidin-4-one, (-)-2-(3,5-di-t-butyl-4-hydroxy-phenyl)-3-[3-[N-methyl-N-[2-(3,4-methylenedioxyphenoxy)ethyl]amino]propyl]-1,3-thiazolidin-4-one, 2-(3,5-di-t-butyl-4-hydroxyphenyl)-3-[3-[N-ethyl-N-[2-(3,4-methylenedioxy-phenoxy)ethyl]amino]propyl]-1,3-thiazolidin-4-one, 2-(3,5-di-t-butyl-4-hydroxyphenyl)-3-[4-[N-methyl-N-[2-(3,4-methylenedioxyphenoxy)ethyl]amino]butyl]-1,3-thiazolidin-4-one, 2-(3,5-di-t-butyl-4-hydroxyphenyl)-3-[5-[N-methyl-N-[2-(3,4-methylenedioxyphenoxy)ethyl]amino]pentyl]-1,3-thiazolidin-4-one, 2-(3,5-di-t-butyl-4-hydroxyphenyl)-3-[3-[N-methyl-N-[3-(3,4-methylenedioxyphenoxy)propyl]amino]propyl]-1,3-thiazolidin-4-one, 2-(3,5-diisopropyl-4-hydroxyphenyl)-3-[3-[N-methyl-N-[2-(3,4-methylenedioxyphenoxy)ethyl]amino]propyl]-1,3-thiazolidin-4-one, 2-(3,5-di-t-butyl-4-hydroxyphenyl)-3-[3-[1-[4-(2,3,4-trimethoxybenzyl)piperazinyl]]propyl]-1,3-thiazolidin-4-one, 2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, 2,5-cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one (-)-2,5-cis-2-(3,5-di-t-butyl-4-hydroxyphenyl)-3-[3-[N-methyl-N-[2-(3,4- methylenedioxyphenoxy)ethyl]amino]propyl]-5-methyl-1,3-thiazolidin-4-one, (+)-2,5-cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, 2,5-trans-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, 2-(3,5-di-t-butyl-4-hydroxyphenyl)-3-[3-[N-methyl-N-[4-(3,4-methylenedioxyphenoxy)butyl]amino]propyl]-1,3-thiazolidin-4-one, 5-(3,5-di-t-butyl-4-hydroxyphenyl)-1-[3-[N-methyl-N-[2-(3,4-methylenedioxyphenoxy)ethyl]amino]propyl]imidazole, 2-(3,5-di-t-butyl-4-hydroxyphenyl)-3-[3-[N-methyl-N-[2-(3,4-methylenedioxyphenoxy)ethyl]amino]propyl]-1,3-thiazolidin-4-thione, 5-(3,5-di-t-butyl-4-hydroxy-phenyl)-4-ethyl-2-[3-[N-methyl-N-[2-(3,4-methylenedioxyphenoxy)ethyl]amino]propyl]-1,2,4-triazol-3-one, 3-(3,5-di-t-butyl-4-hydroxyphenyl)-5-[3-[N-methyl-N-[2-(3,4-methylenedioxy-phenoxy)ethyl]amino]propoxy]-1,2,4-oxadiazole, 2-(3,5-di-t-butyl-4-hydroxyphenyl)-3-[3-[N-methyl-N-[2-(3,4-methylenedioxyphenoxy)ethyl]amino]propyl]-5-(2-hydroxy-ethyl)-1,3-thiazolidin-4-one, 2-(3,5-di-t-butyl-4-hydroxyphenyl)-3-[3-[N-methyl-N-[2-(3,4-methylenedioxyphenoxy)ethyl]amino]propyl]-5-ethoxycarbonylmethyl-1,3-thiazolidin-4-one, N-[3-[N-methyl-N-[2-(3,4-methylenedioxyphenoxy)ethyl]amino]propyl]-3,5-di-t-butyl-4-hydroxybenzenesulfonamide, 2-(3,5-di-t-butyl-4-hydroxyphenyl)-3-[3-[N-methyl-N-[2-(3,4-methylenedioxy phenoxy)ethyl]amino]propyl]-5-methoxy-1,3-thiazolidin-4-one, 2-(3,5-di-t-butyl-4-hydroxyphenyl)-3-[3-[N-methyl-N-[2-(3,4-methylenedioxyphenoxy)ethyl]amino]propyl]-5-isopropoxy-carbonylmethyl-1,3-thiazolidin-4-one, 2-(3,5-di-t-butyl-4-hydroxyphenyl)-3-[3-[N-methyl-N-[2-(3,4-methylenedioxy-phenoxy)ethyl]amino]propyl]-5-(2-hydroxyethoxy)-1,3-thiazolidin-4-one, spiro[2-(3,5-di-t-butyl-4-hydroxyphenyl)-3-[3-[N-methyl-N-[2-(3,4-methylenedioxyphenoxy)ethyl]amino]propyl]-1,3-thiazolidin-4-one-5,2'-[1,3]dioxolane], 2-(3,5-di-t-butyl-4-hydroxyphenyl)-3-[3-[N-methyl-N-[2-(3,4-methylenedioxyphenoxy)ethyl]amino]propyl]-5-N,N-dimethylcarbamoylmethyl-1,3-thiazolidin-4-one, 2-(3,5-di-t-butyl-4-hydroxyphenyl)-3-[3-[1-(4-benzyl)piperidyl]propyl]-1,3-thiazolidin-4-one, and 2-(3,5-di-t-butyl-4-hydroxyphenyl)-3-[3-[1-[4-(3,4-methylenedioxyphenoxy)piperidyl]]propyl]-1,3-thiazolidin-4-one, and a pharmaceutically acceptable salt thereof.

20. A compound according to claim 1, which is selected from the group of compounds consisting of 2-(3,5-di-t-butyl-4-hydroxyphenyl)-3-[3-[N-methyl-N-[2-(3,4-methylenedioxyphenoxy)ethyl]amino]propyl]-1,3-thiazolidin-4-one, (+)-2-(3,5-di-t-butyl-4-hydroxyphenyl)-3-[3-[N-methyl-N-[2-(3,4-methylenedioxyphenoxy)ethyl]amino]propyl]-1,3-thiazolidin-4-one, (-)-2-(3,5-di-t-butyl-4-hydroxyphenyl)-3-[3-[N-methyl-N-[2-(3,4-methylenedioxyphenoxy)ethyl]amino]propyl]-1,3-thiazolidin-4-one, 2-(3,5-di-t-butyl-4-hydroxyphenyl)-3-[3-[N-ethyl-N-[2-(3,4-methylenedioxyphenoxy)ethyl]amino]propyl]-1,3-thiazoiidin-4-one, 2-(3,5-di-t-butyl-4-hydroxyphenyl)-3-[3-[N-methyl-N-[3-(3,4-methylenedioxyphenoxy)propyl]amino]propyl]-1,3-thiazolidin-4-one, 2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, 2,5-cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, (-)-2,5-cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, (+)-2,5-cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, 2,5-trans-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one, 5-(3,5-di-t-butyl-4-hydroxyphenyl)-1-[3-[N-methyl-N-[2-(3,4-methylenedioxyphenoxy)ethyl]amino]propyl]imidazole, 2-(3,5-di-t-butyl-4-hydroxyphenyl)-3-[3-[N-methyl-N-[2-(3,4-methylenedioxyphenoxy)ethyl]amino]propyl]-1,3-thiazolidin-4-thione, 5-(3,5-di-t-butyl-4-hydroxyphenyl)-4-ethyl-2-[3-[N-methyl-N-[2-(3,4-methylenedioxyphenoxy)ethyl]amino]propyl]-1,2,4-triazol-3-one, 3-(3,5-di-t-butyl-4-hydroxyphenyl)-5-[3-[N-methyl-N-[2-(3,4-methylenedioxy-phenoxy)ethyl]amino]propoxy]-1,2,4-oxadiazole, 2-(3,5-di-t-butyl-4-hydroxyphenyl)-3-[3-[N-methyl-N-[2-(3,4-methylenedioxyphenoxy)ethyl]amino]propyl]-5-(2-hydroxy-ethyl)-1,3-thiazolidin-4-one, 2-(3,5-di-t-butyl-4-hydroxyphenyl)-3-[3-[N-methyl-N-[2-(3,4-methylene dioxyphenoxy)ethyl]amino]propyl]-5-ethoxycarbonylmethyl-1,3-thiazolidin-4-one, N-[3-[N-methyl-N-[2-(3,4-methylenedioxyphenoxy)ethyl]amino]propyl]-3,5-di-t-butyl-4-hydroxybenzenesulfonamide, 2-(3,5-di-t-butyl-4-hydroxyphenyl)-3-[3-[N-methyl-N-[2-(3,4-methylenedioxyphenoxy)ethyl]amino]propyl]-5-methoxy-1,3-thiazolidin-4-one, 2-(3,5-di-t-butyl-4-hydroxyphenyl)-3-[3-[N-methyl-N-[2-(3,4-methylenedioxyphenoxy)ethyl]amino]propyl]-5-isopropoxy-carbonylmethyl-1,3-thiazolidin-4-one, 2-(3,5-di-t-butyl-4-hydroxyphenyl)-3-[3-[N-methyl-N-[2-(3,4-methylenedioxy-phenoxy)ethyl]amino]propyl]-5-(2-hydroxyethoxy)-1,3-thiazolidin-4-one, spiro[2-(3,5-di-t-butyl-4-hydroxyphenyl)-3-[3-[N-methyl-N-[2-(3,4-methylenedioxy-phenoxy)ethyl]amino]propyl]-1,3-thiazolidin-4-one-5,2'-[1,3]dioxolane], 2-(3,5-di-t-butyl-4-hydroxy-phenyl)-3-[3-[N-methyl-N-[2-(3,4-methylenedioxyphenoxy)ethyl]amino]propyl]-5-N,N-dimethylcarbamoylmethyl-1,3-thiazolidin-4-one, and 2-(3,5-di-t-butyl-4-hydroxy-phenyl)-3-[3-[1-(4-benzyl)piperidyl]propyl]-1,3-thiazolidin-4-one,and a pharmaceutically acceptable salt thereof.

21. 2-(3,5-Di-t-butyl-4-hydroxyphenyl)-3-[3-[N-methyl-N-[2-(3,4-methylenedioxyphenoxy)ethyl]amino]propyl]-1,3-thiazolidin-4-one according to claim 1 and a pharmaceutically acceptable salt thereof or a possible optical isomer thereof.

22. (+)-2-(3,5-Di-t-butyl-4-hydroxyphenyl)-3-[3-[N-methyl-N-[2-(3,4-methylenedioxyphenoxy)ethyl]amino]propyl]-1,3-thiazolidin-4-one according to claim 1 and a pharmaceutically acceptable salt thereof.

23. (-)-2-(3,5-Di-t-butyl-4-hydroxyphenyl)-3-[3-[N-methyl-N-[2-(3,4-methylenedioxyphenoxy)ethyl]amino]propyl]-1,3-thiazolidin-4-one according to claim 1 and a pharmaceutically acceptable salt thereof.

24. 2-(3,5-Di-t-butyl-4-hydroxyphenyl)-3-[3-[N-methyl-N-[2-(3,4-methylenedioxyphenoxy)ethyl]amino]propyl]-5-methyl-1,3-thiazolidin-4-one according to claim 1 and a pharmaceutically acceptable salt thereof or a possible stereoisomer or optical isomer thereof.

25. 2,5-Cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one according to claim 1 and a pharmaceutically acceptable salt thereof or a possible optical isomer thereof.

26. 2,5-Trans-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one according to claim 1 and a pharmaceutically acceptable salt thereof or a possible optical isomer thereof.

27. (-)-2,5-Cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one according to claim 1 and a pharmaceutically acceptable salt thereof.

28. (+)-2,5-Cis-2-(3,5-di-t-butyl-4-hydroxyphenyl)-3-[3-[N-methyl-N-[2-(3,4-methylenedioxyphenoxy)ethyl]amino]propyl]-5-methyl-1,3-thiazolidin-4-one according to claim 1 and a pharmaceutically acceptable salt thereof.

29. A therapeutic agent comprising a compound or a possible stereoisomer or optical isomer thereof or a pharmaceutically acceptable salt thereof of any one of claims 1 to 28.

30. A use of a compound represented by formula (I'): 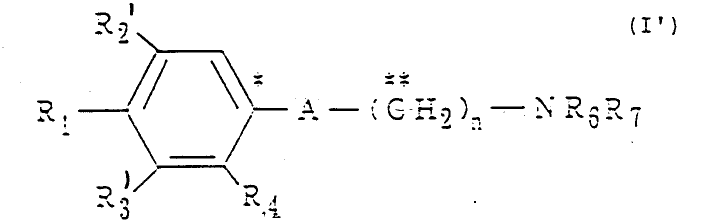 wherein
R₁, R₄, A, R₆ and R₇ and n are as defined in claim 1 and
R₂ and R₃, which may be the same or different, each represents a hydrogen atom, a hydroxyl group, a halogen atom, a lower alkyl group having 1 to 6 carbon atoms or a lower alkoxy group having 1 to 6 carbon atoms, for producing a medicament for treating ischemic diseases.

31. The use according to claim 30, A represents a fragment represented by formula (II), (IV), (VI), (VII), (VIII), (X), (XI), (XII), (XV), or (XVI), as defined in Claim 1.

32. The use according to claim 30, wherein R₁ represents a hydroxy group, an acyloxy group having 1 to 9 carbon atoms or a lower alkoxy group having 1 to 6 carbon atoms.

33. The use according to claim 32, wherein R₂' and R₃', which may be the same or different, each represents a hydrogen atom or a lower alkyl group having 1 to 4 carton atoms, R₄ represents a hydrogen atom or methyl group, A represents a fragment represented by formula (II), (IV), (V), (VI), (VII) or (VIII) as defined in claim 1 and n represents an integer of 2, 3, 4 or 5.

34. The use according to claim 33, wherein R₂' and R₃', which may be the same or different, each represents a methyl group, an ethyl group, an isopropyl group or a t-butyl group, and A represents a fragment represented by formula (II) or (IV).

35. A use of a compound or a possible stereoisomer or optical isomer thereof or a pharmaceutically acceptable salt thereof of any one of claims 6 to 28 for producing a medicament for treating ischemic diseases.

## Patentansprüche

1. Verbindung der Formel (I): 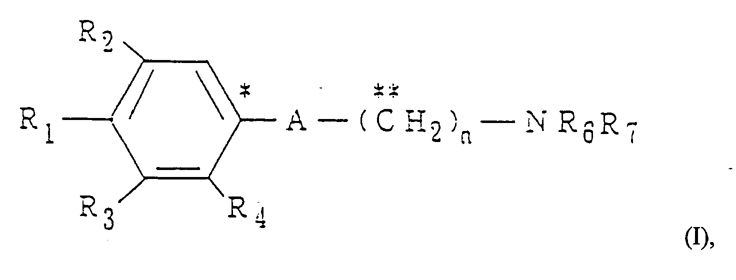 in der
R₁ ein Wasserstoffatom, eine Hydroxylgruppe, einen Acyloxyrest mit 1 bis 9 Kohlenstoffatomen oder einen Niederalkoxyrest mit 1 bis 6 Kohlenstoffatomen bedeutet;
R₂ und R₃, die gleich oder verschieden sein können, jeweils eine Hydroxyl-, Isopropyl- oder t-Butylgruppe darstellen;
R₄ ein Wasserstoffatom oder einen Niederalkylrest mit 1 bis 6 Kohlenstoffatomen bedeutet;
A ein Fragment der Formel (II): 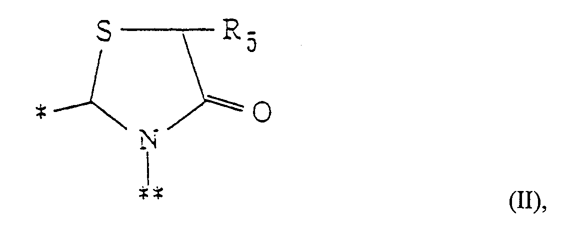 in der
R₅ ein Wasserstoffatom, einen substituierten oder unsubstituierten Niederalkylrest mit 1 bis 6 Kohlenstoffatomen, einen substituierten oder unsubstituierten Niederalkenylrest mit 1 bis 6 Kohlenstoffatomen, einen substituierten oder unsubstituierten Alkoxyrest mit 1 bis 6 Kohlenstoffatomen, einen substituierten oder unsubstituierten Arylrest oder einen substituierten oder unsubstituierten heterocyclischen Rest oder einen 5- oder 6-gliedrigen Ring mit 2 oder mehr Sauerstoffatomen oder Schwefelatomen darstellt, wobei in diesem Fall das Kohlenstoffatom, an das er gebunden ist, ein Spiroatom ist; oder
ein Fragment der Formel (III):
B (III)
bedeutet, in der
B ein Fragment, ausgewählt aus der folgenden Gruppe von Fragmenten der Formel (IV), (V), (VI), (VII), (VIII), (X), (XI), (XII), (XIII), (XIV), (XV) oder (XVI): 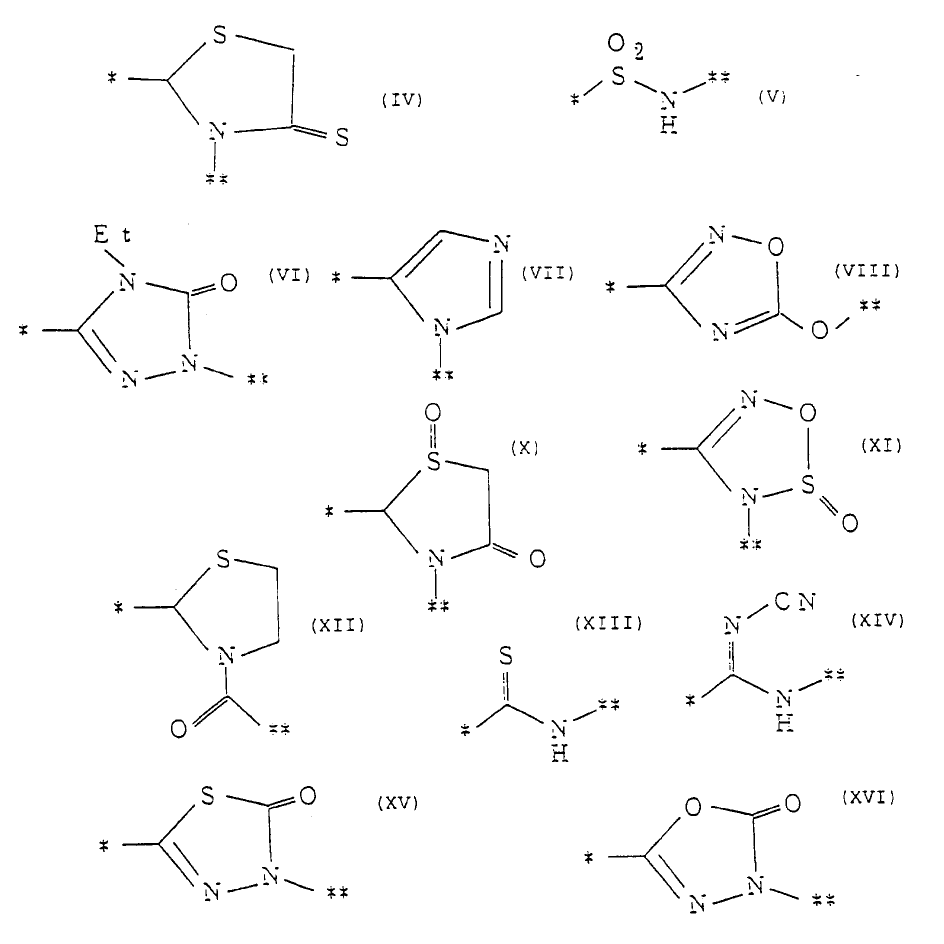 darstellt;
R₆ und R₇, die gleich oder verschieden sein können, jeweils ein Wasserstoffatom, einen substituierten oder unsubstituierten Niederalkylrest mit 1 bis 6 Kohlenstoffatomen, einen substituierten oder unsubstituierten Niederalkenylrest mit 1 bis 6 Kohlenstoffatomen, einen substituierten oder unsubstituierten Arylrest oder einen substituierten oder unsubstituierten heterocyclischen Rest bedeuten, mit der Maßgabe, dass R₆ und R₇ nicht beide eine Methylgruppe darstellen, oder
R₆ und R₇ zusammen einen substituierten oder unsubstituierten Ring bilden, der ein kondensierter Ring sein kann; und
n die ganze Zahl 2, 3, 4, 5 oder 6 ist,
oder ein mögliches Stereoisomer oder optisches Isomer davon oder ein pharmazeutisch verträgliches Salz davon.

2. Verbindung nach Anspruch 1, wobei R₂ und R₃ beide eine t-Butylgruppe bedeuten.

3. Verbindung nach Anspruch 1, wobei R₁ eine Hydroxylgruppe, einen Acyloxyrest mit 1 bis 9 Kohlenstoffatomen oder einen Niederalkoxyrest mit 1 bis 6 Kohlenstoffatomen darstellt.

4. Verbindung nach Anspruch 3, wobei R₂ und R₃, die gleich oder verschieden sein können, jeweils eine Isopropyl- oder t-Butylgruppe bedeuten, R₄ ein Wasserstoffatom oder eine Methylgruppe darstellt, A ein Fragment der Formel (II), (IV), (V), (VI), (VII) oder (VIII) bedeutet, und n die ganze Zahl 2, 3, 4 oder 5 ist.

5. Verbindung nach Anspruch 4, wobei A ein Fragment der Formel (II) oder (IV) darstellt.

6. Verbindung nach Anspruch 5, wobei R₂ und R₃ beide eine t-Butylgruppe bedeuten, R₄ ein Wasserstoffatom darstellt, und A ein Fragment der Formel (II) bedeutet.

7. Verbindung nach Anspruch 6, wobei R₁ eine Hydroxyl- oder Methoxygruppe darstellt.

8. Verbindung nach Anspruch 7, wobei R₁ eine Hydroxylgruppe bedeutet.

9. Verbindung nach Anspruch 8, wobei n 3 ist.

10. Verbindung nach Anspruch 3, wobei R₁ einen Acyloxyrest mit 1 bis 9 Kohlenstoffatomen darstellt, R₂ und R₃ beide eine t-Butylgruppe bedeuten, R₄ ein Wasserstoffatom darstellt, A ein Fragment der Formel (II) bedeutet, und n die ganze Zahl 2, 3, 4 oder 5 ist.

11. Verbindung nach Anspruch 3, wobei R₁ einen Niederalkoxyrest mit 1 bis 6 Kohlenstoffatomen darstellt, R₂ und R₃ beide eine t-Butylgruppe bedeuten, R₄ ein Wasserstoffatom darstellt, A ein Fragment der Formel (II) bedeutet, und n die ganze Zahl 2, 3, 4 oder 5 ist.

12. Verbindung nach Anspruch 11, wobei R₁ eine Methoxygruppe darstellt.

13. Verbindung nach Anspruch 6, wobei R₅ ein Wasserstoffatom, einen substituierten oder unsubstituierten Niederalkylrest mit 1 bis 6 Kohlenstoffatomen, einen substituierten oder unsubstituierten Niederalkoxyrest mit 1 bis 6 Kohlenstoffatomen oder einen substituierten oder unsubstituierten Arylrest oder einen 5- oder 6-gliedrigen Ring mit 2 oder mehr Sauerstoffatomen oder Schwefelatomen bedeutet, wobei in diesem Fall das Kohlenstoffatom, an das er gebunden ist, ein Spiroatom ist; R₆ und R₇, die gleich oder verschieden sein können, jeweils ein Wasserstoffatom oder einen substituierten oder unsubstituierten Niederalkylrest mit 1 bis 6 Kohlenstoffatomen darstellen, mit der Maßgabe, dass R₆ und R₇ nicht beide eine Methylgruppe bedeuten, oder R₆ und R₇ zusammen einen substituierten oder unsubstituierten Ring bilden, der ein kondensierter Ring sein kann.

14. Verbindung nach Anspruch 13, wobei R₆ und R₇, die gleich oder verschieden sein können, jeweils einen substituierten oder unsubstituierten Niederalkylrest mit 1 bis 6 Kohlenstoffatomen darstellen, mit der Maßgabe, dass R₆ und R₇ nicht beide eine Methylgruppe bedeuten, oder R₆ und R₇ zusammen einen substituierten oder unsubstituierten Ring bilden, der ein kondensierter Ring sein kann.

15. Verbindung nach Anspruch 14, wobei R₆ einen substituierten oder unsubstituierten Niederalkylrest mit 1 bis 3 Kohlenstoffatomen bedeutet; R₇ einen Rest, ausgewählt aus einer 2-(3,4-Methylendioxyphenoxy)ethyl-, 3-(3,4-Methylendioxyphenoxy)propyl-, 4-(3,4-Methylendioxyphenoxy)-n-butyl- und 3,4-Dimethoxyphenylmethylgruppe, darstellt; oder -NR₆R₇ einen Rest, ausgewählt aus einer 4-(N-2-Benzothiazolyl-N-methylamino)piperidyl-, 4-Phenylmethylpiperidyl-, 4-(3,4-Methylendioxyphenoxy)piperidyl- und 4-(2,3,4-Trimethoxyphenylmethyl)piperazinylgruppe, bedeutet.

16. Verbindung nach Anspruch 15, wobei R₆ eine Methyl- oder Ethylgruppe darstellt.

17. Verbindung nach Anspruch 13, wobei R₅ ein Wasserstoffatom, einen substituierten oder unsubstituierten Niederalkylrest mit 1 bis 6 Kohlenstoffatomen, einen substituierten oder unsubstituierten Niederalkoxyrest mit 1 bis 6 Kohlenstoffatomen oder einen 5- oder 6-gliedrigen Ring mit 2 oder mehr Sauerstoffatomen oder Schwefelatomen bedeutet, wobei in diesem Fall das Kohlenstoffatom, an das er gebunden ist, ein Spiroatom ist; R₆ eine Methylgruppe darstellt; R₇ einen Rest, ausgewählt aus einer 2-(3,4-Methylendioxyphenoxy)ethyl-, 3-(3,4-Methylendioxyphenoxy)propyl-, 4-(3,4-Methylendioxyphenoxy)-n-butyl- und 3,4-Dimethoxyphenylmethylgruppe, bedeutet; oder -NR₆R₇ einen Rest, ausgewählt aus einer 4-(N-2-Benzothiazolyl-N-methylamino)piperidyl-, 4-Phenylmethylpiperidyl-, 4-(3,4-Methylendioxyphenoxy)piperidyl- und 4-(2,3,4-Trimethoxyphenylmethyl)piperazinylgruppe, darstellt.

18. Verbindung nach einem der Ansprüche 13-17, wobei n 3 ist.

19. Verbindung nach Anspruch 1, ausgewählt aus 2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on, (+)-2-(3,5-Di-t-butyl-4-hydroxyphenyl)-3-[3-[N-methyl-N-[2-(3,4-methylendioxyphenoxy)-ethyl]amino]propyl]-1,3-thiazolidin-4-on, (-)-2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on, 2-(3,5-Di-t-butyl-4-hydroxyphenyl)-3-[3-[N-ethyl-N-[2-(3,4-methylendioxyphenoxy)ethyl]amino]-propyl]-1,3-thiazolidin-4-on, 2-(3,5-Di-t-butyl-4-hydroxyphenyl)-3-[4-[N-methyl-N-[2-(3,4-methylendioxyphenoxy)ethyl]amino]butyl]-1,3-thiazolidin-4-on, 2-(3,5-Di-t-butyl-4-hydroxyphenyl)-3-[5-[N-methyl-N-[2-(3,4-methylendioxyphenoxy)ethyl]amino]pentyl]-1,3-thiazolidin-4-on, 2-(3,5-Di-t-butyl-4-hydroxyphenyl)-3-[3-[N-methyl-N-[3-(3,4-methylendioxyphenoxy)propyl]amino]propyl]-1,3-thiazolidin-4-on, 2-(3,5-Diisopropyl-4-hydroxyphenyl)-3-[3-[N-methyl-N-[2-(3,4-methylendioxyphenoxy)ethyl]amino]propyl)-1,3-thiazolidin-4-on, 2-(3,5-Di-t-butyl-4-hydroxyphenyl)-3-[3-[1-[4-(2,3,4-trimethoxybenzyl)piperazinyl]]propyl]-1,3-thiazolidin-4-on, 2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, 2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, (-)-2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, (+)-2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, 2,5-trans-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, 2-(3,5-Di-t-butyl-4-hydroxyphenyl)-3-[3-[N-methyl-N-[4-(3,4-methylendioxyphenoxy)butyl]amino]propyl]-1,3 thiazolidin-4-on, 5-(3,5-Di-t-butyl-4-hydroxyphenyl)-1-[3-[N-methyl-N-[2-(3,4-methylendioxyphenoxy)ethyl]amino]propyl]imidazol, 2-(3,5-Di-t-butyl-4-hydroxyphenyl)-3-[3-[N-methyl-N-[2-(3,4-methylendioxyphenoxy)ethyl] amino]propyl]-1,3-thiazolidin-4-thion, 5-(3,5-Di-t-butyl-4-hydroxyphenyl)-4-ethyl-2-[3-[N-methyl-N-[2-(3,4-methylendioxyphenoxy)ethyl]amino]propyl]-1,2,4-triazol-3-on, 3-(3,5-Di-t-butyl-4-hydroxyphenyl)-5-[3-[N-methyl-N-[2-(3,4-methylendioxyphenoxy)ethyl]amino]propoxy]-1,2,4-oxadiazol, 2-(3,5-Di-t-butyl-4-hydroxyphenyl)-3-[3-[N-methyl-N-[2-(3,4-methylendioxyphenoxy)ethyl]amino]propyl]-5-(2-hydroxyethyl)-1,3-thiazolidin-4-on, 2-(3,5-Di-t-butyl-4-hydroxyphenyl)-3-[3-[N-methyl-N-[2-(3,4-methylendioxyphenoxy)ethyl]amino]propyl]-5-ethoxycarbonylmethyl-1,3-thiazolidin-4-on, N-[3-[N-Methyl-N-[2-(3,4-methylendioxyphenoxy)ethyl]amino]propyl]-3,5-di-t-butyl-4-hydroxybenzolsulfonamid, 2-(3,5-Di-t-butyl-4-hydroxyphenyl)-3-[3-[N-methyl-N-[2-(3,4-methylendioxyphenoxy)ethyl]amino]propyl]-5-methoxy-1,3-thiazolidin-4-on, 2-(3,5-Di-t-butyl-4-hydroxyphenyl)-3-[3-[N-methyl-N-[2-(3,4-methylendioxyphenoxy)ethyl]amino]propyl]-5-isopropoxycarbonylmethyl-1,3-thiazolidin-4-on, 2-(3 5-Di-t-butyl-4-hydroxyphenyl)-3-[3 [N-methyl-N-[2-(3,4-methylendioxyphenoxy)ethyl]amino]propyl]-5-(2-hydroxyethoxy)-1,3-thiazolidin-4-on, spiro-[2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on-5'2'-[1,3]dioxolan], 2-(3,5-Di-t-butyl-4-hydroxyphenyl)-3-[3-[N-methyl-N-[2-(3,4-methylendioxyphenoxy)ethyl]amino]propyl]-5-N,N-dimethylcarbamoylmethyl-1,3-thiazolidin-4-on, 2-(3,5-Di-t-butyl-4-hydroxyphenyl)-3-[3-[1-(4-benzyl)piperidyl]propyl]-1,3-thiazolidin-4-on und 2-(3,5-Di-t-butyl-4-hydroxyphenyl)-3-[3-[1-[4-(3,4-methylendioxyphenoxy)piperidyl]]propyl]-1,3-thiazolidin-4-on, und ein pharmazeutisch verträgliches Salz davon.

20. Verbindung nach Anspruch 1, ausgewählt aus 2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on, (+)-2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on, (-)-2-(3,5-Di-t-butyl-4-hydroxyphenyl)-3-[3-[N-methyl-N-[2-(3,4-methylendioxyphenoxy)ethyl]amino]propyl]-1,3 thiazolidin-4-on, 2-(3,5-Di-t-butyl-4-hydroxyphenyl)-3-[3-[N-ethyl-N-[2-(3,4-methylendioxyphenoxy)ethyl]amino]propyl]-1,3-thiazolidin-4-on, 2-(3,5-Di-t-butyl-4-hydroxyphenyl)-3-[3-[N-methyl-N-[3-(3,4-methylendioxyphenoxy)propyl]amino]propyl]-1,3-thiazolidin-4-on, 2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, 2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, (-)-2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, (+)-2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, 2,5-trans-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on, 5-(3,5-Di-t-butyl-4-hydroxyphenyl)-1-[3-[N-methyl-N-[2-(3,4-methylendioxyphenoxy)ethyl]amino]propyl]imid azol, 2-(3,5-Di-t-butyl-4-hydroxyphenyl)-3-[3-[N-methyl-N-[2-(3,4-methylendioxyphenoxy)ethyl]amino]propyl]-1,3-thiazolidin-4-thion, 5-(3,5-Di-t-butyl-4-hydroxyphenyl)-4-ethyl-2-[3-[N-methyl-N-[2-(3,4-methylendioxyphenoxy)ethyl]amino]propyl]-1,2,4-triazol-3-on, 3-(3,5-Di-t-butyl-4-hydroxyphenyl)-5-[3-[N-methyl-N-[2-(3,4-methylendioxyphenoxy)ethyl]amino]propoxy]-1,2,4-oxadiazol, 2-(3,5-Di-t-butyl-4-hydroxyphenyl)-3-[3-[N-methyl-N-[2-(3,4-methylendioxyphenoxy)ethyl]amino]propyl]-5-(2-hydroxyethyl)-1,3-thiazolidin-4-on, 2-(3,5-Di-t-butyl-4-hydroxyphenyl)-3-[3-[N-methyl-N-[2-(3,4-methylendioxyphenoxy)ethyl]amino]propyl]-5-ethoxycarbonylmethyl-1,3-thiazolidin-4-on, N-[3-[N-Methyl-N-[2-(3,4-methylendioxyphenoxy)ethyl]amino]propyl]-3,5-di-t-butyl-4-hydroxybenzolsulfonamid, 2-(3,5-Di-t-butyl-4-hydroxyphenyl)-3-[3-[N-methyl-N-[2-(3,4-methylendioxyphenoxy)ethyl]amino]propyl]-5-methoxy-1,3-thiazolidin-4-on, 2-(3,5-Di-t-butyl-4-hydroxyphenyl)-3-[3-[N-methyl-N-[2-(3,4-methylendioxyphenoxy)ethyl]amino]propyl]-5-isopropoxycarbonylmethyl-1,3-thiazolidin-4-on, 2-(3,5-Di-t-butyl-4-hydroxyphenyl)-3-[3-[N-methyl-N-[2-(3,4-methylendioxyphenoxy)ethyl]amino]propyl]-5-(2-hydroxyethoxy)-1,3-thiazolidin-4-on, spiro-[2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on-5,2'-[1,3]dioxolan], 2-(3,5-Di-t-butyl-4-hydroxyphenyl)-3-[3-[N-methyl-N-[2-(3,4-methylendioxyphenoxy)ethyl]amino]propyl]-5-N,N-dimethylcarbamoylmethyl-1,3-thiazolidin-4-on und 2-(3,5-Di-t-butyl-4-hydroxyphenyl)-3-[3-[1-(4-benzyl)piperidyl]propyl]-1,3-thiazolidin-4-on, und ein pharmazeutisch verträgliches Salz davon.

21. 2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on nach Anspruch 1 und ein pharmazeutisch verträgliches Salz davon oder ein mögliches optisches Isomer davon.

22. (+)-2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on nach Anspruch 1 und ein pharmazeutisch verträgliches Salz davon.

23. (-)-2-(3,5-Di-t-butyl-4-hydroxyphenyl)-3-[3-[N-methyl-N-[2-(3,4-methylendioxyphenoxy)ethyl]amino]propyl]-1,3-thiazolidin-4-on nach Anspruch 1 und ein pharmazeutisch verträgliches Salz davon.

24. 2-(3,5-Di-t-butyl-4-hydroxyphenyl)-3-[3-[N-methyl-N-[2-(3.4-methylendioxyphenoxy)ethyl]amino]propyl]-5-methyl-1,3-thiazolidin-4-on nach Anspruch 1 und ein pharmazeutisch verträgliches Salz davon oder ein mögliches Stereoisomer oder optisches Isomer davon.

25. 2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on nach Anspruch 1 und ein pharmazeutisch verträgliches Salz davon oder ein mögliches optisches Isomer davon.

26. 2,5-trans-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on nach Anspruch 1 und ein pharmazeutisch verträgliches Salz davon oder ein mögliches optisches Isomer davon.

27. (-)-2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on nach Anspruch 1 und ein pharmazeutisch verträgliches Salz davon.

28. (+)-2,5-cis-2-(3,5-Di-t-butyl-4-hydroxyphenyl)-3-[3-[N-methyl-N-[2-(3,4-methylendioxyphenoxy)ethyl]amino]propyl]-5-methyl-1,3-thiazolidin-4-on nach Anspruch 1 und ein pharmazeutisch verträgliches Salz davon.

29. Arzneimittel, umfassend eine Verbindung oder ein mögliches Stereoisomer oder optisches Isomer davon oder ein pharmazeutisch verträgliches Salz davon, nach einem der Ansprüche 1 bis 28.

30. Verwendung einer Verbindung der Formel (I'): 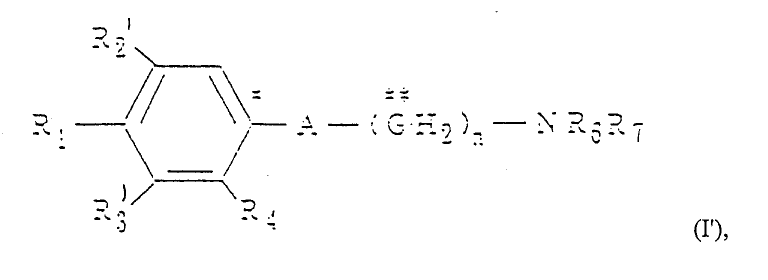 in der
R₁, R₄, A, R₆, R₇ und n wie in Anspruch 1 definiert sind, und
R₂ und R₃, die gleich oder verschieden sein können, jeweils ein Wasserstoffatom, eine Hydroxylgruppe, ein Halogenatom, einen Niederalkylrest mit 1 bis 6 Kohlenstoffatomen oder einen Niederalkoxyrest mit 1 bis 6 Kohlenstoffatomen bedeuten, zur Herstellung eines Medikaments zur Behandlung ischämischer Erkrankungen.

31. Verwendung nach Anspruch 30, wobei A ein Fragment der Formel (II), (IV), (VI), (VII), (VIII), (X), (XI), (XII), (XV) oder (XVI), wie in Anspruch 1 definiert, darstellt.

32. Verwendung nach Anspruch 30, wobei R₁ eine Hydroxylgruppe, einen Acyloxyrest mit 1 bis 9 Kohlenstoffatomen oder einen Niederalkoxyrest mit 1 bis 6 Kohlenstoffatomen bedeutet.

33. Verwendung nach Anspruch 32, wobei R₂' und R₃', die gleich oder verschieden sein können, jeweils ein Wasserstoffatom oder einen Niederalkylrest mit 1 bis 4 Kohlenstoffatomen darstellen, R₄ ein Wasserstoffatom oder eine Methylgruppe bedeutet, A ein Fragment der Formel (II), (IV), (V), (VI), (VII) oder (VIII), wie in Anspruch 1 definiert, darstellt, und n die ganze Zahl 2, 3, 4 oder 5 ist

34. Verwendung nach Anspruch 33, wobei R₂' und R₃', die gleich oder verschieden sein können, jeweils eine Methyl-, Ethyl-, Isopropyl- oder t-Butylgruppe bedeuten, und A ein Fragment der Formel (II) oder (IV) darstellt.

35. Verwendung einer Verbindung oder eines möglichen Stereoisomers oder optischen Isomers davon oder eines phamazeutisch verträglichen Salzes davon nach einem der Ansprüche 6 bis 28 zur Herstellung eines Medikaments zur Behandlung ischämischer Erkrankungen.

## Revendications

1. Composé représenté par la formule (I): 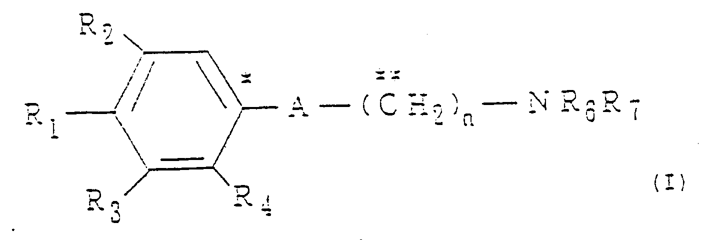 dans laquelle
R₁ représente un atome d'hydrogène, un groupe hydroxyle, un groupe acyloxy comportant 1 à 9 atomes de carbone, ou un groupe alcoxy inférieur comportant 1 à 6 atomes de carbone;
R₂ et R₃, qui peuvent être identiques ou différents, représentent chacun un groupe hydroxyle, un groupe isopropyle ou un groupe t-butyle;
R₄ représente un atome d'hydrogène, un groupe alkyle inférieur comportant 1 à 6 atomes de carbone;
A représente un fragment représenté par la formule (II): 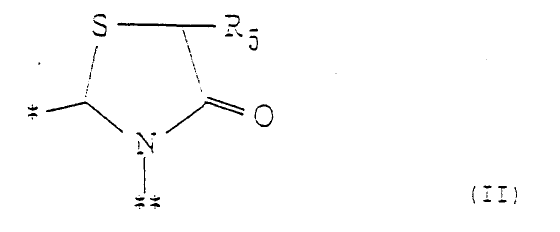 dans laquelle
R₅ représente un atome d'hydrogène, un groupe alkyle inférieur substitué ou non substitué comportant 1 à 6 atomes de carbone, un groupe alcényle inférieur substitué ou non substitué comportant 1 à 6 atomes de carbone, un groupe alcoxy substitué ou non substitué comportant 1 à 6 atomes de carbone, un groupe aryle substitué ou non substitué ou un groupe hétérocyclique substitué ou non substitué, ou un noyau à 5 ou 6 chaînons contenant au moins deux atomes d'oxygène ou atomes de soufre, auquel cas l'atome de carbone auquel il est fixé est un atome spiro: ou
un fragment représenté par la formule (III):
B (III)
dans laquelle
B représente un fragment choisi dans le groupe constitué par les fragments suivants, représentés par la formule (IV), (V), (VI), (VII), (VIII), (X), (XI), (XII), (XIII), (XIV), (XV) ou (XVI): 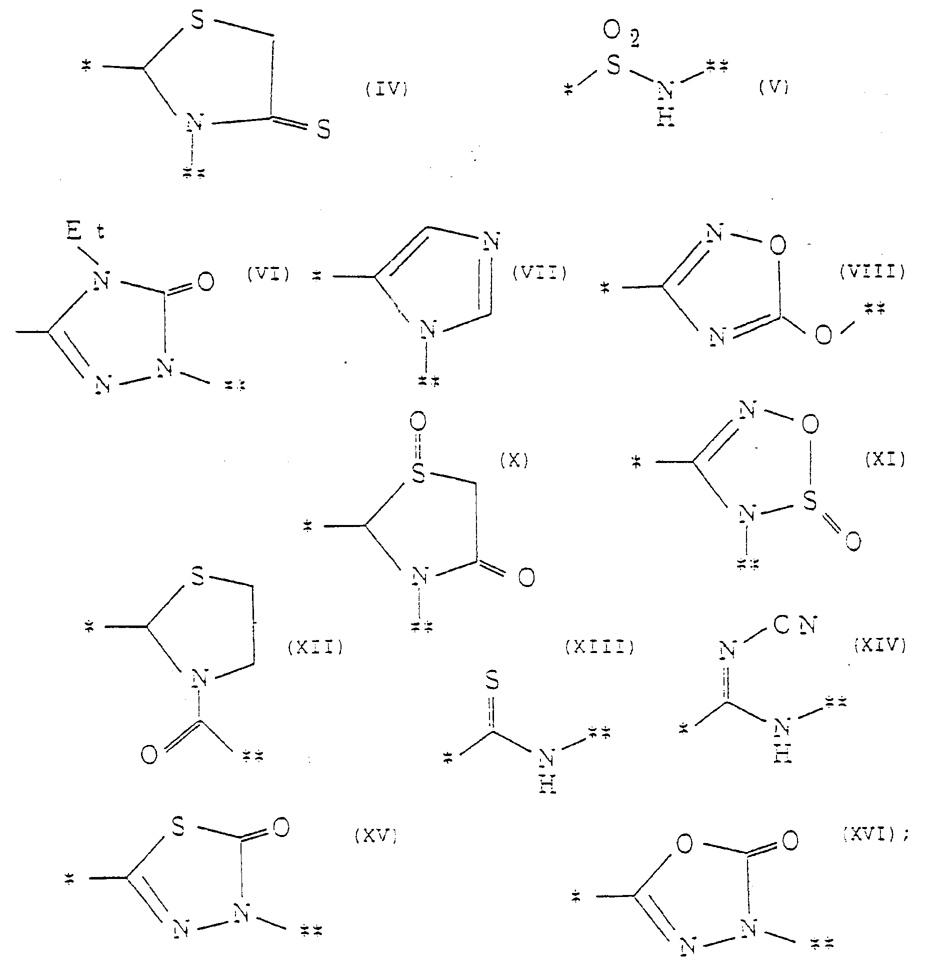
R₆ et R₇, qui peuvent être identiques ou différents, représentent chacun un atome d'hydrogène, un groupe alkyle inférieur substitué ou non substitué comportant 1 à 6 atomes de carbone, un groupe alcényle inférieur substitué ou non substitué comportant 1 à 6 atomes de carbone, un groupe aryle substitué ou non substitué ou un groupe hétérocyclique substitué ou non substitué, pourvu que R₆ et R₇ ne représentent pas simultanément un groupe méthyle, ou bien
R₆ et R₇ sont pris conjointement pour former un noyau substitué ou non substitué qui peut être un noyau condensé; et
n représente un nombre entier de 2, 3, 4, 5 ou 6,
ou stéréoisomère ou isomère optique éventuel de ce composé, ou sel pharmaceutiquement acceptable de ce composé.

2. Composé selon la revendication 1, dans lequel R₂ et R₃ représentent tous deux un groupe t-butyle.

3. Composé selon la revendication 1, dans lequel R₁ représente un groupe hydroxy, un groupe acyloxy comportant 1 à 9 atomes de carbone ou un groupe alcoxy inférieur comportant 1 à 6 atomes de carbone.

4. Composé selon la revendication 3, dans lequel R₂ et R₃, qui peuvent être identiques ou différents, représentent chacun un groupe isopropyle ou un groupe t-butyle, R₄ représente un atome d'hydrogène ou un groupe méthyle, A représente un fragment représenté par les formules (II), (IV), (V), (VI), (VII) ou (VIII) et n représente un nombre entier de 2, 3, 4 ou 5.

5. Composé selon la revendication 4, dans lequel A représente un fragment représenté par la formule (II) ou (IV).

6. Composé selon la revendication 5, dans lequel R₂ et R₃ représentent tous deux un groupe t-butyle, R₄ représente un atome d'hydrogène et A représente un fragment représenté par la formule (II).

7. Composé selon la revendication 6, dans lequel R₁ représente un groupe hydroxy ou un groupe méthoxy.

8. Composé selon la revendication 7, dans lequel R₁ représente un groupe hydroxy.

9. Composé selon la revendication 8, dans lequel n vaut 3.

10. Composé selon la revendication 3, dans lequel R₁ représente un groupe acyloxy comportant 1 à 9 atomes de carbone, R₂ et R₃ représentent tous deux un groupe t-butyle, R₄ représente un atome d'hydrogène, A représente un fragment représenté par la formule (II) et n représente un nombre entier de 2, 3, 4 ou 5.

11. Composé selon la revendication 3, dans lequel R₁ représente un groupe alcoxy inférieur comportant 1 à 6 atomes de carbone, R₂ et R₃ représentent tous deux un groupe t-butyle, R₄ représente un atome d'hydrogène, A représente un fragment représenté par la formule (II) et n représente un nombre entier de 2, 3, 4 ou 5.

12. Composé selon la revendication 11, dans lequel R₁ représente un groupe méthoxy.

13. Composé selon la revendication 6, dans lequel R₅ représente un atome d'hydrogène, un groupe alkyle inférieur substitué ou non substitué comportant 1 à 6 atomes de carbone, un groupe alcoxy inférieur substitué ou non substitué comportant 1 à 6 atomes de carbone ou un groupe aryle substitué ou non substitué, ou un noyau à 5 ou 6 chaînons contenant deux ou plus de deux atomes d'oxygène ou de soufre, auquel cas l'atome de carbone auquel il est fixé est un atome spiro; R₆ et R₇, qui peuvent être identiques ou différents, représentent chacun un atome d'hydrogène ou un groupe alkyle inférieur substitué ou non substitué comportant 1 à 6 atomes de carbone, à condition que R₆ et R₇ ne représentent pas simultanément un groupe méthyle, ou bien R₆ et R₇ sont pris conjointement pour former un noyau substitué ou non substitué qui peut être un noyau condensé.

14. Composé selon la revendication 13, dans lequel R₆ et R₇, qui peuvent être identiques ou différents, représentent chacun un groupe alkyle inférieur substitué ou non substitué comportant 1 à 6 atomes de carbone, à condition que R₆ et R₇ ne représentent pas simultanément un groupe méthyle, ou bien R₆ et R₇ sont pris conjointement pour former un noyau substitué ou non substitué qui peut être un noyau condensé.

15. Composé selon la revendication 14, dans lequel R₆ représente un groupe alkyle inférieur substitué ou non substitué comportant 1 à 3 atomes de carbone; R₇ représente un groupe choisi dans le groupe constitué par un groupe 2-(3,4-méthylènedioxyphénoxy)éthyle, un groupe 3-(3,4-méthylènedioxyphénoxy)propyle, un groupe 4-(3,4-méthylènedioxyphénoxy)-n-butyle et un groupe 3,4-diméthoxyphénylméthyle; ou bien -NR₆R₇ est choisi dans le groupe constitué par un groupe 4-(N-2-benzothiazolyl-N-méthylamino)pipéridyle, un groupe 4-phénylméthylpipéridyle, un groupe 4-(3,4-méthylènedioxyphénoxy)pipéridyle et un groupe 4-(2,3,4-triméthoxyphénylméthyl)pipérazinyle.

16. Composé selon la revendication 15, dans lequel R₆ représente un groupe méthyle ou un groupe éthyle.

17. Composé selon la revendication 13, dans lequel R₅ représente un atome d'hydrogène, un groupe alkyle inférieur substitué ou non substitué comportant 1 à 6 atomes de carbone, un groupe alcoxy inférieur substitué ou non substitué comportant 1 à 6 atomes de carbone, ou un noyau à 5 ou 6 chaînons contenant deux ou plus de deux atomes d'oxygène ou de soufre, auquel cas l'atome de carbone auquel il est fixé est un atome spiro; R₆ représente un groupe méthyle; R₇ représente un groupe choisi dans le groupe constitué par un groupe 2-(3,4-méthylènedioxyphénoxy)éthyle, un groupe 3-(3,4-méthylènedioxyphénoxy)propyle, un groupe 4-(3,4-méthylènedioxyphénoxy)-n-butyle et un groupe 3,4-diméthoxyphénylméthyle; ou bien -NR₆R₇ représente un groupe choisi dans le groupe constitué par un groupe 4-(N-2-benzothiazolyl-N-méthylamino)pipéridyle, un groupe 4-phénylméthylpipéridyle, un groupe 4-(3,4-méthylènedioxyphénoxy)pipéridyle et un groupe 4-(2,3,4-triméthoxyphénylméthyl)pipérazinyle.

18. Composé selon l'une quelconque des revendications 13-17, dans lequel n vaut 3.

19. Composé selon la revendication 1, qui est choisi dans le groupe de composés constitué par la 2-(3,5-di-t-butyl-4-hydroxyphényl)-3-[3-[N-méthyl-N-[2-(3,4-méthylènedioxy-phénoxy)éthyl] amino]propyl]-1,3-thiazolidin-4-one, la (+)-2-(3,5-di-t-butyl-4-hydroxyphényl)-3-[3-[N-méthyl-N-[2-(3,4-méthylènedioxyphénoxy)éthyl]amino]propyl]-1,3 -thiazolidin-4-one, la (-)-2-(3,5-di-t-butyl-4-hydroxy-phényl)-3-[3-[N-méthyl-N-[2-(3,4-méthylènedioxyphénoxy)éthyl] amino]propyl]-1,3-thiazolidin-4-one, la 2-(3,5-di-t-butyl-4-hydroxyphényl)-3-[3-[N-éthyl-N-[2-(3,4-méthylènedioxyphénoxy)éthyl]amino]propyl]-1,3-thiazolidin-4-one, la 2-(3,5-di-t-butyl-4-hydroxyphényl)-3-[4-[N-méthyl-N-[2-(3,4-méthylènedioxyphénoxy)éthyl]amino]butyl]-1,3-thiazolidin-4-one, la 2-(3,5-di-t-butyl-4-hydroxyphényl)-3-[5-[N-méthyl-N-[2-(3,4-méthylènedioxyphénoxy)éthyl]amino]pentyl]-1,3-thiazolidin-4-one, la 2-(3,5-di-t-butyl-4-hydroxyphényl)-3-[3-[N-méthyl-N-[3-(3,4-méthylènedioxyphénoxy)propyl]amino]propyl]-1,3-thiazolidin-4-one, la 2-(3,5-diisopropyl-4-hydroxyphényl)-3-[3-[N-méthyl-N-[2-(3,4-méthylènedioxyphénoxy)éthyl]amino]propyl]-1,3-thiazolidin-4-one, la 2-(3,5-di-t-butyl-4-hydroxyphényl)-3-[3-[1-[4-(2,3,4-triméthoxybenzyl)pipérazinyl]]propyl]-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la 2,5-cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la (-)-2,5-cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la (+)-2,5-cis-2-(3,5-di-t-butyl-4-hydroxyphényl)-3-[3-[N-méthyl-N-[2-(3,4-méthylènedioxyphénoxy)éthyl] amino]propyl]-5-méthyl-1,3-thiazolidin-4-one, la 2,5-trans-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la 2-(3,5-di-t-butyl-4-hydroxyphényl)-3-[3-[N-méthyl-N-[4-(3,4-méthylènedioxyphénoxy)butyl]amino]propyl]-1,3-thiazolidin-4-one, la 5-(3,5-di-t-butyl-4-hydroxyphényl)-1-[3-[N-méthyl-N-[2-(3,4-méthylènedioxyphénoxy)éthyl]amino]propyl]imidazole, la 2-(3,5-di-t-butyl-4-hydroxyphényl)-3-[3-[N-méthyl-N-[2-(3,4-méthylènedioxyphénoxy)éthyl]amino]propyl]-1,3-thiazolidin-4-thione, la 5-(3,5-di-t-butyl-4-hydroxyphényl)-4-éthyl-2-[3-[N-méthyl-N-[2-(3,4-méthylènedioxyphénoxy)éthyl]amino]propyl]-1,2,4-triazol-3-one, la 3-(3,5-di-t-butyl-4-hydroxyphényl)-5-[3-[N-méthyl-N-[2-(3,4-méthylènedioxy-phénoxy)éthyl]amino]propoxy]-1,2,4-oxadiazole, la 2-(3,5-di-t-butyl-4-hydroxyphényl)-3-[3-[N-méthyl-N-[2-(3,4-méthylènedioxyphénoxy)éthyl]amino]propyl]-5-(2-hydroxy-éthyl)-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-éthoxycarbonylméthyl-1,3-thiazolidin-4-one, la N-[3-[N-méthyl-N-[2-(3,4-méthylènedioxyphénoxy)éthyl]amino]propyl]-3,5-di-t-butyl-4-hydroxybenzènesulfonamide, la 2-(3,5-di-t-butyl-4-hydroxyphényl)-3-[3-[N-méthyl-N-[2-(3,4-méthylènedioxyphénoxy)éthyl]amino]propyl]-5-méthoxy-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-isopropoxy-carbonylméthyl-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-(2-hydroxyéthoxy)-1,3-thiazolidin-4-one, le spiro-[2-(3,5-di-t-butyl-4-hydroxyphényl)-3-[3-[N-méthyl-N-[2-(3,4-méthylènedioxyphénoxy)éthyl]amino]propyl]-1,3-thiazolidin-4-one-5,2'-[1,3]dioxolane], la 2-(3,5-di-t-butyl-4-hydroxyphényl)-3-[3-[N-méthyl-N-[2-(3,4-méthylènedioxyphénoxy)éthyl]amino]propyl]-5-N,N-diméthylcarbamoylméthyl-1,3-thiazolidin-4-one, la 2-(3,5-di-t-butyl-4-hydroxyphényl)-3-[3-[1-(4-benzyl)pipéridyl]propyl]-1,3-thiazolidin-4-one et la 2-(3,5-di-t-butyl-4-hydroxyphényl)-3-[3-[1-[4-(3,4-méthylènedioxyphénoxy)pipéridyl]]propyl]-1,3-thiazolidin-4-one, et sel pharmaceutiquement acceptable de ce composé.

20. Composé selon la revendication 1, qui est choisi dans le groupe de composés constitué par la 2-(3,5-di-t-butyl-4-hydroxyphényl)-3-[3-[N-méthyl-N-[2-(3,4-méthylènedioxyphénoxy)éthyl]amino]propyl]-1,3-thiazolidin-4-one, la (+)-2-(3,5-di-t-butyl-4-hydroxyphényl)-3-[3-[N-méthyl-N-[2-(3,4-méthylènedioxyphénoxy)éthyl]amino]propyl]-1,3-thiazolidin-4-one, la (-)-2-(3,5-di-t-butyl-4-hydroxyphényl)-3-[3-[N-méthyl-N-[2-(3,4-méthylènedioxyphénoxy)éthyl]amino]propyl]-1,3-thiazolidin-4-one, la 2-(3,5-di-t-butyl-4-hydroxyphényl)-3-[3-[N-éthyl-N-[2-(3,4-méthylènedioxyphénoxy)éthyl]amino]propyl]-1,3-thiazolidin-4-one, la 2-(3,5-di-t-butyl-4-hydroxyphényl)-3-[3-[N-méthyl-N-[3-(3,4-méthylènedioxyphénoxy)propyl]amino]propyl]-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la 2,5-cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la (-)-2,5-cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la (+)-2,5-cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, la 2,5-trans-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one, le 5-(3,5-di-t-butyl-4-hydroxyphényl)-1-[3-[N-méthyl-N-[2-(3,4-méthylènedioxyphénoxy)éthyl]amino]propyl]imidazole, la 2-(3,5-di-t-butyl-4-hydroxyphényl)-3-[3-[N-méthyl-N-[2-(3,4-méthylènedioxyphénoxy)éthyl]amino]propyl]-1,3-thiazolidin-4-thione, la 5-(3,5-di-t-butyl-4-hydroxyphényl)-4-éthyl-2-[3-[N-méthyl-N-[2-(3,4-méthylènedioxyphénoxy)éthyl]amino]propyl]-1,2,4-triazol-3-one, le 3-(3,5-di-t-butyl-4-hydroxyphényl)-5-[3-[N-méthyl-N-[2-(3,4-méthylènedioxyphénoxy)éthyl]amino]propoxy]-1,2,4-oxadiazole, la 2-(3,5-di-t-butyl-4-hydroxyphényl)-3-[3-[N-méthyl-N-[2-(3,4-méthylènedioxyphénoxy)éthyl]amino]propyl]-5-(2-hydroxy-éthyl)-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-éthoxycarbonylméthyl-1,3-thiazolidin-4-one, le N-[3-[N-méthyl-N-[2-(3,4-méthylènedioxyphénoxy)éthyl]amino]propyl]-3,5-di-t-butyl-4-hydroxybenzènesulfonamide, la 2-(3,5-di-t-butyl-4-hydroxyphényl)-3-[3-[N-méthyl-N-[2-(3,4-méthylènedioxyphénoxy)éthyl]amino]propyl]-5-méthoxy-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-isopropoxycarbonyl-méthyl-1,3-thiazolidin-4-one, la 2-(3,5-di-t-butyl-4-hydroxyphényl)-3-[3-[N-méthyl-N-[2-(3,4-méthylènedioxyphénoxy)éthyl]amino]propyl]-5-(2-hydroxyéthoxy)-1,3-thiazolidin-4-one, le spiro-[2-(3,5-di-t-butyl-4-hydroxyphényl)-3-[3-[N-méthyl-N-[2-(3,4-méthylènedioxyphénoxy)éthyl]amino]propyl]-1,3-thiazolidin-4-one-5,2'-[1,3]dioxolane], la 2-(3,5-di-t-butyl-4-hydroxyphényl)-3-[3-[N-méthyl-N-[2-(3,4-méthylènedioxyphénoxy)éthyl]amino]propyl]-5-N,N-diméthylcarbamoylméthyl-1,3-thiazolidin-4-one, et la 2-(3,5-di-t-butyl-4-hydroxy-phényl)-3-[3-[1-(4-benzyl)pipéridyl]propyl]-1,3-thiazolidin-4-one, et sel pharmaceutiquement acceptable de ce composé.

21. 2-(3,5-Di-t-butyl-4-hydroxyphényl)-3-[3-[N-méthyl-N-[2-(3,4-méthylènedioxyphénoxy)éthyl]amino]propyl]-1,3-thiazolidin-4-one selon la revendication 1 et sel pharmaceutiquement acceptable de ce composé ou isomère optique éventuel de ce composé.

22. (+)-2-(3,5-Di-t-butyl-4-hydroxyphényl)-3-[3-[N-méthyl-N-[2-(3,4-méthylènedioxyphénoxy)éthyl]amino]propyl]-1,3-thiazolidin-4-one selon la revendication 1 et sel pharmaceutiquement acceptable de ce composé.

23. (-)-2-(3,5-Di-t-butyl-4-hydroxyphényl)-3-[3-[N-méthyl-N-[2-(3,4-méthylènedioxyphénoxy)éthyl]amino]propyl]-1,3-thiazolidin-4-one selon la revendication 1 et sel pharmaceutiquement acceptable de ce composé.

24. 2-(3,5-Di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one selon la revendication 1 et sel pharmaceutiquement acceptable de ce composé ou stéréoisomère ou isomère optique éventuel de ce composé.

25. 2,5-Cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3- thiazolidin-4-one selon la revendication 1 et sel pharmaceutiquement acceptable de ce composé ou isomère optique éventuel de ce composé.

26. 2,5-Trans-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one selon la revendication 1 et sel pharmaceutiquement acceptable de ce composé ou isomère optique éventuel de ce composé.

27. (-)-2,5-Cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one selon la revendication 1 et sel pharmaceutiquement acceptable de ce composé.

28. (+)-2,5-Cis-2-(3,5-di-t-butyl-4-hydroxyphényl)-3-[3-[N-méthyl-N-[2-(3,4-méthylènedioxyphénoxy)éthyl]amino]propyl]-5-méthyl-1,3-thiazolidin-4-one selon la revendication 1 et sel pharmaceutiquement acceptable de ce composé.

29. Agent thérapeutique comprenant un composé ou un stéréoisomère ou isomère optique éventuel de ce composé, ou un sel pharmaceutiquement acceptable de ce composé, selon l'une quelconque des revendications 1 à 28.

30. Utilisation d'un composé représenté par la formule (I'): 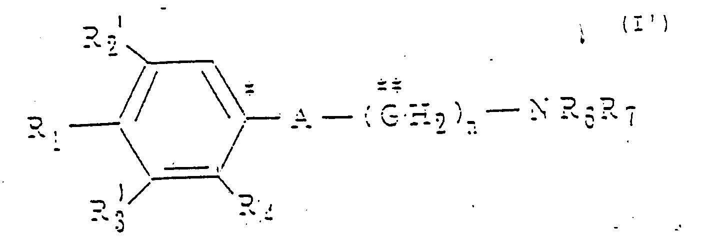 dans laquelle
R₁, R₄, A, R₆ et R₇ et n sont tels que définis dans la revendication 1 et
R₂ et R₃, qui peuvent être identiques ou différents, représentent chacun un atome d'hydrogène, un groupe hydroxyle, un atome d'halogène, un groupe alkyle inférieur comportant 1 à 6 atomes de carbone ou un groupe alcoxy inférieur comportant 1 à 6 atomes de carbone, pour la production d'un médicament destiné à traiter les maladies ischémiques.

31. Utilisation selon la revendication 30, dans laquelle A représente un fragment représenté par la formule (II), (IV), (VI), ,(VII), (VIII), (X), (XI), (XII), (XV) ou (XVI), tel que défini dans la revendication 1.

32. Utilisation selon la revendication 30, dans laquelle R₁ représente un groupe hydroxy, un groupe acyloxy comportant 1 à 9 atomes de carbone ou un groupe alcoxy inférieur comportant 1 à 6 atomes de carbone.

33. Utilisation selon la revendication 32, dans laquelle R₂' et R₃', qui peuvent être identiques ou différents, représentent chacun un atome d'hydrogène ou un groupe alkyle inférieur comportant 1 à 4 atomes de carbone, R₄ représente un atome d'hydrogène ou un groupe méthylé, A représente un fragment représenté par la formule (II), (IV), (V), (VI), (VII) ou (VIII), telle que définie dans la revendication 1, et n représente un nombre entier de 2, 3, 4 ou 5.

34. Utilisation selon la revendication 33, dans laquelle R₂' et R₃', qui peuvent être identiques ou différents, représentent chacun un groupe méthyle, un groupe éthyle, un groupe isopropyle ou un groupe t-butyle, et A représente un fragment représenté par la formule (II) ou (IV).

35. Utilisation d'un composé ou d'un stéréoisomère ou d'un isomère optique éventuel de ce composé, ou d'un sel pharmaceutiquement acceptable de ce composé, selon l'une quelconque des revendications 6 à 28, pour la production d'un médicament destiné à traiter les maladies ischémiques.
